# NEUE EUROPÄISCHE PATENTSCHRIFT

(11) **EP 1 326 613 B2**
(45) Veröffentlichungstag und Bekanntmachung des Hinweises auf die Entscheidung über den Einspruch: **18.04.2007**
(45) Hinweis auf die Patenterteilung: 02.06.2004
(21) Anmeldenummer: 01972099.4
(22) Anmeldetag: 10.10.2001
(51) Int. Cl.: A61K 31/4985, A61K 31/519

(54) **VERWENDUNG VON SUBSTITUIERTEN IMIDAZO ¬1,2-A|PYRIDIN-, -PYRIMIDIN- UND -PYRAZIN-3-YL-AMIN-DERIVATEN ZUR HERSTELLUNG VON MEDIKAMENTEN ZUR NOS-INHIBIERUNG**
USE OF SUBSTITUTED IMIDAZO 1,2-A|PYRIDINE-, IMIDAZO ¬1,2-A|PYRIMIDINE AND IMIDAZO ¬1,2-A|PYRAZINE-3-YL-AMINE DERIVATIVES FOR PRODUCING NOS-INHIBITING MEDICAMENTS
UTILISATION DE DERIVES SUBSTITUES D'IMIDAZO ¬1,2-A|PYRIDINE, D'IMIDAZO ¬1,2-A|PYRIMIDINE ET D'IMIDAZO ¬1,2-A|PYRAZINE-3-YL-AMINE DANS LA PRODUCTION DE MEDICAMENTS INHIBITEURS DE NOS

(30) Priorität: 13.10.2000 DE 10050663
(43) Veröffentlichungstag der Anmeldung: 16.07.2003
(73) Patentinhaber: Grünenthal GmbH, 52078 Aachen (DE)
(72) Erfinder: SUNDERMANN, Bernd, 52066 Aachen (DE); MAUL, Corinna, 52066 Aachen (DE); HENNIES, Hagen-Heinrich, 52152 Simmerath (DE); SCHNEIDER, Johannes, 52223 Stolberg (DE)
(74) Vertreter: Brosch, Oliver
(86) Internationale Anmeldenummer: PCT/EP2001/011701
(87) Internationale Veröffentlichungsnummer: WO 2002/030428

(56) Entgegenhaltungen:
- EP-A- 0 418 071
- WO-A-01/02711
- WO-A-01/27109
- WO-A-01/81344
- WO-A-02/02557
- WO-A-91/08211
- WO-A-98/39342
- FR-A- 2 638 161
- US-A- 4 450 164
- MURAD F: "Discovery of Some of the Biological Effetcs of Nitric Oxide and Its Role in Cell Signalling (Nobel Lecture)" ANGEWANDTE CHEMIE. INTERNATIONAL EDITION, VERLAG CHEMIE. WEINHEIM, DE, Bd. 38, 1999, Seiten 1856-1868, XP002159759 ISSN: 0570-0833
- BIENAYME H ET AL: "A NEW HETEROCYCLIC MULTICOMPONENT REACTION FOR THE COMBINATORIAL SYNTHESIS OF FUSED 3-AMINOIMIDAZOLES" ANGEWANDTE CHEMIE. INTERNATIONAL EDITION, VERLAG CHEMIE. WEINHEIM, DE, Bd. 37, Nr. 16, 1998, Seiten 2234-2237, XP000978871 ISSN: 0570-0833
- BLACKBURN, C., ET AL.: "Parallel Synthesis of 3-Aminoimidazo[1,2-a]pyridines and pyrazines by a New Three-Component Condensation" TETRAHEDRON LETT., Bd. 39, 1998, Seiten 3635-3638, XP002192771
- Synlett, June 1998
- Clin. Neuros., 1998, vol. 5, pp. 28-33
- Tetrahedron Letter, vol. 39 (1998), pp. 3635-3638
- Chem. Pharm. Bull, vol. 40, no. 5 (1992), pp. 1170-1176
- Pathol. Biol., vol. 48 (2000), pp. 648-657
- Drugs of the Future, vol. 23, no. 2 (1998), pp. 123-132
- GRA 3019 EP, 2005

## Beschreibung

Die Erfindung betrifft die Verwendung von substituierten Imidazo[1,2-a]pyridin-, -pyrimidin- und -pyrazin-3-yl-amin-Derivaten zur Herstellung von Medikamenten zur NOS-Inhibierung, zur Herstellung von Medikamenten zur Behandlung von Migräne sowie zur Herstellung von Medikamenten zur Behandlung von, Multipler Sklerose, Morbus Parkinson, Morbus Alzheimer, Morbus Huntington, Entzündungsschmerz, cerebraler Ischämie, Diabetes, Meningitis, Arteriosklerose und/oder für die Wundheilung.

Stickstoffmonoxid (NO) reguliert zahlreiche physiologische Prozesse, unter anderem die Neurotransmission, Relaxation und Proliferation von glatter Muskulatur, die Adhäsion und Aggregation von Thrombozyten sowie die Gewebeverletzung und Entzündung. Aufgrund der Vielzahl von Signalfunktionen wird NO mit einer Reihe von Krankheiten in Verbindung gebracht (s. z.B. L. J. Ignarro, *Angew. Chem.* (1999), 111, 2002-2013 und F. Murad, *Angew. Chem. Int. Ed.* (1999), 111, 1976-1989). Eine wichtige Rolle bei der therapeutischen Beeinflussung diesef Krankheiten spielt dabei das für die physiologische Bildung von NO verantwortliche Enzym, die NO-Synthase (NOS). Bislang wurden drei verschiedene Isoformen der NO-Synthase, nämlich die beiden konstitutiven Formen nNOS und eNOS sowie die induzierbare Form iNOS, identifiziert (s. A. J. Hobbs, A. Higgs, S. Moncada, *Annu. Rev. Pharmacol. Toxicol.* (1999), *39*, 191-220; I. C. Green, P.-E. Chabrier, *DDT* (1999), 4, 47-49; P.-E. Chabrier et al., *Cell*. *Mol. Life Sci.* (1999), 55, 1029-1035).

Die Hemmung der NO-Synthase eröffnet neue Therapieansätze für verschiedene Krankheiten, die mit NO in Zusammenhang stehen (A. J. Hobbs et al., *Annu. Rev. Pharmacol. Toxicol*. (1999), *39*, 191-220; I. C. Green, P.-E. Chabrier, *DDT* (1999), 4, 47-49; P.-E. Chabrier et al., *Cell. Mol. Life Sci*. (1999), 55, 1029-1035), wie beispielsweise Migräne (L. L. Thomsen, J. Olesen, *Clinical Neuroscience* (1998), *5*, 28-33; L. H. Lassen et al., *The Lancet* (1997), *349*, 401-402), septischer Schock, neurodegenerative Erkrankungen wie Multiple Sklerose, Morbus Parkinson, Morbus Alzheimer oder Morbus Huntington, Entzündungen, Entzündungsschmerz, cerebrale Ischämie, Diabetes, Meningitis und Arteriosklerose. Darüber hinaus kann die NOS-Inhibierung einen Effekt auf die Wundheilung, auf Tumoren und auf die Angiogenese haben sowie eine unspezifische Immunität gegen Microorganismen bewirken (A. J. Hobbs et al., *Annu. Rev. Pharmacol. Toxicol*. (1999), *39*, 191-220).

Bislang bekannte Wirkstoffe, die die NO-Synthase hemmen, sind neben L-NMMA und L-NAME - d.h. Analoga des L-Arginins, aus dem in-vivo unter Beteiligung von NOS NO und Citrullin gebildet werden - u.a. S-Methyl-L-citrullin, Aminoguanidin, S-Methylisoharnstoff, 7-Nitroindazol und 2-Mercaptoethylguanidin (A. J. Hobbs et al., *Annu. Rev. Pharmacol. Toxicol*. (1999), *39*, 191-220).

Der vorliegenden Erfindung lag demgegenüber die Aufgabe zugrunde, neue wirksame NOS-Inhibitoren zur Verfügung zu stellen.

Überraschenderweise wurde gefunden, daß substituierte Imidazo[1,2-a]pyridin-, -pyrimidin- und -pyrazin-3-yl-amin-Derivate der allgemeinen Struktur I 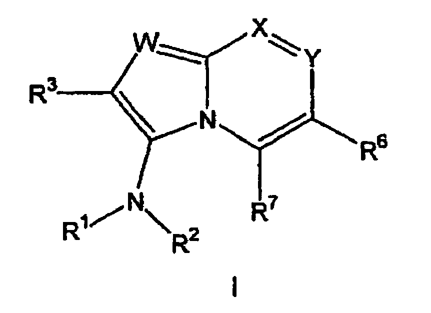 worin
- X: CR⁴ oder N bedeutet,
- Y: CR⁵ oder N bedeutet und
- X und Y: nicht zugleich N bedeuten,
- W: N oder NR⁸ bedeutet,
- R¹: C₁₋₁₂-Alkyl, wobei Alkyl geradkettig oder verzweigt ist und gesättigt oder ungesättigt ist und unsubstituiert oder ein- oder mehrfach substituiert ist, C₃₋₈-Cycloalkyl oder CH₂-C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,
- R²: Wasserstoff oder C(=O)R⁹ bedeutet,
- R³: C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert ist, Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,
- R⁴, R⁵, R⁶ und R⁷: unabhängig voneinander Wasserstoff oder C₁₋₈-Alkyl, wobei Alkyl geradkettig oder verzweigt ist und gesättigt oder ungesättigt ist und unsubstituiert oder eih- oder mehrfach substituiert ist, C₃₋₈-Cycloalkyl oder OH₂-C₃₋₈-Cycloalkyl, wobei Cycloalkyl gesättigt oder ungesättigt ist und unsubstituiert oder ein- oder mehrfach substituiert ist, F, Cl, Br, I, CN, NO₂, NH₂, C(=O)R⁹, CO₂H, CO₂R¹⁰, OH oder OR¹¹ bedeuten,
oder
- R⁴ und R⁵ oder R⁵ und R⁶ oder R⁶ und R⁷: für eine viergliedrige gesättigte oder ungesättigte Kohlenwasserstoffbrücke mit keinem, 1, 2 oder 3 Heteroatomen, die aus der Gruppe, die N, O und S enthält, ausgewählt sind, stehen und die anderen Reste von R⁴, R⁵, R⁶ und R⁷ Wasserstoff bedeuten,
- R⁸: C(=O)R⁹ bedeutet,
- R⁹: C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl oder CH₂-C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-Aryl oder C₁₋₈-Alkyl-Heteroaryl bedeutet, wobei Alkyl geradkettig oder verzweigt ist und gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet, und
- R¹⁰ und R¹¹: unabhängig voneinander C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl oder CH₂-C₃₋₈-Cycloalkyl, wobei Cycloalkyl gesättigt oder ungesättigt ist und unsubstituiert oder ein- oder mehrfach substituiert ist, Aryl, wobei Aryl unsubstituiert oder ein- oder mehrfach substituiert ist, C₁₋₈-Alkyl-Aryl, wobei Alkyl geradkettig oder verzweigt ist und gesättigt oder ungesättigt ist und unsubstituiert oder ein- oder mehrfach substituiert ist und Aryl unsubstituiert oder ein- oder mehrfach substituiert ist, bedeuten,
mit Ausnahme von Verbindungen der allgemeinen Struktur I, in denen zugleich
R¹ = tert.-Butyl,
R² = H,
R³ = C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert ist, Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,
X=CR⁴mit R⁴=H,
Y = CR⁵ mit R⁵ = CH₃,
R⁶ = H und
R⁷ = H oder C₁₋₄-Alkanyl, wobei Alkanyl geradkettig oder verzweigt und unsubstituiert oder ein- oder mehrfach substituiert ist, bedeuten,
wobei man im Zusammenhang mit "Alkyl" und "Alkanyl" der ausgenommenen Verbindungen unter dem Begriff "substituiert" die Substitution eines Wasserstoffrestes durch F, Cl, Br, I, -CN, NH₂, NH-Alkyl, NH-Aryl, NH-Heteroaryl, NH-Alkyl-Aryl, NH-Alkyl-Heteroaryl, NH-Heterocyclyl, NH-Alkyl-OH, N(Alkyl)₂, N(Alkyl-Aryl)₂, N(Alkyl-Heteroaryl)₂, N(Heterocyclyl)₂, N(Alkyl-OH)₂, NO, NO₂, SH, S-Alkyl, S-Aryl, S-Heteroaryl, S-Alkyl-Aryl, S-Alkyl-Heteroaryl, S-Heterocyclyl, S-Alkyl-OH, S-Alkyl-SH, OH, O-Alkyl, O-Aryl, O-Heteroaryl, O-Alkyl-Aryl, O-Alkyl-Heteroaryl, O-Heterocyclyl, O-Alkyl-OH, CHO, C(=O)C₁₋₆-Alkyl, C(=S)C₁₋₆-Alkyl, C(=O)Aryl, C(=S)Aryl, C(=O)C₁₋₆-Alkyl-Aryl, 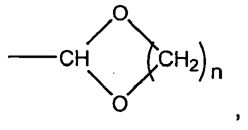 mit n = 1, 2 oder 3, C(=S)C₁₋₆-Alkyl-Aryl, C(=O)-Heteroaryl, C(=S)-Heteroaryl, C(=O)-Heterocyclyl, C(=S)-Heterocyclyl, CO₂H, CO₂-Alkyl, CO₂-Alkyl-Aryl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Alkyl-Heteroaryl)₂, C(=O)N(Heterocyclyl)₂, SO-Alkyl, SO₂-Alkyl, SO₂NH₂, SO₃H, Cycloalkyl, Aryl, Heteroaryl oder Heterocyclyl versteht, wobei unter mehrfach substituierten Resten solche Reste zu verstehen sind, die entweder an verschiedenen oder an gleichen Atomen mehrfach substituiert sind,
sehr wirksame NOS-Inhibitoren darstellen.

Gegenstand der vorliegenden Erfindung ist daher die Verwendung der Verbindungen der wie oben definierten allgemeinen Struktur I in Form ihrer Basen oder ihrer pharmazeutisch annehmbaren Salze zur Herstellung eines Medikaments zur NO-Synthase-Inhibierung. Ferner ist die Verwendung einer Verbindung der allgemeinen Struktur I in Form ihrer Base oder eines ihrer pharmazeutisch annehmbaren Salze zur Herstellung eines Medikaments zur Behandlung von Migräne sowie zur Behandlung von Multipler Sklerose, Morbus Parkinson, Morbus Alzheimer, Morbus Huntington, Entzündungsschmerz, cerebraler Ischämie, Diabetes, Meningitis, Arteriosklerose und/oder für die Wundheilung Gegenstand der vorliegenden Erfindung.

Es ist bevorzugt, daß von den erfindungsgemäßen Verwendungen solche Verbindungen der allgemeinen Struktur I, in denen zugleich R¹ = tert.-Butyl, R² = H, X = CR⁴ mit R⁴ = H, Y = CR⁵ mit R⁵ = Methyl, R⁶ = H und R⁷ = H oder C₁₋₄-Alkanyl (wobei Alkanyl geradkettig oder verzweigt und unsubstituiert oder ein- oder mehrfach substituiert ist), ausgenommen sind.

Die Ausdrücke "C₁₋₈-Alkyl" und "C₁₋₁₂-Alkyl" umfassen im Sinne dieser Erfindung acyclische gesättigte oder ungesättigte Kohlenwasserstoffreste, die verzweigt- oder geradkettig sowie unsubstituiert oder ein- oder mehrfach substituiert sein können, mit 1 bis 8 bzw. 1 bis 12 C-Atomen, d.h. C₁₋₈-Alkanyle, C₂₋₈-Alkenyle und C₂₋₈-Alkinyle bzw. C₁₋₁₂-Alkanyle, C₂₋₁₂-Alkenyle und C₂₋₁₂-Alkinyle. Dabei weisen Alkenyle mindestens eine C-C-Doppelbindung und Alkinyle mindestens eine C-C-Dreifachbindung auf. Vorteilhaft ist Alkyl aus der Gruppe ausgewählt, die Methyl, Ethyl, n-Propyl, 2-Propyl, n-Butyl, iso-Butyl, sec.-Butyl, tert.-Butyl, n-Pentyl, iso-Pentyl, neo-Pentyl, n-Hexyl, 2-Hexyl, n-Octyl, 1,1,3,3-Tetramethylbutyl, n-Decyl, n-Dodecyl; Ethylenyl (Vinyl), Ethinyl, Propenyl (-CH₂CH=CH₂, -CH=CH-CH₃, -C(=CH₂)-CH₃), Propinyl (-CH-C≡CH, -C≡C-CH₃), Butenyl, Butinyl, Pentenyl, Pentinyl, Hexenyl, Hexinyl, Octenyl und Octinyl umfaßt.

Der Ausdruck "C₃₋₈-Cycloalkyl" bedeutet für die Zwecke dieser Erfindung cyclische Kohlenwasserstoffe mit 3 bis 8 Kohlenstoffatomen, die gesättigt oder ungesättigt, unsubstituiert oder ein- oder mehrfach substituiert sein können. Vorteilhaft ist C₃₋₈-Cycloalkyl aus der Gruppe ausgewählt, die Cyclopropyl, Cyclobutyl, Cyclopentyl, Cyclohexyl, Cycloheptyl, Cyclooctyl, Cyclopentenyl, Cyclohexenyl, Cycloheptenyl und Cyclooctenyl enthält. Besonders bevorzugt steht Cycloalkyl für Cyclohexyl.

Der Ausdruck "Heterocyclyl" steht für einen 3-, 4-, 5-, 6- oder 7-gliedrigen cyclischen organischen Rest, der mindestens 1, ggf. auch 2, 3, 4 oder 5 Heteroatome enthält, wobei die Heteroatome gleich oder verschieden sind und der cyclische Rest gesättigt oder ungesättigt, aber nicht aromatisch ist und unsubstituiert oder ein- oder mehrfach substituiert sein kann. Der Heterocyclus kann auch Teil eines bi- oder polycyclischen Systems sein. Bevorzugte Heteroatome sind Stickstoff, Sauerstoff und Schwefel. Es ist bevorzugt, daß der Heterocyclyl-Rest ausgewählt ist aus der Gruppe, die Tetrahydrofuryl, Tetrahydropyranyl, Pyrrolidinyl, Piperidinyl, Piperazinyl und Morpholinyl enthält, wobei die Bindung an die Verbindung der allgemeinen Struktur I über jedes beliebige Ringglied des Heterocyclyl-Restes erfolgen kann.

Der Ausdruck "Aryl" bedeutet im Sinne dieser Erfindung aromatische Kohlenwasserstoffe, u.a. Phenyle, Naphthyle und Phenanthrenyle. Die Aryl-Reste können auch mit weiteren gesättigten, (partiell) ungesättigten oder aromatischen Ringsystemen kondensiert sein. Jeder Aryl-Rest kann unsubstituiert oder einfach oder mehrfach substituiert vorliegen, wobei die Aryl-Substituenten gleich oder verschieden und in jeder beliebigen und möglichen Position des Aryls sein können. Vorteilhafterweise ist Aryl aus der Gruppe ausgewählt, die Phenyl, 1-Naphthyl, 2-Naphthyl und Phenanthren-9-yl, welche jeweils unsubstituiert oder ein- oder mehrfach substituiert sein können, enthält.

Der Ausdruck "Heteroaryl" steht für einen 5-, 6- oder 7-gliedrigen cyclischen aromatischen Rest, der mindestens 1, ggf. auch 2, 3, 4 oder 5 Heteroatome, enthält, wobei die Heteroatome gleich oder verschieden sind und der Heterocyclus unsubstituiert oder ein- oder mehrfach substituiert sein kann; im Falle der Substitution am Heterocyclus können die Heteroarylsubstituenten gleich oder verschieden sein und in jeder beliebigen und möglichen Position des Heteroaryls sein. Der Heterocyclus kann auch Teil eines bi- oder polycyclischen Systems sein. Bevorzugte Heteroatome sind Stickstoff, Sauerstoff und Schwefel. Es ist bevorzugt, daß der Heteroaryl-Rest ausgewählt ist aus der Gruppe, die Pyrrolyl, Indolyl, Furyl (Furanyl), Benzofuranyl, Thienyl (Thiophenyl), Benzothienyl, Pyrazolyl, Imidazolyl, Thiazolyl, Oxazolyl, Isoxazoyl, Pyridinyl, Pyridazinyl, Pyrimidinyl, Pyrazinyl, Pyranyl, Indolyl, Indazolyl, Purinyl, Pyrimidinyl, Indolizinyl, Chinolinyl, Isochinolinyl, Chinazolinyl, Carbazolyl, Phenazinyl, Phenothiazinyl enthält, wobei die Bindung an die Verbindungen der allgemeinen Struktur I über jedes beliebige und mögliche Ringglied des Heteroaryl-Restes erfolgen kann. Besonders bevorzugte Heteroaryl-Reste sind für die Zwecke dieser Erfindung Pyridin-2-yl, Pyridin-3-yl, Furan-2-yl, Furan-3-yl, Thien-2-yl (2-Thiophen), Thien-3-yl (3-Thiophen) und Benzo[b]furan-2-yl, die jeweils unsubstituiert oder ein- oder mehrfach substituiert sein können.

Die Ausdrücke "C₁₋₈-Alkyl-C₃₋₈-Cycloalkyl" bzw. "CH₂-C₃₋₈-Cycloalkyl", "C₁₋₈-Alkyl-Heterocyclyl", "C₁₋₈-Alkyl-Aryl" oder "C₁₋₈-Alkyl-Heteroaryl" bedeuten für die Zwecke der vorliegenden Erfindung, daß C₁₋₈-Alkyl (bzw. CH₂) und Cycloalkyl, Heterocyclyl, Aryl und Heteroaryl die oben definierten Bedeutungen haben und der Cycloalkyl-, Heterocyclyl-, Aryl- bzw. Heteroaryl-Rest über eine C₁₋₈-Alkyl-Gruppe (bzw. im Falle von "CH₂-C₃₋₈-Cycloalkyl" über eine CH₂-Gruppe) an die Verbindung der allgemeinen Struktur I gebunden ist.

Im Zusammenhang mit "Alkyl", "Alkanyl", "Alkenyl" und "Alkinyl" versteht man unter dem Begriff "substituiert" im Sinne dieser Erfindung die Substitution eines Wasserstoffrestes durch F, Cl, Br, I, -CN, -N≡C, NH₂, NH-Alkyl, NH-Aryl, NH-Heteroaryl, NH-Alkyl-Aryl, NH-Alkyl-Heteroaryl, NH-Heterocyclyl, NH-Alkyl-OH, N(Alkyl)₂, N(Alkyl-Aryl)₂, N(Alkyl-Heteroaryl)₂, N(Heterocyclyl)₂, N(Alkyl-OH)₂, NO, NO₂, SH, S-Alkyl, S-Aryl, S-Heteroaryl, S-Alkyl-Aryl, S-Alkyl-Heteroaryl, S-Heterocyclyl, S-Alkyl-OH, S-Alkyl-SH, OH, O-Alkyl, O-Aryl, O-Heteroaryl, O-Alkyl-Aryl, O-Alkyl-Heteroaryl, O-Heterocyclyl, O-Alkyl-OH, CHO, C(=O)C₁₋₆-Alkyl, C(=S)C₁₋₆-Alkyl, C(=O)Aryl, C(=S)Aryl, C(=O)C₁₋₆-Alkyl-Aryl, 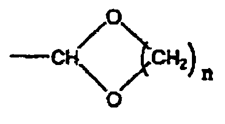 mit n = 1, 2 oder 3, C(=S)C₁₋₆-Alkyl-Aryl, C(=O)-Heteroaryl, C(=S)-Heteroaryl, C(=O)-Heterocyclyl, C(=S)-Heterocyclyl, CO₂H, CO₂-Alkyl, CO₂-Alkyl-Aryl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Alkyl-Heteroaryl)₂, C(=O)N(Heterocyclyl)₂, SO-Alkyl, SO₂-Alkyl, SO₂NH₂, SO₃H, PO(O-C₁₋₆-Alkyl)₂, Si(C₁₋₆-Alkyl)₃, Si(C₃₋₈-Cycloalkyl)₃, Si(CH₂-C₃₋₈-Cycloalkyl)₃, Si(Phenyl)₃, Cycloalkyl, Aryl, Heteroaryl oder Heterocyclyl, wobei unter mehrfach substituierten Resten solche Reste zu verstehen sind, die entweder an verschiedenen oder an gleichen Atomen mehrfach, z.B. zwei- oder dreifach, substituiert sind, beispielsweise dreifach am gleichen C-Atom wie im Falle von CF₃ oder -CH₂CF₃ oder an verschiedenen Stellen wie im Falle von -CH(OH)-CH=CH-CHCl₂. Die Mehrfachsubstitution kann mit dem gleichen oder mit verschiedenen Substituenten erfolgen. Ggf. kann ein Substituent auch seinerseits substituiert sein; so umfaßt -OAlkyl u.a. auch -O-CH₂-CH₂-O-CH₂-CH₂-OH. Besonders bevorzugt für die Zwecke der vorliegenden Erfindung bedeutet "Alkyl" in diesem Zusammenhang Methyl, Ethyl, CH₂-OH, CH₂CO₂H, CH₂CO₂Methyl, CH₂PO(O-C₁₋₆-Alkanyl)₂, CH₂Si(C₁₋₆-Alkanyl)₃, CH₂Si(C₃₋₈-Cycloalkyl)₃, CH₂Si(CH₂-C₃₋₈-Cycloalkyl)₃, CH₂Si(Phenyl)₃, CH₂CH₂-Morpholin-4-yl, CH₂-Aryl, CF₃ oder (CH₂)ₙ-N≡C mit n = 2, 3, 4, 5 oder insbesondere 6.

In Bezug auf "Aryl", "Heterocyclyl", "Heteroaryl" sowie "Cycloalkyl" versteht man im Sinne dieser Erfindung unter "ein- oder mehrfach substituiert" die ein- oder mehrfache, z.B. zwei-, drei- oder vierfache, Substitution eines oder mehrerer Wasserstoffatome des Ringsystems durch F, Cl, Br, I, CN, NH₂, NH-Alkyl, NH-Aryl, NH-Heteroaryl, NH-Alkyl-Aryl, NH-Alkyl-Heteroaryl, NH-Heterocyclyl, NH-Alkyl-OH, N(Alkyl)₂, N(Alkyl-Aryl)₂, N(Alkyl-Heteroaryl)₂, N(Heterocyclyl)₂, N(Alkyl-OH)₂, NO, NO₂, SH, S-Alkyl, S-Cycloalkyl, S-Aryl, S-Heteroaryl, S-Alkyl-Aryl, S-Alkyl-Heteroaryl, S-Heterocyclyl, S-Alkyl-OH, S-Alkyl-SH, OH, O-Alkyl, O-Cycloalkyl, O-Aryl, O-Heteroaryl, O-Alkyl-Aryl, O-Alkyl-Heteroaryl, O-Heterocyclyl, O-Alkyl-OH, CHO, C(=O)C₁₋₆-Alkyl, C(=S)C₁₋₆-Alkyl, C(=O)Aryl, C(=S)Aryl, C(=O)-C₁₋₆-Alkyl-Aryl, 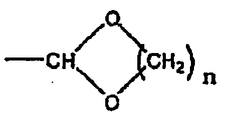 mit n = 1, 2 oder 3, C(=S)C₁₋₆-Alkyl-Aryl, C(=O)-Heteroaryl, C(=S)-Heteroaryl, C(=O)-Heterocyclyl, C(=S)-Heterocyclyl, CO₂H, CO₂-Alkyl, CO₂-Alkyl-Aryl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Alkyl-Heteroaryl)₂, C(=O)N(Heterocyclyl)₂, S(O)-Alkyl, S(O)-Aryl, SO₂-Alkyl, SO₂-Aryl, SO₂NH₂, SO₃H, CF₃, =O, =S; Alkyl, Cycloalkyl, Aryl, Heteroaryl und/oder Heterocyclyl; an einem oder ggf. verschiedenen Atomen (wobei ein Substituent ggf. seinerseits substituiert sein kann). Die Mehrfachsubstitution erfolgt dabei mit dem gleichen oder mit unterschiedlichen Substituenten. Für "Aryl" sind dabei besonders bevorzugte Substituenten -F, -Cl, -Br, -CF₃, -OH, -O-CH₃, -O-CH₂CH₃, Methyl, n-Propyl, Carboxy (-CO₂H), Nitro, 4-Chlorphenoxy, Acetoxy und Dimethylamino. Für "Heteroaryl" sind besonders bevorzugte Substituenten Methyl-OH, -O-CH₃, -CH₂OH, -NO₂, -CO₂H, -CO₂Ethyl, Acetoxymethyl, -Br, -Cl, -Methylsulfanyl (-S-CH₃), Nitrophenyl, Chlorphenyl und -[1,3]-Dioxolan.

Für "Cycloalkyl" sind besonders bevorzugte Substituenten CO₂H und CO₂Ethyl. Für "Heterocyclyl" sind bevorzugte Substituenten Methyl und Ethyl.

Pharmazeutisch annehmbare Salze im Sinne dieser Erfindung sind solche Salze der erfindungsgemäßen Verbindungen gemäß der allgemeinen Struktur I, die bei pharmazeutischer Verwendung physiologisch - insbesondere bei Anwendung am Säugetier und/oder Menschen - verträglich sind. Solche pharmazeutisch annehmbaren Salze können beispielsweise mit anorganischen oder organischen Säuren gebildet werden.

Vorzugsweise werden die pharmazeutisch annehmbaren Salze der erfindungsgemäßen Verbindungen gemäß der allgemeinen Struktur I mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure gebildet. Bei den gebildeten Salzen handelt es sich u.a. um Hydrochloride, Hydrobromide, Phosphate, Carbonate, Hydrogencarbonate, Formiate, Acetate, Oxalate, Succinate, Tartrate, Fumarate, Citrate und Glutaminate. Ebenfalls bevorzugt sind Solvate und insbesondere die Hydrate der erfindungsgemäßen Verbindungen, die z.B. durch Kristallisation aus wäßriger Lösung erhalten werden können.

Sofern die Verbindungen der allgemeinen Struktur I mindestens ein Asymmetriezentrum aufweisen, können sie in Form ihrer Racemate, in Form der reinen Enantiomeren und/oder Diastereomeren oder in Form von Mischungen dieser Enantiomeren bzw. Diastereomeren vorliegen, und zwar sowohl in Substanz als auch als pharmazeutisch annehmbare Salze dieser Verbindungen. Die Mischungen können in jedem beliebigen Mischungsverhältnis der Stereoisomeren vorliegen. Chirale Verbindungen der allgemeinen Struktur I liegen bevorzugt als enantiomerenreine Verbindungen vor.

Es ist bevorzugt, solche Verbindungen der allgemeinen Struktur I (in Form ihrer Basen oder ihrer pharmazeutisch annehmbaren Salze) für die erfindungsgemäße Herstellung eines Medikaments zur NOS-Inhibierung, zur Behandlung von Migräne bzw. zur Behandlung von, Multipler Sklerose, Morbus Parkinson, Morbus Alzheimer, Morbus Huntington, Entzündungsschmerz, cerebraler Ischämie, Diabetes, Meningitis, Arteriosklerose und/oder für die Wundheilung zu verwenden, in denen
- R¹: Methyl, Ethyl, n-Propyl, 2-Propyl, n-Butyl, tert-Butyl, n-Hexyl, n-Octyl, 1,1,3,3-Tetramethylbutyl, CH₂Aryl, wobei Aryl unsubstituiert oder ein- oder mehrfach substituiert ist, CH₂CO₂-C₁₋₆-Alkyl, wobei Alkyl geradkettig oder verzweigt ist, CH₂PO(O-C₁₋₆-Alkyl)₂, wobei Alkyl geradkettig oder verzweigt ist, CH₂SiR¹²R¹³R¹⁴, CH₂CH₂-Morpholin-4-yl, (CH₂)ₙ-NC mit n = 2, 3, 4, 5 oder 6, C₃₋₈-Cycloarkyl, wobei Cycloalkyl unsubstituiert oder ein- oder mehrfach substituiert ist, oder Phenyl, wobei Phenyl unsubstituiert oder ein- oder mehrfach substituiert ist, bedeutet,
- R²: H oder C(=O)-C₁₋₄-Alkyl bedeutet,
- R³: Methyl, Ethyl, n-Propyl, 2-Propyl, n-Butyl, tert-Butyl, Cyclopropyl, Cyclopentyl oder Cyclohexyl, die unsubstituiert dder ein- oder mehrfach subsituiert sind, Phenyl, wobei Phenyl unsubstituiert oder ein- oder mehrfach substituiert ist, 1-Naphthyl oder 2-Naphthyl, wobei Naphthyl unsubstituiert oder ein- oder mehrfach substituiert ist, 9-Phenanthrenyl, Pyrrol-2-yl, Pyrrol-3-yl, Pyridin-2-yl, Pyridin-3-yl oder Pyridin-4-yl, wobei Pyrrolyl oder Pyridinyl unsubstituiert oder ein- oder mehrfach substiuiert sind, Furan-2-yl oder Furan-3-yl, wobei Furanyl unsubstituiert oder ein- oder mehrfach substituiert ist, Thien-2-yl oder Thien-3-yl, wobei Thienyl unsubstituiert oder ein- oder mehrfach substituiert ist, Imidazol-2-yl, Imidazol-4-yl, Imidazol-5-yl, wobei Imidazolyl unsubstituiert oder ein- oder mehrfach substituiert ist, Thiazol-2-yl, Thiazol-4-yl, Thiazol-5-yl, wobei Thiazolyl unsubstituiert oder ein- oder mehrfach substituiert ist, Oxazol-2-yl, Oxazol-4-yl, Oxazol-5-yl, wobei Oxazolyl unsubstituiert oder ein- oder mehrfach substituiert ist, Isooxazol-3-yl, Isooxazol-4-yl, Isooxazol-5-yl, wobei Isooxazolyl unsubstituiert oder ein- oder mehrfach substituiert ist, Indol-2-yl, Benzofuran-2-yl oder Benzofuran-3-yl bedeuten,
- R⁴, R⁵, R⁶ und R⁷: unabhängig voneinander H, Methyl, Ethyl, n-Propyl, 2-Propyl, n-Butyl, tert-Butyl, CF₃, F, Cl, Br, I, CO₂H, CO₂Methyl, CO₂Ethyl, C(=O)CH₃ oder NO₂ bedeuten oder R⁶ und R⁷ die Kohlenwasserstoffbrücke -CH=CH-CH=CH- bilden,
- R⁸: C(=O)CH₃ bedeutet, und
- R¹², R¹³ und R¹⁴: unabhängig voneinander C₁₋₆-Alkyl, wobei Alkyl geradkettig oder verzweigt ist und unsubstituiert oder ein- oder mehrfach substituiert ist, C₃₋₈-Cycloalkyl oder CH₂-C₃₋₈-Cycloalkyl, wobei Cycloalkyl unsubstituiert oder ein- oder mehrfach substituiert ist, oder Phenyl, wobei Phenyl unsubstituiert oder ein- oder mehrfach substituiert ist, bedeuten,
mit Ausnahme von Verbindungen der allgemeinen Struktur I, in denen zugleich
R¹ = tert.-Butyl,
R² = H,
R³ = C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert ist, Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,
X=CR⁴mit R⁴=H,
Y = CR⁵ mit R⁵ = CH₃,
R⁶ = H und
R⁷ = H oder C₁₋₄-Alkanyl, wobei Alkanyl geradkettig oder verzweigt und unsubstituiert oder ein- oder mehrfach substituiert ist, bedeuten,
wobei man im Zusammenhang mit "Alkyl" und "Alkanyl" der ausgenommenen Verbindungen unter dem Begriff "substituiert" die Substitution eines Wasserstoffrestes durch F, Cl, Br, I, -CN, NH₂, NH-Alkyl, NH-Aryl, NH-Heteroaryl, NH-Alkyl-Aryl, NH-Alkyl-Heteroaryl, NH-Heterocyclyl, NH-Alkyl-OH, N(Alkyl)₂, N(Alkyl-Aryl)₂, N(Alkyl-Heteroaryl)₂, N(Heterocyclyl)₂, N(Alkyl-OH)₂, NO, NO₂, SH, S-Alkyl, S-Aryl, S-Heteroaryl, S-Alkyl-Aryl, S-Alkyl-Heteroaryl, S-Heterocyclyl, S-Alkyl-OH, S-Alkyl-SH, OH, O-Alkyl, O-Aryl, O-Heteroaryl, O-Alkyl-Aryl, O-Alkyl-Heteroaryl, O-Heterocyclyl, O-Alkyl-OH, CHO, C(=O)C₁₋₆-Alkyl, C(=S)C₁₋₆-Alkyl, C(=O)Aryl, C(=S)Aryl, C(=O)C₁₋₆-Alkyl-Aryl, 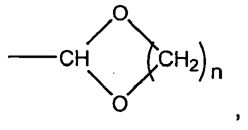 mit n = 1, 2 oder 3, C(=S)C₁₋₆-Alkyl-Aryl, C(=O)-Heteroaryl, C(=S)-Heteroaryl, C(=O)-Heterocyclyl, C(=S)-Heterocyclyl, CO₂H, CO₂-Alkyl, CO₂-Alkyl-Aryl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Alkyl-Heteroaryl)₂, C(=O)N(Heterocyclyl)₂, SO-Alkyl, SO₂-Alkyl, SO₂NH₂, SO₃H, Cycloalkyl, Aryl, Heteroaryl oder Heterocyclyl versteht, wobei unter mehrfach substituierten Resten solche Reste zu verstehen sind, die entweder an verschiedenen oder an gleichen Atomen mehrfach substituiert sind.

Weiter bevorzugt ist die erfindungsgemäße Verwendung von Verbindungen der allgemeinen Struktur I, in denen
- R¹: Methyl, Ethyl, n-Propyl, 2-Propyl, n-Butyl, tert-Butyl, n-Hexyl, n-Octyl, 1,1,3,3-Tetramethylbutyl, CH₂Aryl, wobei Aryl unsubstituiert oder ein- oder mehrfach substituiert ist, CH₂CO₂-C₁₋₆-Alkyl, wobei Alkyl geradkettig oder verzweigt ist, CH₂PO(O-C₁₋₆-Alkyl)₂, wobei Alkyl geradkettig oder verzweigt ist, CH₂SiR¹²R¹³R¹⁴, CH₂CH₂-Morpholin-4-yl, (CH₂)ₙ-NC mit n = 2, 3, 4, 5 oder 6, C₃₋₈-Cycloalkyl, wobei Cycloalkyl unsubstituiert oder ein- oder mehrfach substituiert ist, oder Phenyl, wobei Phenyl unsubstituiert oder ein- oder mehrfach substituiert ist, bedeutet,
- R²: H oder C(=O)-C₁₋₄-Alkyl bedeutet,
- R³: Methyl, Ethyl, n-Propyl, 2-Propyl, n-Butyl, tert-Butyl, Cyclopropyl, Cyclopentyl oder Cyclohexyl, die unabhängig voneinander unsubstituiert oder ein- oder mehrfach subsituiert sind, Phenyl, wobei Phenyl unsubstituiert oder einfach oder gleich oder verschieden mehrfach mit Methyl, Ethyl, n-Propyl, Prop-2-yl, n-Butyl, sec.-Butyl, tert-Butyl, iso-Butyl, CF₃, OH, OMethyl, OEthyl, F, Cl, Br, I, CN, NO₂, 4-Chlorphenoxy, Acetoxy, Dimethylamino substituiert ist, 1-Naphthyl oder 2-Naphthyl, wobei Naphthyl unsubstituiert oder einfach oder gleich oder verschieden mehrfach mit Methyl, Ethyl, n-Propyl, Prop-2-yl, n-Butyl, sec.-Butyl, tert-Butyl, iso-Butyl, CF₃, OH, OMethyl, OEthyl, F, Cl, Br, I, CN, NO₂, 4-Chlorphenoxy, Acetoxy, Dimethylamino substituiert ist, 9-Phenanthrenyl, Pyrrol-2-yl, Pyridin-2-yl, Pyridin-3-yl oder Pyridin-4-yl, wobei Pyridinyl unsubstituiert oder einfach oder gleich oder verschieden mehrfach mit Methyl, Ethyl, n-Propyl, Prop-2-yl, n-Butyl, sec.-Butyl, tert-Butyl, iso-Butyl, CF₃, OH, OMethyl, OEthyl, F, Cl, Br, I, CN, NO₂, 4-Chlorphenoxy, Acetoxy, Dimethylamino, Carboxy, Carboxymethyl, Carboxyethyl, Hydroxymethyl, Chlorphenyl, Nitrophenyl, [1,3]-Dioxolan, Methylsulfanyl substituiert ist, Furan-2-yl oder Furan-3-yl, wobei Furanyl unsubstituiert oder einfach oder gleich oder verschieden mehrfach mit Methyl, Ethyl, n-Propyl, Prop-2-yl, n-Butyl, sec.-Butyl, tert-Butyl, iso-Butyl, CF₃, OH, OMethyl, OEthyl, F, Cl, Br, I, CN, NO₂, 4-Chlorphenoxy, Acetoxy, Dimethylamino, Carboxy, Carboxymethyl, Carboxyethyl, Hydroxymethyl, Chlorphenyl, Nitrophenyl, [1,3]-Dioxolan, Methylsulfanyl substituiert ist, Thien-2-yl oder Thien-3-yl, wobei Thienyl unsubstituiert oder einfach oder gleich oder verschieden mehrfach mit Methyl, Ethyl, n-Propyl, Prop-2-yl, n-Butyl, sec.-Butyl, tert-Butyl, iso-Butyl, CF₃, OH, OMethyl, OEthyl, F, Cl, Br, I, CN, NO₂, 4-Chlorphenoxy, Acetoxy, Dimethylamino, Carboxy, Carboxymethyl, Carboxyethyl, Hydroxymethyl, Chlorphenyl, Nitrophenyl, [1,3]-Dioxolan, Methylsulfanyl substituiert ist, Indol-2-yl, Benzofuran-2-yl oder Benzofuran-3-yl bedeuten,
- R⁴, R⁵, R⁶ und R⁷: unabhängig voneinander H, Methyl, Ethyl, n-Propyl, 2-Propyl, n-Butyl, tert-Butyl, CF₃, F, Cl, Br, I, CO₂H, CO₂Methyl, CO₂Ethyl, C(=O)CH₃ oder NO₂ bedeuten oder R⁶ und R⁷ die Kohlenwasserstoffbrücke -CH=CH-CH=CH- bilden,
- R⁸: C(=O)CH₃ bedeutet, und
- R¹², R¹³ und R¹⁴: unabhängig voneinander C₁₋₆-Alkyl, wobei Alkyl geradkettig oder verzweigt ist und unsubstituiert oder ein- oder mehrfach substituiert ist, C₃₋₈-Cycloalkyl oder CH₂-C₃₋₈-Cycloalkyl, wobei Cycloalkyl unsubstituiert oder ein- oder mehrfach substituiert ist, oder Phenyl, wobei Phenyl unsubstituiert oder ein- oder mehrfach substituiert ist, bedeuten,
mit Ausnahme von Verbindungen der allgemeinen Struktur I, in denen zugleich
R¹ = tert.-Butyl,
R² = H,
R³ = C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert ist, Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,
X=CR⁴mit R⁴=H,
Y = CR⁵ mit R⁵ = CH₃,
R⁶ = H und
R⁷ = H oder C₁₋₄-Alkanyl, wobei Alkanyl geradkettig oder verzweigt und unsubstituiert oder ein- oder mehrfach substituiert ist, bedeuten,
wobei man im Zusammenhang mit "Alkyl" und "Alkanyl" der ausgenommenen Verbindungen unter dem Begriff "substituiert" die Substitution eines Wasserstoffrestes durch F, Cl, Br, I, -CN, NH₂, NH-Alkyl, NH-Aryl, NH-Heteroaryl, NH-Alkyl-Aryl, NH-Alkyl-Heteroaryl, NH-Heterocyclyl, NH-Alkyl-OH, N(Alkyl)₂, N(Alkyl-Aryl)₂, N(Alkyl-Heteroaryl)₂, N(Heterocyclyl)₂, N(Alkyl-OH)₂, NO, NO₂, SH, S-Alkyl, S-Aryl, S-Heteroaryl, S-Alkyl-Aryl, S-Alkyl-Heteroaryl, S-Heterocyclyl, S-Alkyl-OH, S-Alkyl-SH, OH, O-Alkyl, O-Aryl, O-Heteroaryl, O-Alkyl-Aryl, O-Alkyl-Heteroaryl, O-Heterocyclyl, O-Alkyl-OH, CHO, C(=O)C₁₋₆-Alkyl, C(=S)C₁₋₆-Alkyl, C(=O)Aryl, C(=S)Aryl, C(=O)C₁₋₆-Alkyl-Aryl, 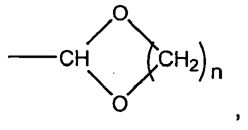 mit n = 1, 2 oder 3, C(=S)C₁₋₆-Alkyl-Aryl, C(=O)-Heteroaryl, C(=S)-Heteroaryl, C(=O)-Heterocyclyl, C(=S)-Heterocyclyl, CO₂H, CO₂-Alkyl, CO₂-Alkyl-Aryl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Alkyl-Heteroaryl)₂, C(=O)N(Heterocyclyl)₂, SO-Alkyl, SO₂-Alkyl, SO₂NH₂, SO₃H, Cycloalkyl, Aryl, Heteroaryl oder Heterocyclyl versteht, wobei unter mehrfach substituierten Resten solche Reste zu verstehen sind, die entweder an verschiedenen oder an gleichen Atomen mehrfach substituiert sind.

Besonders bevorzugt ist die erfindungsgemäße Verwendung von Verbindungen der allgemeinen Struktur I, in denen
- R¹: Methyl, n-Butyl, 1,1,3,3-Tetramethylbutyl, Benzyl, 2-Chlorbenzyl, 2-Methoxybenzyl, CH₂CO₂CH₃, (CH₂)₆-NC, Cyclopentyl, Cyclohexyl, Phenyl, 2,6-Dimethylphenyl, 3-Chlorphenyl oder 3-Chlor-4-fluorphenyl bedeutet,
- R²: H oder C(=O)CH₃ bedeutet,
- R³: Methyl, tert-Butyl, Cyclohexyl, Phenyl, 2-Methylphenyl, 3-Methylphenyl, 4-Methylphenyl, 2-Trifluormethylphenyl, 3-Trifluormethylphenyl, 4-Trifluormethylphenyl, 2-Hydroxyphenyl, 2-Methoxyphenyl, 3-Hydroxyphenyl, 3- Methoxyphenyl, 2-Fluorphenyl, 3-Fluorphenyl, 4-Fluorphenyl, 2-Chlorphenyl, 3-Chlorphenyl, 4-Chlorphenyl, 2-Bromphenyl, 3-Bromphenyl, 4-Bromphenyl, 2-Nitrophenyl, 3-Nitrophenyl, 4-Nitrophenyl, 3-(4-Chlorphenoxy)-phenyl, 2,4-Dimethylphenyl, 2,3-Dimethoxyphenyl, 3,4-Dimethoxyphenyl, 3-Methoxy-4-acetoxyphenyl, 2,3-Dichlorphenyl, 2,4-Dichlorphenyl, 2-Chlor-4-fluorphenyl, 2-Chlor-6-fluorphenyl, 4-Brom-2-fluorphenyl, 3,4,5-Trimethoxyphenyl, 1-Naphthyl, 2-Ethoxy-naphth-1-yl, 4-Dimethylamino-naphth-1-yl, 9-Phenanthrenyl, Pyrrol-2-yl, N-Methylpyrrol-2-yl, Pyridin-2-yl, Pyridin-3-yl, Pyridin-4-yl, Furan-2-yl, Furan-3-yl, 5-Methyl-furan-2-yl, 4,5-Dimethyl-furan-2-yl, 5-Hydroxymethyl-furan-2-yl, 5-Acetoxymethyl-furan-2-yl, 5-Carboxyfuran-2-yl, 5-[1,3]-Dioxolan-furan-2-yl, 3-Brom-furan-2-yl, 5-Brom-furan-2-yl, 5-Nitro-furan-2-yl, 5-(2-Nitrophenyl)-furan-2-yl, 5-(2-Chlorphenyl)-furan-2-yl, 5-(3-Chlorphenyl)-furan-2-yl, 5-(3-Chlorphenyl)-furan-3-yl, 5-(4-Chlorphenyl)-furan-2-yl, Benzo[b]furan-2-yl, Thien-2-yl, Thien-3-yl, 5-Methyl-thien-2-yl, 5-Carboxy-thien-2-yl, 3-Brom-thien-2-yl, 5-Chlor-thien-2-yl oder 5-Methylsulfanyl-thien-2-yl bedeutet,
- R⁴: H, CH₃, Cl, Br oder CO₂H bedeutet,
- R⁵: H, CH₃, C₂H₅ oder Cl bedeutet,
- R⁶: H, CH₃, Cl, Br oder NO₂,
- R⁷: H, CH₃ oder n-C₃H₇ bedeutet und
- R⁸: C(=O)CH₃ bedeutet.
Ganz besonders bevorzugt ist dabei die Verwendung von Verbindungen, in denen R⁴ und R⁶ H bedeuten, R⁵ H, CH₃ oder C₂H₅ bedeutet und R⁷ H oder CH₃ bedeutet.

Vorzugsweise sind die Verbindungen der allgemeinen Struktur I (in Form ihrer Basen oder ihrer pharmazeutisch annehmbaren Salze), die für die erfindungsgemäße Herstellung eines Medikaments zur NOS-Inhibierung, zur Behandlung von Migräne bzw. zur Behandlung von Multipler Sklerose, Morbus Parkinson, Morbus Alzheimer, Morbus Huntington, Entzündungsschmerz, cerebraler Ischämie, Diabetes, Meningitis, Arteriosklerose und/oder für die Wundheilung zu verwendet werden, aus der Gruppe ausgewählt, die enthält:
Cyclohexyl-(5,7-dimethyl-2-pyridin-4-yl-imidazo[1,2-a]pyridin-3-yl)-amin, (5,7-Dimethyl-2-pyridin-4-yl-imidazo[1,2-a]pyridin-3-yl)-(1,1,3,3-tetramethylbutyl)-amin,
{6-[5,7-Dimethyl-2-(1H-pyrrol-2-yl)-imidazo[1,2-a]pyridin-3-ylamino]-hexyl}methylidyne-ammonium,
[2-(3,4-Dimethoxy-phenyl)-7-methyl-imidazo[1,2-a]pyridin-3-yl]-(1,1,3,3-tetramethyl-butyl)-amin,
Cyclohexyl-(7-methyl-2-pyridin-4-yl-imidazo[1,2-a]pyridin-3-yl)-amin;
(2-Furan-2-yl-5,7-dimethyl-imidazo[1,2-a]pyridin-3-yl)-(1,1,3,3-tetramethyl-butyl)-amin,
(1,1,3,3-Tetramethyl-butyl)-(2,5,7-trimethyl-imidazo[1,2-a]pyridin-3-yl)-amin,
Cyclohexyl-(7-methyl-2-o-tolyl-imidazo[1,2-a]pyridin-3-yl)-amin,
Cyclohexyl-(7-methyl-2-thiophen-2-yl-imidazo[1,2-a]pyridin-3-yl)-amin,
(5,7-Dimethyl-2-pyridin-3-yl-imidazo[1,2-a]pyridin-3-yl)-(1,1,3,3-tetramethyl-butyl)-amin,
Cyclohexyl-[7-methyl-2-(2-trifluormethyl-phenyl)-imidazo[1,2-a]pyridin-3-yl]-amin,
(7-Methyl-2-pyridin-3-yl-imidazo[1,2-a]pyridin-3-yl)-(1,1,3,3-tetramethyl-butyl)-amin,
Cyclohexyl-(7-methyl-2-naphthalen-1-yl-imidazo[1,2-a]pyridin-3-yl)-amin,
[2-(2-Fluor-phenyl)-7-methyl-imidazo[1,2-a]pyridin-3-yl]-(1,1,3,3-tetramethyl-butyl)-amin,
(2,7-Dimethyl-imidazo[1,2-a]pyridin-3-ylamino)-essigsäuremethylester,
Methylidyne-[6-(7-methyl-2-pyridin-3-yl-imidazo[1,2-a]pyridin-3-ylamino)-hexyl]-ammonium,
Cyclohexyl-[2-(2-fluor-phenyl)-7-methyl-imidazo[1,2-a]pyridin-3-yl]-amin,
Cyclohexyl-(7-methyl-2-pyridin-3-yl-imidazo[1,2-a]pyridin-3-yl)-amin,
Cyclohexyl-(2-furan-2-yl-5,7-dimethyl-imidazo[1,2-a]pyridin-3-yl)-amin,
Cyclohexyl-(5,7-dimethyl-2-pyridin-3-yl-imidazo[1,2-a]pyridin-3-yl)-amin,
(2-Furan-2-yl-7-methyl-imidazo[1,2-a]pyridin-3-yl)-(1,1,3,3-tetramethyl-butyl)-amin,
(7-Methyl-2-pyridin-4-yl-imidazo[1,2-a]pyridin-3-yl)-(1,1,3,3-tetramethyl-butyl)-amin,
Butyl-(2-cyclohexyl-7-methyl-imidazo[1,2-a]pyridin-3-yl)-amin,
3-[5,7-Dimethyl-3-(1,1,3,3-tetramethyl-butylamino)-imidazo[1,2-a]pyridin-2-yl]-phenol,
(2,6-Dimethyl-phenyl)-(5,7-dimethyl-2-o-tolyl-imidazo[1,2-a]pyridin-3-yl)-amin,
(2,6-Dimethyl-phenyl)-[2-(2-fluor-phenyl)-5,7-dimethyl-imidazo[1,2-a]pyridin-3-yl]-amin,
Cyclohexyl-(2,5,7-trimethyl-imidazo[1,2-a]pyridin-3-yl)-amin,
[5,7-Dimethyl-2-(1H-pyrrol-2-yl)-imidazo[1,2-a]pyridin-3-yl]-(1,1,3,3-tetramethyl-butyl)-amin,
Butyl-(2-cyclohexyl-5,7-dimethyl-imidazo[1,2-a]pyridin-3-yl)-amin,
(5,7-Dimethyl-2-o-tolyl-imidazo[1,2-a]pyridin-3-yl)-(1,1,3,3-tetramethyl-butyl)-amin,
[2-(2,3-Dimethoxy-phenyl)-7-methyl-imidazo[1,2-a]pyridin-3-yl]-(2,6-dimethyl-phenyl)-amin,
(2,7-Dimethyl-imidazo[1,2-a]pyridin-3-yl)-(1,1,3,3-tetramethyl-butyl)-amin,
[2-(5-[1,3]Dioxolan-2-yl-furan-2-yl)-7-methyl-imidazo[1,2-a]pyridin-3-yl]-(1,1,3,3-tetramethyl-butyl)-amin,
[2-(3-Brom-thiophen-2-yl)-5,7-dimethyl-imidazo[1,2-a]pyridin-3-yl]-(2,6-dimethyl-phenyl)-amin,
(2,6-Dimethyl-phenyl)-[2-(2-fluor-phenyl)-7-methyl-imidazo[1,2-a]pyridin-3-yl]-amin,
(2-Cyclohexyl-5,7-dimethyl-imidazo[1,2-a]pyridin-3-yl)-(1,1,3,3-tetramethyl-butyl)-amin,
[6-(2-Furan-2-yl-5,7-dimethyl-imidazo[1,2-a]pyridin-3-ylamino)-hexyl]-methylidyne-ammonium,
(7-Methyl-2-o-tolyl-imidazo[1,2-a]pyridin-3-yl)-(1,1,3,3-tetramethyl-butyl)-amin,
[2-(2,3-Dichlor-phenyl)-7-methyl-imidazo[1,2-a]pyridin-3-yl]-(2,6-dimethyl-phenyl)-amin,
[2-(2,3-Dimethoxy-phenyl)-5,7-dimethyl-imidazo[1,2-a]pyridin-3-yl]-(2,6-dimethyl-phenyl)-amin,
Butyl-[2-(2,3-dimethoxy-phenyl)-5,7-dimethyl-imidazo[1,2-a]pyridin-3-yl]-amin,
Methylidyne-[6-(7-methyl-2-p-tolyl-imidazo[1,2-a]pyridin-3-ylamino)-hexyl]-ammonium,
Essigsäure 5-(3-cyclohexylamino-5,7-dimethyl-imidaza[1,2-a]pyridin-2-yl)-furan-2-ylmethylester,
[2-(2-Methoxy-phenyl)-5,7-dimethyl-imidazo[1,2-a]pyridin-3-yl]-(1,1,3,3-tetramethyl-butyl)-amin,
[2-(3,4-Dimethoxy-phenyl)-7-methyl-imidazo[1,2-a]pyridin-3-yl]-(2,6-dimethyl-phenyl)-amin,
3-(3-Butylamino-7-methyl-imidazo[1,2-a]pyridin-2-yl)-phenol,
(2-Benzofuran-2-yl-7-methyl-imidazo[1,2-a]pyridin-3-yl)-(1,1,3,3-tetramethyl-butyl)-amin,
(2-Benzofuran-2-yl-5,7-dimethyl-imidazo[1,2-a]pyridin-3-yl)-(2,6-dimethyl-phenyl)-amin,
Essigsäure 5-(3-cyclohexylamino-7-methyl-imidazo[1,2-a]pyridin-2-yl)-furan-2-ylmethylester,
[6-(5,7-Dimethyl-2-pyridin-3-yl-imidazo[1,2-a]pyridin-3-ylamino)-hexyl]-methylidyne-ammonium,
Butyl-[2-(2-methoxy-phenyl)-7-methyl-imidazo[1,2-a]pyridin-3-yl]-amin,
{6-[2-(2-Methoxy-phenyl)-5,7-dimethyl-imidazo[1,2-a]pyridin-3-ylamino]-hexyl}-methylidyne-ammonium,
{5-[5,7-Dimethyl-3-(1,1,3,3-tetramethyl-butylamino)-imidazo[1,2-a]pyridin-2-yl]-furan-2-yl}-methanol,
(7-Methyl-2-naphthalen-1-yl-imidazo[1,2-a]pyridin-3-yl)-(1,1,3,3-tetramethyl-butyl)-amin,
(2-Benzofuran-2-yl-5,7-dimethyl-imidazo[1,2-a]pyridin-3-yl)-(1,1,3,3-tetramethyl-butyl)-amin,
Cyclohexyl-(2,7-dimethyl-imidazo[1,2-a]pyridin-3-yl)-amin,
[2-(2,3-Dichlorphenyl)-8-methyl-imidazo[1,2-a]pyridin-3-yl]-(1,1,3,3-tetramethyl-butyl)-amin,
(7-Methyl-2-p-tolyl-imidazo[1,2-a]pyridin-3-yl)-(1,1,3,3-tetramethyl-butyl)-amin,
(2,6-Dimethyl-phenyl)-[2-(2-methoxy-phenyl)-7-methyl-imidazo[1,2-a]pyridin-3-yl]-amin,
3-(3-Butylamino-5,7-dimethyl-imidazo[1,2-a]pyridin-2-yl)-phenol,
Butyl-[2-(2,3-dimethoxy-phenyl)-7-methyl-imidazo[1,2-a]pyridin-3-yl]-amin,
{6-[5,7-Dimethyl-2-(2-trifluormethyl-phenyl)-imidazo[1,2-a]pyridin-3-ylamino]-hexyl}-methylidyne-ammonium,
Cyclohexyl-(2-cyclohexyl-5,7-dimethyl-imidazo[1,2-a]pyridin-3-yl)-amin,
[2-(2,3-Dichlorphenyl)-5,7-dimethyl-imidazo[1,2-a]pyridin-3-yl]-(2,6-dimethyl-phenyl)-amin,
(2,6-Dimethyl-phenyl)-[2-(2-methoxy-phenyl)-5,7-dimethyl-imidazo[1,2-a]pyridin-3-y)]-amin,
{2-[5-(2-Chlorphenyl)-furan-2-yl]-7-methyl-imidazo[1,2-a]pyridin-3-yl}-(1,1,3,3-tetramethyl-butyl)-amin,
5-[7-Methyl-3-(1,1,3,3-tetramethyl-butylamino)-imidazo[1,2-a]pyridin-2-yl]-furan-2-carbonsäure,
Cyclohexyl-[2-(2-methoxy-phenyl)-8-methyl-imidazo[1,2-a]pyridin-3-yl]-amin,
3-[7-Methyl-3-(1,1,3,3-tetramethyl-butylamino)-imidazo[1,2-a]pyridin-2-yl]-phenol,
[2-(2,3-Dichlorphenyl)-5,7-dimethyl-imidazo[1,2-a]pyridin-3-yl]-(1,1,3,3-tetramethyl-butyl)-amin,
[2-(2,4-Dichlorphenyl)-7-methyl-imidazo[1,2-a]pyridin-3-yl]-(1,1,3,3-tetramethyl-butyl)-amin,
[2-(5-Bromfuran-2-yl)-7-methyl-imidazo[1,2-a]pyridin-3-yl]-(1,1,3,3-tetramethyl-butyl)-amin,
5-(3-Cyclohexylamino-5,7-dimethyl-imidazo[1,2-a]pyridin-2-yl)-furan-2-carbonsäure,
[6-(2-Cyclohexyl-5,7-dimethyl-imidazo[1,2-a]pyridin-3-ylamino)-hexyl]-methylidyne-ammonium,
[2-(2,4-Dichlorphenyl)-5,7-dimethyl-imidazo[1,2-a]pyridin-3-yl]-(1,1,3,3-tetramethyl-butyl)-amin,
(2-Benzofuran-2-yl-7-methyl-imidazo[1,2-a]pyridin-3-yl)-(2,6-dimethyl-phenyl)-amin,
5-(3-Cyclohexylamino-7-methyl-imidazo[1,2-a]pyridin-2-yl)-furan-2-carbonsäure,
{6-[2-(2-Bromphenyl)-5,7-dimethyl-imidazo[1,2-a]pyridin-3-ylamino]-hexyl}methylidyne-ammonium,
(5,7-Dimethyl-2-p-tolyl-imidazo[1,2-a]pyridin-3-yl)-(1,1,3,3-tetramethyl-butyl)-amin,
[2-(2,3-Dichlorphenyl)-8-methyl-imidazo[1,2-a]pyridin-3-yl]-(2,6-dimethyl-phenyl)-amin,
Methylidyne-[6-(7-methyl-2-o-tolyl-imidazo[1,2-a]pyridin-3-ylamino)-hexyl]-ammonium,
{2-[5-(3-Chlorphenyl)-furan-2-yl]-7-methyl-imidazo[1,2-a]pyridin-3-yl}-(1,1,3,3-tetramethyl-butyl)-amin,
Cyclohexyl-[7-methyl-2-(5-nitro-furan-2-yl)-imidazo[1,2-a]pyridin-3-yl]-amin,
[2-(2-Bromphenyl)-7-methyl-imidazo[1,2-a]pyridin-3-yl]-cycloheoyl-amin,
[2-(2-Methoxy-phenyl)-7-methyl-imidazo[1,2-a]pyridin-3-yl]-(1,1,3,3-tetramethyl-butyl)-amin,
{5-[7-Methyl-3-(1,1,3,3-tetramethyl-butylamino)-imidazo[1,2-a]pyridin-2-yl]-furan-2-yl}-methanol.
(6-{2-[5-(2-Chlorphenyl)-furan-2-yl]-5-methyl-imidazo[1,2-a]pyridin-3-ylamino}-hexyl)-methylidyne-ammonium,
Cyclohexyl-[5,7-dimethyl-2-(5-nitro-furan-2-yl)-imidazo[1,2-a]pyridin-3-yl]-amin,
Cyclohexyl-[2-(4,5-dimethyl-furan-2-yl)-7-methyl-imidazo[1,2-a]pyridin-3-yl]-amin,
[6-(5,7-Dimethyl-2-o-tolyl-imidazo[1,2-a]pyridin-3-ylamino)-hexyl]-methylidyne-ammonium,
Methylidyne-[6-(7-methyl-2-pyridin-2-yl-imidazo[1,2-a]pyridin-3-ylamino)-hexyl]-ammonium,
[2-(2,3-Dimethoxy-phenyl)-5,7-dimethyl-imidazo[1,2-a]pyridin-3-yl]-(1,1,3,3-tetramethyl-butyl)-amin,
{6-[2-(3,4-Dimethoxy-phenyl)-7-methyl-imidazo[1,2-a]pyridin-3-ylamino]-hexyl}-methylidyne-ammonium,
Cyclohexyl-(8-methyl-2-pyridin-4-yl-imidazo[1,2-a]pyridin-3-yl)-amin,
[2-(2,3-Dichlor-phenyl)-6-methyl-imidazo[1,2-a]pyridin-3-yl]-(1,1,3,3-tetramethyl-butyl)-amin,
5-(3-Butylamino-imidazo[1,2-a]pyrazin-2-yl)-thiophen-2-carbonsäure,
Cyclohexyl-(5,7-dimethyl-2-pyridin-2-yl-imidazo[1,2-a]pyridin-3-yl)-amin,
(2-Benzofuran-2-yl-8-methyl-imidazo[1,2-a]pyridin-3-yl)-(1,1,3,3-tetramethyl-butyl)-amin,
{6-[2-(2-Fluor-phenyl)-5,7-dimethyl-imidazo[1,2-a]pyridin-3-ylamino]-hexyl}methylidyne-ammonium,
[2-(2,3-Dimethoxy-phenyl)-8-methyl-imidazo[1,2-a]pyridin-3-yl]-(1,1,3,3-tetramethyl-butyl)-amin,
Methylidyne-[6-(7-methyl-2-phenanthren-9-yl-imidazo[1,2-a]pyridin-3-ylamino)-hexyl]-ammonium,
Cyclohexyl-(2-furan-2-yl-8-methyl-imidazo[1,2-a]pyridin-3-yl)-amin,
Methylidyne-[6-(7-methyl-2-naphthalen-1-yl-imidazo[1,2-a]pyridin-3-ylamino)-hexyl]-ammonium,
(6-Methyl-2-pyridin-4-yl-imidazo[1,2-a]pyridin-3-yl)-(1,1,3,3-tetramethylbutyl)-amin,
(7-Methyl-2-pyridin-3-yl-imidazo[1,2-a]pyrimidin-3-yl)-(1,1,3,3-tetramethyl-butyl)-amin,
[6-(5,7-Dimethyl-2-naphthalen-1-yl-imidazo[1,2-a]pyridin-3-ylamino)-hexyl]-methylidyne-ammonium,
3-[3-(2,6-Dimethyl-phenylamino)-5,7-dimethyl-imidazo[1,2-a]pyridin-2-yl]-phenol,
(2,6-Dimethyl-phenyl)-(8-methyl-2-o-tolyl-imidazo[1,2-a]pyridin-3-yl)-amin,
{6-[2-(3-Hydroxy-phenyl)-8-methyl-imidazo[1,2-a]pyridin-3-ylamino]-hexyl}methylidyne-ammonium,
{5-[3-(2,6-Dimethyl-phenylamino)-7-methyl-imidazo[1,2-a]pyrimidin-2-yl]-furan-2-yl}-methanol,
(8-Methyl-2-p-tolyl-imidazo[1,2-a]pyridin-3-yl)-(1,1,3,3-tetramethyl-butyl)-amin,
[2-(2,4-Dichlorphenyl)-6-methyl-imidazo[1,2-a]pyridin-3-yl]-(2,6-dimethyl-phenyl)-amin,
Butyl-[2-(2,4-dichlor-phenyl)-6-methyl-imidazo[1,2-a]pyridin-3-yl]-amin,
Butyl-[2-(4-dimethylamino-naphthalen-1-yl)-imidazo[1,2-a]pyrazin-3-yl]-amin,
{6-[2-(2-Brom-phenyl)-7-methyl-imidazo[1,2-a]pyridin-3-ylamino]-hexyl}methylidyne-ammonium,
Butyl-[2-(2-methoxy-phenyl)-6-methyl-imidazo[1,2-a]pyridin-3-yl]-amin,
(2-Cyclohexyl-8-methyl-imidazo[1,2-a]pyridin-3-yl)-(1,1,3,3-tetramethyl-butyl)-amin,
Cyclohexyl-(7-methyl-2-pyridin-2-yl-imidazo[1,2-a]pyridin-3-yl)-amin,
Cyclohexyl-(2-furan-2-yl-7-methyl-imidazo[1,2-a]pyridin-3-yl)-amin,
(2-Cyclohexyl-5,7-dimethyl-imidazo[1,2-a]pyridin-3-ylamino)-essigsäuremethylester,
N-(2-Furan-2-yl-5,7-dimethyl-imidazo[1,2-a]pyridin-3-yl)-N-(1,1,3,3-tetramethyl-butyl)-acetamid,
N-*tert*-Butyl-N-(7-methyl-2-pyridin-3-yl-imidazo[1,2-a]pyridin-3-yl)-acetamid,
N-*tert*-Butyl-N-(2-furan-2-yl-5,7-dimethyl-imidazo[1,2-a]pyridin-3-yl)-acetamid
N-(5,7-Dimethyl-2-pyridin-4-yl-imidazo[1,2-a]pyridin-3-yl)-N-(1,1,3,3-tetramethyl-butyl)-acetamid,
N-(5,7-Dimethyl-2-pyridin-3-yl-imidazo[1,2-a]pyridin-3-yl)-N-(1,1,3,3-tetramethyl-butyl)-acetamid,
N-(2,6-Dimethyl-phenyl)-N-(5,7-dimethyl-2-o-tolyl-imidazo[1,2-a]pyridin-3-yl)-acetamid,
N-(2-Furan-2-yl-7-methyl-imidazo[1,2-a]pyridin-3-yl)-N-(1,1,3,3-tetramethyl-butyl)-acetamid,
N-(1,1,3,3-Tetramethyl-butyl)-N-(2,5,7-trimethyl-imidazo[1,2-a]pyridin-3-yl)-acetamid,
N-Cyclohexyl-N-(7-methyl-2-thiophen-2-yl-imidazo[1,2-a]pyridin-3-yl)-acetamid,
N-*tert*-Butyl-N-(2,5,7-trimethyl-imidazo[1,2-a]pyridin-3-yl)-acetamid,
5-[3-(Acetyl-*tert*-butyl-amino)-imidazo[1,2-a]pyrazin-2-yl]-thiophen-2-carbonsäure,
5-{3-[Acetyl-(1,1,3,3-tetramethyl-butyl)-amino]-5,7-dimethyl-imidazo[1,2-a]pyridin-2-yl}-furan-2-carbonsäure,
N-[2-(5-Hydroxymethyl-furan-2-yl)-5,7-dimethyl-imidazo[1,2-a]pyridin-3-yl]-N-(1,1,3,3-tetramethyl-butyl)-acetamid,
N-[2-(3-Brom-thiophen-2-yl)-5,7-dimethyl-imidazo[1,2-a]pyridin-3-yl]-N-(2,6-dimethyl-phenyl)-acetamid,
N-*tert*-Butyl-N-(2-cyclohexyl-5,7-dimethyl-imidazo[1,2-a]pyridin-3-yl)-acetamid,
Essigsäure 5-[3-(acetyl-cyclohexyl-amino)-5,7-dimethyl-imidazo[1,2-a]pyridin-2-yl]-furan-2-ylmethyl ester,
{6-[Acetyl-(2-furan-2-yl-5,7-dimethyl-imidazo[1,2-a]pyridin-3-yl)-amino]-hexyl}-methylidyne-ammonium,
N-[2-(2,3-Dichlor-phenyl)-7-methyl-imidazo[1,2-a]pyridin-3-yl]-N-(2,6-dimethyl-phenyl)-acetamid,
N-[2-(3-Brom-thiophen-2-yl)-imidazo[1,2-a]pyridin-3-yl]-N-cyclohexylacetamid,
N-(5,7-Dimethyl-2-p-tolyl-imidazo[1,2-a]pyridin-3-yl)-N-(1,1,3,3-tetramethyl-butyl)-acetamid,
N-Cyclohexyl-N-(7-methyl-2-pyridin-4-yl-imidazo[1,2-a]pyridin-3-yl)-acetamid,
N-Cyclohexyl-N-[7-methyl-2-(2-trifluormethyl-phenyl)-imidazo[1,2-a]pyridin-3-yl]-acetamid,
N-(6,8-Dibrom-2-furan-2-yl-imidazo[1,2-a]pyridin-3-yl)-N-(1,1,3,3-tetramethyl-butyl)-acetamid,
N-(7-Methyl-2-pyridin-4-yl-imidazo[1,2-a]pyridin-3-yl)-N-(1,1,3,3-tetramethyl-butyl)-acetamid,
Essigsäure-5-[3-(acetyl-cyclohexyl-amino)-7-methyl-imidazo[1,2-a]pyridin-2-yl]-furan-2-ylmethylester,
N-(7-Methyl-2-pyridin-3-yl-imidazo[1,2-a]pyridin-3-yl)-N-(1,1,3,3-tetramethyl-butyl)-acetamid,
N-[2-(2,3-Dichlor-phenyl)-5,7-dimethyl-imidazo[1,2-a]pyridin-3-yl]-N-(2,6-dimethyl-phenyl)-acetamid,
N-Cyclohexyl-N-[5,7-dimethyl-2-(5-methyl-furan-2-yl)-imidazo[1,2-a]pyridin-3-yl]-acetamid,
N-Butyl-N-[2-(2,3-dimethoxy-phenyl)-5,7-dimethyl-imidazo[1,2-a]pyridin-3-yl]-acetamid,
N-[2-(2-Methoxy-phenyl)-5,7-dimethyl-imidazo[1,2-a]pyridin-3-yl]-N-(1,1,3,3-tetramethyl-butyl)-acetamid,
N-Cyclohexyl-N-[5,7-dimethyl-2-(5-nitro-furan-2-yl)-imidazo[1,2-a]pyridin-3-yl]-acetamid,
[Acetyl-(2,7-dimethyl-imidazo[1,2-a]pyridin-3-yl)-amino]-essigsäuremethylester,
N-Cyclohexyl-N-(2,5,7-trimethyl-imidazo[1,2-a]pyridin-3-yl)-acetamid,
5-{3-[Acetyl-(1,1,3,3-tetramethyl-butyl)-amino]-imidazo[1,2-a]pyridin-2-yl}thiophen-2-carbonsäure,
N-[2-(2,4-Dichlor-phenyl)-5,7-dimethyl-imidazo[1,2-a]pyridin-3-yl]-N-(1,1,3,3-tetramethyl-butyl)-acetamid,
N-Cyclohexyl-N-[7-methyl-2-(5-nitro-furan-2-yl)-imidazo[1,2-a]pyridin-3-yl]-acetamid,
N-(2-*tert*-Butyl-5,7-dimethyl-imidazo[1,2-a]pyridin-3-yl)-N-(2,6-dimethyl-phenyl)-acetamid,
N-(2,6-Dimethyl-phenyl)-N-[2-(2-methoxy-phenyl)-5,7-dimethyl-imidazo[1,2-a]pyridin-3-yl]-acetamid,
N-[2-(3-Hydroxy-phenyl)-5,7-dimethyl-imidazo[1,2-a]pyridin-3-yl]-N-(1,1,3,3-tetramethyl-butyl)-acetamid,
N-(2,6-Dimethyl-phenyl)-N-[2-(2-fluor-phenyl)-5,7-dimethyl-imidazo[1,2-a]pyridin-3-yl]-acetamid,
5-[3-(Acetyl-*tert*-butyl-amino)-5-methyl-imidazo[1,2-a]pyridin-2-yl]-thiophen-2-carbonsäure,
N-(2,6-Dimethyl-phenyl)-N-[2-(2-methoxy-phenyl)-7-methyl-imidazo[1,2-a]pyridin-3-yl]-acetamid,
N-(7-Methyl-2-p-tolyl-imidazo[1,2-a]pyridin-3-yl)-N-(1,1,3,3-tetramethyl-butyl)-acetamid,
5-{3-[Acetyl-(1,1,3,3-tetramethyl-butyl)-amino]-7-methyl-imidazo[1,2-a]pyridin-2-yl}-furan-2-carbonsäure,
N-Cyclohexyl-N-(7-methyl-2-pyridin-3-yl-imidazo[1,2-a]pyridin-3-yl)-acetamid,
N-[2-(5-[1,3]Dioxolan-2-yl-furan-2-yl)-5,7-dimethyl-imidazo[1,2-a]pyridin-3-yl]-N-(1,1,3,3-tetramethyl-butyl)-acetamid,
N-(2-Benzofuran-2-yl-5,7-dimethyl-imidazo[1,2-a]pyridin-3-yl)-N-(2,6-dimethyl-phenyl)-acetamid,
N-*tert*-Butyl-N-(2-furan-2-yl-7-methyl-imidazo[1,2-a]pyridin-3-yl)-acetamid,
N-*tert*-Butyl-N-(7-methyl-2-pyridin-2-yl-imidazo[1,2-a]pyridin-3-yl)-acetamid,
N-Cyclohexyl-N-(5,7-dimethyl-2-pyridin-3-yl-imidazo[1,2-a]pyridin-3-yl)-acetamid,
N-[2-(2,3-Dichlor-phenyl)-5,7-dimethyl-imidazo[1,2-a]pyridin-3-yl]-N-(1,1,3,3-tetramethyl-butyl)-acetamid,
N-[2-(2,3-Dimethoxy-phenyl)-5,7-dimethyl-imidazo[1,2-a]pyridin-3-yl]-N-(1,1,3,3-tetramethyl-butyl)-acetamid,
N-{2-[3-(4-chlor-phenoxy)-phenyl]-imidazo[1,2-a]pyridin-3-yl}-N-(2,6-dimethyl-phenyl)-acetamid,
N-[2-(5-[1,3]Dioxolan-2-yl-furan-2-yl)-7-methyl-imidazo[1,2-a]pyridin-3-yl]-N-(1,1,3,3-tetramethyl-butyl)-acetamid,
5-[3-(Acetyl-cyclohexyl-amino)-5,7-dimethyl-imidazo[1,2-a]pyridin-2-yl]-furan-2-carbonsäure,
N-*tert*-Butyl-N-[7-methyl-2-(5-nitro-furan-2-yl)-imidazo[1,2-a]pyridin-3-yl]-acetamid,
N-[2-(2-Methoxy-phenyl)-7-methyl-imidazo[1,2-a]pyridin-3-yl]-N-(1,1,3,3-tetramethyl-butyl)-acetamid,
N-[2-(5-Methyl-furan-2-yl)-imidazo[1,2-a]pyridin-3-yl]-N-(1,1,3,3-tetramethyl-butyl)-acetamid,
5-[3-(Acetyl-*tert*-butyl-amino)-7-methyl-imidazo[1,2-a]pyridin-2-yl]-furan-2-carbonsäure,
N-[2-(4,5-Dimethyl-furan-2-yl)-imidazo[1,2-a]pyridin-3-yl]-N-(1,1,3,3-tetramethyl-butyl)-acetamid,
N-Cyclohexyl-N-(2-furan-2-yl-5,7-dimethyl-imidazo[1,2-a]pyridin-3-yl)-acetamid,
N-*tert*-Butyl-N-(7-methyl-2-naphthalen-1-yl-imidazo[1,2-a]pyridin-3-yl)-acetamid,
5-{3-[Acetyl-(1,1,3,3-tetramethyl-butyl)-amino]-imidazo[1,2-a]pyrazin-2-yl}thiophen-2-carbonsäure,
N-Butyl-N-(2-o-tolyl-imidazo[1,2-a]pyrimidin-3-yl)-acetamid,
N-[2-(3,4-Dimethoxy-phenyl)-7-methyl-imidazo[1,2-a]pyridin-3-yl]-N-(2,6-dimethyl-phenyl)-acetamid,
N-*tert*-Butyl-N-(7-methyl-2-phenanthren-9-yl-imidazo[1,2-a]pyridin-3-yl)-acetamid,
N-(2,6-Dimethyl-phenyl)-N-[2-(2-fluor-phenyl)-7-methyl-imidazo[1,2-a]pyridin-3-yl]-acetamid,
N-[2-(2-Methoxy-phenyl)-8-methyl-imidazo[1,2-a]pyridin-3-yl]-N-(1,1,3,3-tetramethyl-butyl)-acetamid,
N-(2,6-Dimethyl-phenyl)-N-[2-(3-hydroxy-phenyl)-5,7-dimethyl-imidazo[1,2-a]pyridin-3-yl]-acetamid,
N-(2-*tert*-Butyl-7-methyl-imidazo[1,2-a]pyridin-3-yl)-N-(2,6-dimethyl-phenyl)-acetamid,
Essigsäure-4-{3-[acetyl-(2,6-dimethyl-phenyl)-amino]-6-brom-8-methylimidazo[1,2-a]pyridin-2-yl}-2-methoxy-phenylester,
N-*tert*-Butyl-N-[2-(5-[1,3]dioxolan-2-yl-furan-2-yl)-7-methyl-imidazo[1,2-a]pyridin-3-yl]-acetamid,
[6-(Acetyl-{7-methyl-2-[5-(2-nitro-phenyl)-furan-2-yl]-imidazo[1,2-a]pyridin-3-yl}-amino)-hexyl]-methylidyne-ammonium,
N-(2-Benzofuran-2-yl-7-methyl-imidazo[1,2-a]pyridin-3-yl)-N-(1,1,3,3-tetramethyl-butyl)-acetamid,
N-(2-Benzofuran-2-yl-5,7-dimethyl-imidazo[1,2-a]pyridin-3-yl)-N-(1,1,3,3-tetramethyl-butyl)-acetamid,
5-[3-(Acetyl-*tert*-butyl-amino)-imidazo[1,2-a]pyridin-2-yl]-thiophen-2-carbonsäure,
N-(2-Cyclohexyl-5,7-dimethyl-imidazo[1,2-a]pyridin-3-yl)-N-(1,1,3,3-tetramethyl-butyl)-acetamid,
N-*tert*-Butyl-N-[2-(5-methyl-furan-2-yl)-imidazo[1,2-a]pyridin-3-yl]-acetamid,
N-*tert*-Butyl-N-[2-(5-methylsulfanyl-thiophen-2-yl)-imidazo[1,2-a]pyrimidin-3-yl]-acetamid,
N-[2-(4,5-Dimethyl-furan-2-yl)-5-methyl-imidazo[1,2-a]pyridin-3-yl]-N-(1,1,3,3-tetramethyl-butyl)-acetamid,
N-Butyl-N-[2-(2,4-dichlor-phenyl)-imidazo[1,2-a]pyrimidin-3-yl]-acetamid,
N-[2-(3-Brom-thiophen-2-yl)-6-methyl-imidazo[1,2-a]pyridin-3-yl]-N-cyclohexyl-acetamid,
5-{3-[Acetyl-(2,6-dimethyl-phenyl)-amino]-6-methyl-imidazo[1,2-a]pyridin-2-yl}-thiophen-2-carbonsäure,
N-Butyl-N-[2-(2,3-dimethoxy-phenyl)-7-methyl-imidazo[1,2-a]pyridin-3-yl]-acetamid,
N-*tert*-Butyl-N-[2-(2,3-dichlor-phenyl)-imidazo[1,2-a]pyridin-3-yl]-acetamid,
N-(2-Furan-2-yl-5-propyl-imidazo[1,2-a]pyridin-3-yl)-N-(1,1,3,3-tetramethyl-butyl)-acetamid,
5-[3-(Acetyl-cyclohexyl-amino)-imidazo[1,2-a]pyridin-2-yl]-thiophen-2-carbonsäure,
5-{3-[Acetyl-(1,1,3,3-tetramethyl-butyl)-amino]-8-methyl-imidazo[1,2-a]pyridin-2-yl}-furan-2-carbonsäure,
3-(Acetyl-butyl-amino)-2-pyridin-2-yl-imidazo[1,2-a]pyridin-8-carbonsäure,
{6-[Acetyl-(5,7-dimethyl-2-pyridin-3-yl-imidazo[1,2-a]pyridin-3-yl)-amino]-hexyl}-methylidyne-ammoium,
N-*tert*-Butyl-N-[2-(5-methylsulfanyl-thiophen-2-yl)-imidazo[1,2-a]pyrazin-3-yl]-acetamid,
5-[3-(Acetyl-cyclohexyl-amino)-5-methyl-imidazo[1,2-a]pyridin-2-yl]-thiophen-2-carbonsäure,
N-[2-(5-Methylsulfanyl-thiophen-2-yl)-imidazo[1,2-a]pyrazin-3-yl]-N-(1,1,3,3-tetramethyl-butyl)-acetamid,
N-[2-(2,3-Dichlor-phenyl)-5-methyl-imidazo[1,2-a]pyridin-3-yl]-N-(2,6-dimethyl-phenyl)-acetamid,
N-Butyl-N-[2-(2-methoxy-phenyl)-imidazo[1,2-a]pyridin-3-yl]-acetamid,
(6-{Acetyl-[2-(2-methoxy-phenyl)-6-nitro-imidazo[1,2-a]pyridin-3-yl]-amino}hexyl)-methylidyne-ammonium,
N-(2-Benzofuran-2-yl-7-methyl-imidazo[1,2-a]pyridin-3-yl)-N-(2,6-dimethyl-phenyl)-acetamid,
(6-{Acetyl-[2-(2-methoxy-phenyl)-5,7-dimethyl-imidazo[1,2-a]pyridin-3-yl]-amino}-hexyl)-methylidyne-ammonium,
{6-[Acetyl-(7-methyl-2-pyridin-3-yl-imidazo[1,2-a]pyridin-3-yl)-amino]-hexyl}methylidyne-ammonium,
N-(6-Methyl-2-p-tolyl-imidazo[1,2-a]pyridin-3-yl)-N-(1,1,3,3-tetramethyl-butyl)-acetamid,
Essigsäure-5-{3-[acetyl-(2,6-dimethyl-phenyl)-amino]-5,7-dimethylimidazo[1,2-a]pyridin-2-yl}-furan-2-ylmethylester,
{Acetyl-[2-(3-hydroxy-phenyl)-6-methyl-imidazo[1,2-a]pyridin-3-yl]-amino}essigsäuremethylester,
N-*tert*-Butyl-N-[2-(2-trifluormethyl-phenyl)-imidazo[1,2-a]pyridin-3-yl]-acetamid,
N-Butyl-N-[2-(2-chlor-4-fluor-phenyl)-imidazo[1,2-a]pyridin-3-yl]-acetamid,
N-[2-(2,4-Dichlor-phenyl)-imidazo[1,2-a]pyridin-3-yl]-N-(2,6-dimethyl-phenyl)-acetamid,
5-{3-[Acetyl-(1,1,3,3-tetramethyl-butyl)-amino]-7-methyl-imidazo[1,2-a]pyrimidin-2-yl}-furan-2-carbonsäure,
Essigsäure-5-{3-[acetyl-(1,1,3,3-tetramethyl-butyl)-amino]-imidazo[1,2-a]pyrimidin-2-yl}-furan-2-ylmethylester,
N-(2,7-Dimethyl-imidazo[1,2-a]pyridin-3-yl)-N-(1,1,3,3-tetramethyl-butyl)-acetamid,
Essigsäure-4-[3-(acetyl-cyclohexyl-amino)-5-amino-7-chlor-imidazo[1,2-a]pyrimidin-2-yl]-2-methoxy-phenylester,
Essigsäure-4-[3-(acetyl-cyclohexyl-amino)-5,7-dimethyl-imidazo[1,2-a]pyrimidin-2-yl]-2-methoxy-phenylester,
N-[6-Brom-2-(2-chlor-6-fluor-phenyl)-8-methyl-imidazo[1,2-a]pyridin-3-yl]-N-cyclohexyl-acetamid,
. N-[2-(2-chlor-6-fluor-phenyl)-5-methyl-imidazo[1,2-a]pyridin-3-yl]-N-cyclohexyl-acetamid,
N-Butyl-N-[2-(2,3-dichlor-phenyl)-imidazo[1,2-a]pyrimidin-3-yl]-acetamid,
N-[2-(5-chlor-thiophen-2-yl)-imidazo[1,2-a]pyrimidin-3-yl]-N-(1,1,3,3-tetramethyl-butyl)-acetamid,
[Acetyl-(2-cyclohexyl-7-methyl-imidazo[1,2-a]pyridin-3-yl)-amino]-essigsäuremethylester,
N-*tert*-Butyl-N-[2-(2-chlor-6-fluor-phenyl)-imidazo[1,2-a]pyridin-3-yl]-acetamid,
N-Cyclohexyl-N-(5,7-dimethyl-2-pyridin-2-yl-imidazo[1,2-a]pyridin-3-yl)-acetamid,
Essigsäure-5-[3-(acetyl-cyclohexyl-amino)-5-methyl-imidazo[1,2-a]pyridin-2-yl]-furan-2-ylmethylester,
N-(2,6-Dimethyl-phenyl)-N-[6-methyl-2-(2-trifluormethyl-phenyl)-imidazo[1,2-a]pyridin-3-yl]-acetamid,
N-Cyclohexyl-N-(2-furan-2-yl-7-methyl-imidazo[1,2-a]pyridin-3-yl)-acetamid,
N-Cyclohexyl-N-(7-methyl-2-pyridin-2-yl-imidazo[1,2-a]pyridin-3-yl)-acetamid,
N-Cyclohexyl-N-[2-(5-[1,3]dioxolan-2-yl-furan-2-yl)-5-methyl-imidazo[1,2-a]pyridin-3-yl]-acetamid,
N-*tert*-Butyl-N-(5-propyl-2-pyridin-3-yl-imidazo[1,2-a]pyridin-3-yl)-acetamid,
N-*tert*-Butyl-N-[2-(5-methyl-thiophen-2-yl)-imidazo[1,2-a]pyrimidin-3-yl]-acetamid,
3-[Acetyl-(1,1,3,3-tetramethyl-butyl)-amino]-2-furan-2-yl-imidazo[1,2-a]pyridin-8-carbonsäure,
N-*tert*-Butyl-N-[2-(4,5-dimethyl-furan-2-yl)-6-methyl-imidazo[1,2-a]pyridin-3-yl]-acetamid,
N-{2-[3-(4-chlor-phenoxy)-phenyl]-imidazo[1,2-a]pyridin-3-yl}-N-cyclohexyl-acetamid,
Essigsäure-4-[3-(acetyl-cyclohexyl-amino)-imidazo[1,2-a]pyrimidin-2-yl]-2-methoxy-phenylester,
. N-[2-(5-Brom-furan-2-yl)-8-methyl-imidazo[1,2-a]pyridin-3-yl]-N-(1,1,3,3-tetramethyl-butyl)-acetamid,
N-(2,6-Dimethyl-phenyl)-N-[2-(3-hydroxy-phenyl)-5,7-dimethyl-imidazo[1,2-a]pyrimidin-3-yl]-acetamid,
N-Cyclohexyl-N-[2-(2,3-dichlor-phenyl)-imidazo[1,2-a]pyridin-3-yl]-acetamid,
N-Cyclohexyl-N-[2-(2,4-dichlor-phenyl)-5-methyl-imidazo[1,2-a]pyridin-3-yl]-acetamid,
N-Cyclohexyl-N-[2-(2,4-dichlor-phenyl)-imidazo[1,2-a]pyrimidin-3-yl]-acetamid,
[Acetyl-(2-o-tolyl-imidazo[1,2-a]pyrazin-3-yl)-amino]-essigsäuremethylester,
N-*tert*-Butyl-N-(6,8-dichlor-2-thiophen-2-yl-imidazo[1,2-a]pyridin-3-yl)-acetamid,
N-*tert*-Butyl-N-(5-propyl-2-thiophen-2-yl-imidazo[1,2-a]pyridin-3-yl)-acetamid,
{6-[Acetyl-(7-methyl-2-p-tolyl-imidazo[1,2-a]pyridin-3-yl)-amino]-hexyl}methylidyne-ammonium,
N-Butyl-N-(6-methyl-2-p-tolyl-imidazo[1,2-a]pyridin-3-yl)-acetamid,
(6-{Acetyl-[2-(2-methoxy-phenyl)-7-methyl-imidazo[1,2-a]pyridin-3-yl]-amino}-hexyl)-methylidyne-ammonium,
5-{3-[Acetyl-(1,1,3,3-tetramethyl-butyl)-amino]-6-methyl-imidazo[1,2-a]pyridin-2-yl}-furan-2-carbonsäure,
N-Butyl-N-[2-(3,4,5-trimethoxy-phenyl)-imidazo[1,2-a]pyrazin-3-yl]-acetamid,
N-Butyl-N-[2-(3-hydroxy-phenyl)-imidazo[1,2-a]pyrimidin-3-yl]-acetamid,
[Acetyl-(2-o-tolyl-imidazo[1,2-a]pyrimidin-3-yl)-amino]-essigsäuremethylester,
N-(2-Benzofuran-2-yl-8-methyl-imidazo[1,2-a]pyridin-3-yl)-N-(1,1,3,3-tetramethyl-butyl)-acetamid,
N-Butyl-N-(7-methyl-2-p-tolyl-imidazo[1,2-a]pyrimidin-3-yl)-acetamid,
N-*tert*-Butyl-N-(6,8-dibrom-2-methyl-imidazo[1,2-a]pyridin-3-yl)-acetamid,
{6-[Acetyl-(5,7-dimethyl-2-pyridin-2-yl-imidazo[1,2-a]pyridin-3-yl)-amino]-hexyl}-methylidyne-ammonium,
N-*tert*-Butyl-N-[2-(2-ethoxy-naphthalen-1-yl)-7-methyl-imidazo[1,2-a]pyridin-3-yl]-acetamid,
N-*tert*-Butyl-N-[2-(2-chlor-4-fluor-phenyl)-imidazo[1,2-a]pyridin-3-yl]-acetamid,
Cyclohexyl-[7-methyl-2-(2-trifluoromethyl-phenyl)-imidazo[1,2-a]pyridin-3-yl]-amin-Hydrochlorid,
Cyclohexyl-(5,7-dimethyl-2-pyridin-4-yl-imidazo[1,2-a]pyridin-3-yl)-amin-Hydrochlorid,
(2-Furan-2-yl-5,7-dimethyl-imidazo[1,2-a]pyridin-3-yl)-(1,1,3,3-tetramethylbutyl)-amin-Hydrochlorid,
[2-(2-Fluorphenyl)-7-methyl-imidazo[1,2-a]pyridin-3-yl]-(1,1,3,3-tetramethyl-butyl)-amin-Hydrochlorid,
Cyclohexyl-(7-methyl-2-phenyl-imidazo[1,2-a]pyridin-3-yl)-amin-Hydrochlorid,
(2-Furan-2-yl-7-methyl-imidazo[1,2-a]pyrimidin-3-yl)-(1,1,3,3-tetramethyl-butyl)-amin-Hydrochlorid,
N-{2-[3-(4-Chlorphenoxy)-phenyl]-imidazo[1,2-a]pyridin-3-yl}-N-cyclohexyl-acetamid-Hydrochlorid,
N-Cyclohexyl-N-(7-methyl-2-o-tolyl-imidazo[1,2-a]pyridin-3-yl)-acetamid-Hydrochlorid,
N-(2,6-Dimethyl-phenyl)-N-[2-(2,4-dimethyl-phenyl)-5-methyl-imidazo[1,2-a]pyridin-3-yl]-acetamid-Hydrochlorid,
1-Acetyl-3-(acetyl-cyclohexyl-amino)-7-methyl-2-o-tolyl-imidazo[1,2-a]pyridin-1-ium-Chlorid-Hydrochlorid,
Cyclohexyl-(2-furan-3-yl-5,7-dimethyl-imidazo[1,2-a]pyridin-3-yl)-amin-Hydrochlorid,
Cyclopentyl-(2-furan-3-yl-5,7-dimethyl-imidazo[1,2-a]pyridin-3-yl)-amin-Hydrochlorid,
[2-(4-Bromo-2-fluoro-phenyl)-5,7-dimethyl-imidazo[1,2-a]pyridin-3-yl]-cyclopentyl-amin-Hydrochlorid,
Cyclopentyl-{5,7-dimethyl-2-[5-(2-nitro-phenyl)-furan-2-yl]-imidazo[1,2-a]pyridin-3-yl}-amin-Hydrochlorid,
{2-[5-(4-Chlorphenyl)-furan-2-yl]-5,7-dimethyl-imidazo[1,2-a]pyridin-3-yl}cyclopentyl-amin-Hydrochlorid,
Cyclopentyl-(2-furan-3-yl-5,7-dimethyl-imidazo[1,2-a]pyridin-3-yl)-amin-Hydrochlorid,
(2-Furan-3-yl-5,7-dimethyl-imidazo[1,2-a]pyridin-3-yl)-(1,1,3,3-tetramethyl-butyl)-amin-Hydrochlorid,
Benzyl-(7-methyl-2-thiophen-3-yl-imidazo[1,2-a]pyridin-3-yl)-amin-Hydrochlorid,
Cyclohexyl-(2-furan-3-yl-5,7-dimethyl-imidazo[1,2-a]pyridin-3-yl)-amin-Hydrochlorid,
(2-Furan-3-yl-7-methyl-imidazo[1,2-a]pyridin-3-yl)-(1,1,3,3-tetramethyl-butyl)-amin-Hydrochlorid
(5,7-Dimethyl-2-thiophen-3-yl-imidazo[1,2-a]pyridin-3-yl)-(1,1,3,3-tetramethyl-butyl)-amin-Hydrochlorid,
[7-Ethyl-2-(5-nitro-furan-2-yl)-imidazo[1,2-a]pyridin-3-yl]-(2-methoxy-benzyl)-amin,
(2-Chlorbenzyl)-[7-ethyl-2-(5-nitro-furan-2-yl)-imidazo[1,2-a]pyridin-3-yl]-amin,
[7-Ethyl-2-(5-methyl-furan-2-yl)-imidazo[1,2-a]pyridin-3-yl]-(2-methoxy-benzyl)-amin,
(2-Chlorbenzyl)-(7-ethyl-2-furan-2-yl-imidazo[1,2-a]pyridin-3-yl)-amin,
(3-Chlor-4-fluorphenyl)-[7-ethyl-2-(5-methyl-furan-2-yl)-imidazo[1,2-a]pyridin-3-yl]-amin,
(2-Benzofuran-2-yl-7-ethyl-imidazo[1,2-a]pyridin-3-yl)-(3-chlor-4-fluorphenyl)-amin,
(2-Benzofuran-2-yl-7-ethyl-imidazo[1,2-a]pyridin-3-yl)-(3-chlorphenyl)-amin,
. (3-Chlor-4-fluorphenyl)-{2-[5-(3-chloro-phenyl)-furan-2-yl]-7-ethyl-imidazo[1,2-a]pyridin-3-yl}-amin,
(3-Chlor-4-fluorphenyl)-{2-[5-(2-chlorphenyl)-furan-2-yl]-7-ethyl-imidazo[1,2-a]pyridin-3-yl}-amin,
(3-Chlor-4-fluorphenyl)-[2-(4,5-dimethyl-furan-2-yl)-7-ethyl-imidazo[1,2-a]pyridin-3-yl]-amin.

Die erfindungsgemäß verwendeten Verbindungen der allgemeinen Struktur I 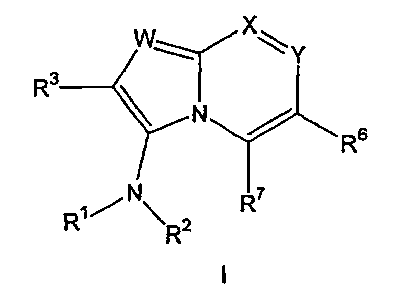 in denen R² für Wasserstoff steht, W für N steht und R¹, R³, R⁶, R⁷, X und Y die oben angegebenen Bedeutungen haben, d.h. Verbindungen der allgemeinen Struktur la, können gemäß folgender Reaktionsgleichung hergestellt werden: 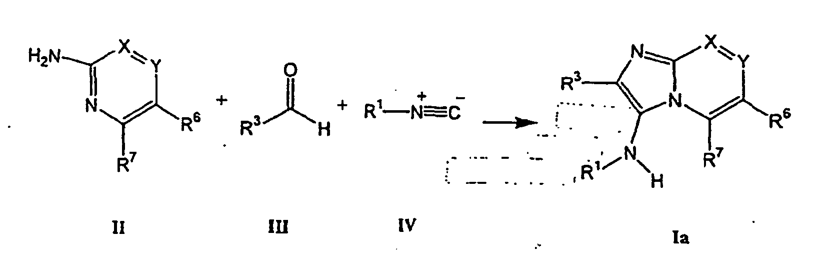 Dabei wird ein Amidin der allgemeinen Struktur II, d.h. ein Aminopyridin (X = CR⁴ und Y = CR⁵) oder ein Aminopyrimidin (X = N und Y = CR⁵) oder ein Aminopyrazin (X = CR⁴ und Y = N), wobei die Reste R⁴ bis R⁷ wie für die Verbindung der allgemeinen Struktur I definiert sind, mit einem Aldehyd der allgemeinen Struktur III, wobei R³ wie für die Verbindung der allgemeinen Struktur I definiert ist, und einem Isonitril der allgemeinen Formel IV, wobei R¹ wie für die Verbindung der allgemeinen Struktur I definiert ist, unter geeigenten Reaktionsbedingungen umgesetzt. Vorzugsweise wird die Reaktion in Gegenwart einer geringen Menge einer Säure, insbesondere 20 %ige wäßrige Perchlorsäure, in einer Dreikomponenten-Eintopf-Umsetzung durchgeführt, die auch semi- oder vollautomatisiert parallelsynthetisch erfolgen kann. Bevorzugt wird die Reaktion in einem organischen Lösungsmittel, insbesondere Dichlormethan oder Acetonitril, bei einer Temperatur von vorzugsweise 0 °C bis 80 °C, insbesondere 15 °C bis 30 °C durchgeführt.

Die Ausgangsverbindungen der allgemeinen Strukturen II, III und IV sind kommerziell erhältlich (z.B. von den Firmen Acros, Geel; Avocado, Port of Heysham; Aldrich, Deisenhofen; Fluka, Seelze; Lancaster, Mülheim; Maybridge, Tintagel; Merck, Darmstadt; Sigma, Deisenhofen; TCl, Japan) und/oder nach im Stand der Technik bekannten Verfahren ohne weiteres zugänglich.

Zur Herstellung der erfindungsgemäß verwendeten Verbindungen der allgemeinen Formel I, bei denen R² nicht Wasserstoff, sondern C(=O)R⁹ bedeutet, können die Verbindungen der allgemeinen Struktur, bei denen R² H bedeutet (d.h. Verbindungen der allgemeinen Formel Ia), entweder ohne Lösungsmittel oder in einem polaren oder unpolaren aprotischen Lösungsmittel, beispielsweise Dimethylsulfoxid (DMSO), Dimethylformamid (DMF), Halogenkohlenwasserstoffen wie beispielsweise Dichlormethan, Acetonitril, aliphatischen Ethern wie Tetrahydrofuran (THF) oder 1,4-Dioxan oder in Kohlenwasserstoffen oder in Gemischen dieser Lösungsmittel, je nach gewünschtem Endprodukt mit einem Säurehalogenid R⁹COHal, worin Hal für Fluor, Chlor, Brom oder lod steht und R⁹ wie oben für die Verbindung der allgemeinen Strukur I definiert ist, innerhalb von z.B. 0,25 bis 12 Stunden bei Temperaturen zwischen 0°C und 160°C gemäß dem folgenden Reaktionsschema umgesetzt werden:

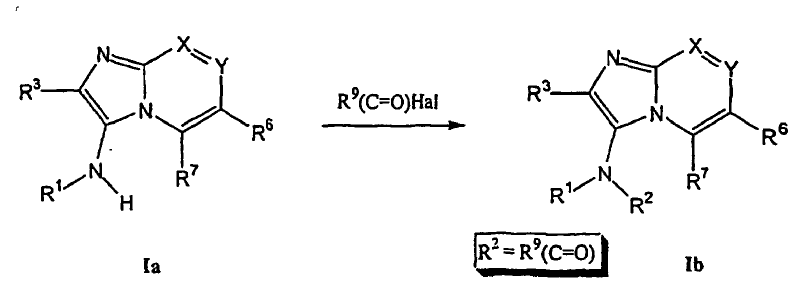

Alternativ können die Verbindungen der allgemeinen Formel la mit einer starken Base, beispielsweise einer metallorganischen Verbindung wie n-Butyllithium, in einem aprotischen Lösungsmittel, wie beispielsweise DMF oder DMSO, vorzugsweise in einem Ether, wie Tetrahydrofuran oder 1,4-Dioxan, bei Temperaturen von vorzugsweise zwischen -70°C und +20°C am exocyclischen Aminostickstoff deprotoniert werden. Die anschließende Zugabe eines Säurehalogenids führt zu den Verbindungen der allgemeinen Formel lb, in denen R² für R⁹(C=O) steht:

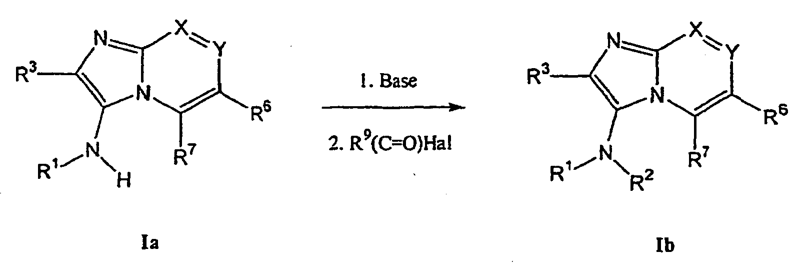

Die nochmalige Umsetzung von Verbindungen der allgemeinen Struktur Ib mit einem Säurehalogenid führt zu den Verbindungen der allgemeinen Struktur I, in denen W NR⁸ bedeutet, d.h. zu Verbindungen der allgemeinen Struktur Ic:

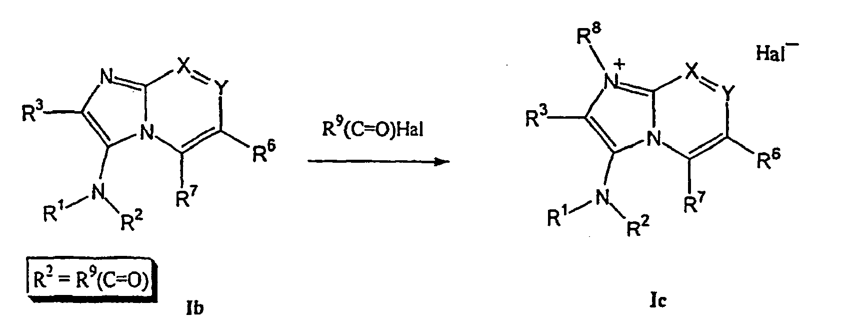

Die erfindungsgemäß verwendenten Verbindungen der allgemeinen Struktur I können sowohl als freie Base als auch als Salz isoliert werden. Die freie Base der erfindungsgemäß verwendenten Verbindung der allgemeinen Struktur I wird üblicherweise nach erfolgter Umsetzung gemäß dem oben beschriebenen Verfahren und anschließender herkömmlicher Aufarbeitung erhalten. Die so gewonnene oder in-situ ohne Isolierung gebildete freie Base kann dann beispielsweise durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in das korrespondierende Salz überführt werden. Bei den gebildeten Salzen handelt es sich u.a. um Hydrochloride, Hydrobromide, Phosphate, Carbonate, Hydrogencarbonate, Formiaté, Acetate, Oxalate, Succinate, Tartrate, Fumarate, Citrate und Glutaminate. Die besonders bevorzugte Hydrochloridbildung kann auch durch Versetzen der in einem geeigneten organischen Lösungsmittel, wie z.B. Butan-2-on (Methylethylketon), gelösten Base mit Trimethylsilylchlorid (TMSCI) herbeigeführt werden.

Soweit die Verbindungen der allgemeinen Struktur I als Racemate oder als Mischungen ihrer verschiedenen Enantiomeren und/oder Diastereomeren erhalten werden, können diese Mischungen nach im Stand der Technik wohlbekannten Verfahren aufgetrennt werden. Geeignete Methoden sind u.a. chromatographische Trennverfahren, insbesondere Flüssigkeitschromatographie-Verfahren unter Normal- oder erhöhtem Druck, bevorzugt MPLC- und HPLC-Verfahren, sowie Verfahren der fraktionierten Kristallisation. Dabei können insbesondere einzelne Enantiomeren z.B. mittels HPLC an chiraler Phase oder mittels Kristallisation von mit chiralen Säuren, etwa (+)-Weinsäure, (-)-Weinsäure oder (+)-10-Camphersulfonsäure, gebildeten diastereomeren Salzen voneinander getrennt werden.

Die durch erfindungsgemäße Verwendung der Verbindungen der allgemeinen Struktur I herstellbaren Arzneimittel zur NOS-Inhibierung, zur Behandlung von Migräne bzw. zur Behandlung von septischem Schock, Multipler Sklerose, Morbus Parkinson, Morbus Alzheimer, Morbus Huntington, Entzündungen, Entzündungsschmerz, cerebraler Ischämie, Diabetes, Meningitis, Arteriosklerose und/oder für die Wundheilung sind üblicherweise pharmazeutische Zusammensetzungen, die neben mindestens einer Verbindung der allgemeinen Struktur I in Form ihrer Base oder eines ihrer pharmazeutisch annehmbaren Salze einen oder mehrere pharmazeutische Hilfsstoffe enthalten.

Diese pharmazeutischen Zusammensetzungen können als flüssige, halbfeste oder feste Arzneiformen und in Form von z.B. Injektionslösungen, Tropfen, Säften, Sirupen, Sprays, Suspensionen, Granulaten, Tabletten, Pellets, Patches, Kapseln, Pflastern, Zäpfchen, Salben, Cremes, Lotionen, Gelen, Emulsionen oder Aerosolen vorliegen und verabreicht werden und enthalten neben mindestens einer Verbindung der allgemeinen Struktur I je nach galenischer Form pharmazeutische Hilfsstoffe, wie z.B. Trägermaterialien, Füllstoffe, Lösungsmittel, Verdünnungsmittel, oberflächenaktive Stoffe, Farbstoffe, Konservierungsstoffe, Sprengmittel, Gleitmittel, Schmiermittel, Aromen und/oder Bindemittel. Diese Hilfsstoffe können beispielsweise sein: Wasser, Ethanol, 2-Propanol, Glycerin, Ethylenglycol, Propylenglycol, Polyethylenglycol, Polypropylenglycol, Glucose, Fructose, Lactose, Saccharose, Dextrose, Melasse, Stärke, modifizierte Stärke, Gelatine, Sorbitol, Inositol, Mannitol, mikrokristalline Cellulose, Methylcellulose, Carboxymethylcellulose, Celluloseacetat, Schellack, Cetylalkohol, Polyvinylpyrrolidon, Paraffine, Wachse, pharmazeutisch annehmbare natürliche und synthetische Gummis, Akaziengummi, Alginate, Dextran, gesättigte und ungesättigte Fettsäuren, Stearinsäure, Magnesiumstearat, Zinkstearat, Glycerylstearat, Natriumlaurylsulfat, genießbare Öle, Sesamöl, Kokusnußöl, Erdnußöl, Sojabohnenöl, Lecithin, Natriumlactat, Polyoxyethylen- und -propylenfettsäureester, Sorbitanfettsäureester, Sorbinsäure, Benzoesäure, Citronensäure, Ascorbinsäure, Tanninsäure, Natriumchlorid, Kaliumchlorid, Magnesiumchlorid, Calciumchlorid, Magnesiumoxid, Zinkoxid, Siliciumdioxid, Titanoxid, Titandioxid, Magnesiumsulfat, Zinksulfat, Calciumsulfat, Pottasche, Calciumphosphat, Dicalciumphosphat, Kaliumbromid, Kaliumiodid, Talkum, Kaolin, Pectin, Crospovidon, Agar und Bentonit.

Die Auswahl der Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich u.a. Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Verbindungen der allgemeinen Struktur I in einem Depot in gelöster Form oder in einem Pflaster, gegebenenfalls unter Zusatz von die Hautpenetration fördernden Mitteln, sind geeignete perkutane Applikationszubereitungen. Oral oder perkutan anwendbare Zubereitungsformen können die Verbindungen der allgemeinen Struktur I verzögert freisetzen.

Die Herstellung der Arzneimittel und pharmazeutischen Zusammensetzungen, die eine der Verbindungen der allgemeinen Struktur I enthalten, erfolgt mit Hilfe von im Stand der Technik der pharmazeutischen Formulierung wohlbekannten Mitteln, Vorrichtungen, Methoden und Verfahren, wie sie beispielsweise in "Remington's Pharmaceutical Sciences", Hrsg. A.R. Gennaro, 17. Ed., Mack Publishing Company, Easton, Pa. (1985), insbesondere in Teil 8, Kapitel 76 bis 93, beschrieben sind.

So kann z.B. für eine feste Formulierung, wie eine Tablette, der Wirkstoff des Arzneimittels, d.h. eine Verbindung der allgemeinen Struktur I oder eines ihrer pharmazeutisch annehmbaren Salze, mit einem pharmazeutischen Träger, z.B. herkömmlichen Tabletteninhaltsstoffen, wie Maisstärke, Lactose, Saccharose, Sorbitol, Talkum, Magnesiumstearat, Dicalciumphosphat oder Gummi, und pharmazeutischen Verdünnungsmitteln, wie z.B. Wasser, gemischt werden, um eine feste Präformulierungs-Zusammensetzung zu bilden, die eine erfindungsgemäße Verbindung oder ein pharmazeutisch annehmbares Salz davon in homogener Verteilung enthält. Unter einer homogenen Verteilung wird hier verstanden, daß der Wirkstoff gleichmäßig über die gesamte Präformulierungs-Zusammensetzung verteilt ist, so daß diese ohne weiteres in gleich wirksame Einheitsdosis-Formen, wie Tabletten, Pillen oder Kapseln, unterteilt werden kann. Die feste Präformulierungs-Zusammensetzung wird anschließend in Einheitsdosis-Formen unterteilt. Die Tabletten oder Pillen des erfindungsgemäßen Arzneimittels bzw. der erfindungsgemäßen Zusammensetzungen können auch beschichtet oder auf andere Weise kompoundiert werden, um eine Dosisform mit verzögerter Freisetzung bereitzustellen. Geeignete Beschichtungsmittel sind u.a. polymere Säuren und Mischungen von polymeren Säuren mit Materialien wie z.B. Schellack, Cetylalkohol und/oder Celluloseacetat.

Die an den Patienten zu verabreichende Wirkstoffmenge variiert und ist abhängig vom Gewicht, dem Alter und der Krankheitsgeschichte des Patienten, sowie von der Applikationsart, der Indikation und dem Schweregrad der Erkrankung. Üblicherweise werden 0,1 bis 5000 mg/kg, insbesondere 1 bis 500 mg/kg, vorzugsweise 2 bis 250 mg/kg Körpergewicht wenigstens einer Verbindung der allgemeinen Struktur I appliziert.

Nachfolgend werden die zur Bestimmung der NOS-Inhibition durch die Verbindungen der allgemeinen Struktur I verwendeten Assays beschrieben:

### NOS-Assay

### Allgemeines

Dieser Assay erlaubt die Bestimmung der prozentualen Hemmung von NO-Synthase durch einen Wirkstoff mittels Messung der NOS-Aktivität bei Einwirken des Wirkstoffs. Dabei wird NO-Synthase zusammen mit radioaktiv markiertem Arginin und dem Wirkstoff unter geeigneten Bedingungen gemischt. Nach Abbruch der NO-Bildungsreaktion zu einem vorgegebenen Zeitpunkt wird die Menge an nicht umgesetztem Arginin direkt oder indirekt bestimmt. Der Vergleich dieser Menge mit der in einem ohne Zusatz von Wirkstoff und unter sonst gleichen Bedingungen aus der Mischung von NOS und Arginin zurückbleibenden Menge an Arginin ergibt die %-Hemmung von NO-Synthase durch den getesteten Wirkstoff. Dieser Assay läßt sich wie folgt durchführen:
(a) Inkubation der NO-Synthase mit markiertem Arginin als Substrat in einem Reaktionsgefäß,
(b) Trennung des markierten Arginins von dem gegebenenfalls als Produkt der enzymatischen Reaktion entstandenen, markierten Citrullin zu einem Zeitpunkt, zu dem die Konzentration an Citrullin ansteigt,
(c) Messung der Menge an jeweils abgetrenntem Arginin.

Die Trennung erfolgt über eine Filterplatten-Membran.

Dieser NOS-Assay eignet sich insbesondere für ein "High Throughput Screening" (HTS) auf Mikrotiterplatten (MTP).

### HTS-NOS-Assay: Allgemeine Verfahrensweise

In diesem HTS-NOS-Assay wird radioaktives Arginin als Substrat benutzt. Das Assayvolumen kann je nach Art der Mikrotiterplatte (MTP) im Bereich zwischen 25 µl und 250 µl gewählt werden. In Abhängigkeit von der benutzten Enzymquelle werden Cofaktoren und Coenzyme zugefügt. Die Inkubation der Ansätze in dieser Mikrotiterplatte (Assay-MTP) gemäß Schritt (a) wird bei Raumtemperatur vorgenommen und beträgt je nach verwendeter Enzymaktivität (units) zwischen 5 und 60 Minuten. Zum Ende der Inkubation (Schritt (a)) wird die Platte in einen Zellharvester plaziert, der mit einer MTP bestückt ist, die eine Kationenaustauschermembran als Filterboden besitzt (Filter-MTP). Alle Ansätze der Assay-MTP werden in diese Filter-MTP überführt und über eine Kationenaustauscher-Filter-Platte, einen mit Phosphatgruppen beladenen Papierfilter, abgesaugt. Die Filter-MTP wird anschließend mit Puffer oder Wasser gewaschen. Mit Hilfe dieser Vorgehensweise wird das verbliebene Substrat Arginin auf dem Kationenaustauscher gebunden, während das enzymatisch gebildete radioaktive Citrullin quantitativ ausgewaschen wird. Nach Trocknen der Filter-MTP und Zugabe von Szintillationsflüssigkeit kann das gebundene Arginin am Szintillationszähler ausgezählt werden. Eine nicht gehemmte NOS-Reaktion spiegelt sich in einer geringen Radioaktivität wieder. Eine gehemmte Enzymreaktion bedeutet, daß das radioaktive Arginin nicht umgesetzt worden ist. Das heißt, auf dem Filter befindet sich eine hohe Radioaktivität.

### Verwendete Materialien

- Arginin, L-[2, 3, 4-³H]-monohydrochlorid; Best.-Nr. NET-1123, Fa. NEN
- CaCl₂ wasserfrei; Best.- Nr. 2388.1000; Fa. Merck KGaA
- 1.4-Dithiothreitol (DTT), Best.-Nr. 708984; Fa. ROCHE
- Na₂EDTA-Dihydrat; Best.-Nr. 03680; Fa. FLUKA
- HEPES, Best:-Nr. H-3375; Fa. SIGMA
- NADPH, Tetranatriumsalz; Best.-Nr. 1585363; Fa. ROCHE
- TRIS; BEST.-Nr. 93349; Fa. FLUKA
   - Enzym-Präparationspuffer:: 50 mM Tris-HCl mit 1 mM EDTA: Der pH-Wert des Puffers wurde bei 4 °C auf 7,4 eingestellt.
   - Inkubationspuffer (-medium):: 50 mM HEPES mit 1 mM EDTA; 1,25 mM
   CaCl₂ und 1 mM Dithiothreitol.
   Der pH-Wert des Puffers wurde bei 25 °C auf 7,4 eingestellt.
   - Waschmedium:: H₂O

### Enzympräparation

Als Ausgangsgewebe wurden Ratten-Cerebelli benutzt. Die Tiere wurden betäubt und getötet, das Gehirngewebe, das Cerebellum, wurde herauspräpariert, pro Rattenkleinhirn wurde 1 ml Enzympräparationspuffer (4 °C) hinzugegeben, und es wurde mit einem Polytron-Homogenisierer für 1 min bei 6000 U/min aufgeschlossen. Danach erfolgte Zentrifugation bei 4 °C für 15 min bei 20 000 g und anschließend Abdekantieren des Überstand und portioniertes Einfrieren bei - 80 °C (Verwerfen des Niederschlags).

### Inkubationsansatz:

Verwendet wurden 96-well MTP mit einer "Well"-Kapazität von ≤ 250 µl Pipettierreihenfolge: siehe Tabelle 1:

**Tabelle 1:**

| **Substanz** | **Molarität i.A.** | **µl** | *** Protein i.A:** |
|---|---|---|---|
| Inkubat.-Puffer | - | 100 | - |
| Testsubstanz | variabel; vorzugsweise 10⁻⁵M | variabel; vorzugsweise 20 µl | - |
| NADPH | 0,5 mM | 20 | - |
| Enzym (s. Beispiel 3) | - | variabel; maximales Volumen der Enzymlösung = 50 µl | variabel; maximale einsetzbare Proteinmenge = 100 µg |
| [³H]Substrat | variabel; vorzugsweise 50 nM | variabel; vorzugsweise 10 µl | - |
| Endvolumen: | | max. 250 µl | |

| | | | |
|---|---|---|---|
| * Proteinbestimmung nach O.H. Lowry et al; J. Biol.Chem. **193**, 265 (1951) i.A. = im Ansatz | | | |

Nach beendetem Pipettiervorgang wurde ein Deckel auf diese MTP (Assay-MTP) gelegt. Inkubation bei 25 °C (Raumtemperatur (RT)) für 5 - 60 min, je nach Menge und Aktivität des eingesetzten Enzyms.

Anschließend wurde der Inhalt der Assay-MTP mit Hilfe eines 96-well Cell-Harvesters in eine 96-well Kationenaustauscher MTP (Filter-MTP) transferiert und abgesaugt. Es schloß sich eine einmalige Wäsche mit 200 ml H₂O (aus einer Wanne) an.

Dann wurde die Platte für 1 h bei 60 °C im Trockenschrank getrocknet. Dann wurde die Bodenseite der Filter-MTP von unten her exakt mit einem "back seal" versiegelt. Danach wurden pro well 35 µl Szintillator hinzupipettiert. Ferner wurde die Plattenoberseite mit einem "top seal" versiegelt. Nach 1 h Wartezeit wurde die Platte am β-Counter ausgemessen.

Im HTS-Betrieb wurden das Inkubationsmedium, NADPH- und Enzymlösung vor Beginn des Pipettierschrittes vereint, um nicht zeitaufwendig drei separate Pipettierungen vornehmen zu müssen.

Die Ergebnisse von Beispielverbindungen im NOS-Assay sind in Tabelle 3 wiedergegeben.

### Citrullin-Assay

Dieser Assay wurde wie von D. S. Bredt und S. H. Snyder (*Proc. Natl. Acad. Sci. USA* (1990), 87, 682-685) beschrieben durchgeführt. Die Ergebnisse von Beispielverbindungen im Citrullin-Assay sind in Tabelle 4 wiedergegeben.

Die folgenden Beispiele dienen der näheren Erläuterung der Erfindung, ohne sie darauf zu beschränken.

### Beispiele:

Die Verbindungen der allgemeinen Struktur I wurden nach den folgenden allgemeinen Synthesevorschriften (AAV) hergestellt:

### Allgemeine Arbeitsvorschrift 1 (AAV 1)

Ein Rundbodenröhrchen aus Glas (Durchmesser 16 mm, Länge 125 mm) mit Gewinde wurde mit einem Rührer versehen und mit einem Schraubdeckel mit Septum verschlossen. Das Röhrchen wurde auf einen auf 15°C temperierten Reaktorblock gestellt. Es wurden nacheinander folgende Reagenzien hinzupipettiert:
1.) 1 ml einer 0,1 M Amidin-II-Lösung + 10 µl 20 %ige wäßrige HClO₄, in Dichlormethan
2.) 0,5 ml einer 0,3 M Lösung des Aldehyds III in Dichlormethan
3.) 0,575 ml einer 0,2 M Isonitril-IV-Lösung in Dichlormethan

Das Reaktionsgemisch wurde bei 15°C 12 h lang gerührt. Danach wurde die Reaktionslösung abfiltriert. Das Röhrchen wurde dabei zweimal mit je 1 ml Dichlormethan und 200 µl Wasser gespült.

Das Reaktionsgemisch wurde mit 3 ml einer 10%igen NaCl-Lösung und 1,5 ml Dichlormethan versetzt und gründlich durchmischt. Die organische Phase wurde abgetrennt und die wäßrige Phase erneut mit 1,5 ml Dichlormethan extrahiert. Die vereinigten organischen Phasen wurden über 2,4 g MgSO₄ (granuliert) getrocknet. Das Lösungsmittel wurde in einer Vakuumzentrifuge entfernt.

Die eingesetzten Chemikalien und Lösungsmittel wurden kommerziell erworben. Jede Substanz wurde mit ESI-MS und/oder NMR analysiert.

Die gemäß AAV 1 hergestellten Beispiele 1-142 und 313-322 wurden im HTS-NOS-Assay automatisiert getestet. Die Ergebnisse sind in Tabelle 2 wiedergegeben.

**Tabelle 2**

| **Beispiel- Nr.** | **Verbindung** | **HTS-NOS-** **Assay: %-** **Hemmung** **(10 µM)** | **Masse** **berechnet** | **Masse** **gefunden** |
|---|---|---|---|---|
| 2 | Cyclohexyl-(5,7-dimethyl-2-pyridin-4-yl-imidazo[1,2-a]pyridin-3-yl)-amin | 62 | 320,43 | 321,3 |
| 3 | (5,7-Dimethyl-2-pyridin-4-yl-imidazo[1,2-a]pyridin-3-yl)-(1,1,3,3-tetramethyl-butyl)-am in | 60 | 350,5 | 351,4 |
| 4 | {6-[5,7-Dimethyl-2-(1H-pyrrol-2-yl)-imidazo[1,2-a]pyridin-3-ylamino]-hexyl}-methylidyne-ammonium | 54 | 336,46 | 336,4 |
| 6 | [2-(3,4-Dimethoxy-phenyl)-7-methylimidazo[1,2-a]pyridin-3-yl]-(1,1,3,3-tetramethyl-butyl)-amin | 53 | 395,54 | 396,3 |
| 7 | Cyclohexyl-(7-methyl-2-pyridin-4-yl-imidazo[1,2-a]pyridin-3-yl)-amin | 65 | 306,41 | 307,4 |
| 8 | (2-Furan-2-yl-5,7-dimethyl-imidazo[1,2-a]pyridin-3-yl)-(1,1,3,3-tetramethyl-butyl)-amin | 52 | 339,48 | 340,4 |
| 9 | (1,1,3,3-Tetramethyl-butyl)-(2,5,7-trimethyl-imidazo[1,2-a]pyridin-3-yl)-amin | 59 | 287,45 | 288,4 |
| 10 | Cyclohexyl-(7-methyl-2-o-tolylimidazo[1,2-a]pyridin-3-yl)-amin | 54 | 319,45 | 320,4 |
| 11 | Cyclohexyl-(7-methyl-2-thiophen-2-yl-imidazo[1,2-a]pyridin-3-yl)-amin | 60 | 311,45 | 312,4 |
| 12 | (5,7-Dimethyl-2-pyridin-3-yl-imidazo[1,2-a]pyridin-3-yl)-(1,1,3,3-tetramethyl-butyl)-amin | 61 | 350,5 | 351,3 |
| 13 | Cyclohexyl-[7-methyl-2-(2-trifluormethyl-phenyl)-imidazo[1,2-a]pyridin-3-yl]-amin | 57 | 373,42 | 374,5 |
| 15 | (7-Methyl-2-pyridin-3-yl-imidazo[1,2-a]pyridin-3-yl)-(1,1,3,3-tetramethylbutyl)-amin | 59 | 336,48 | 337,4 |
| 16 | Cyclohexyl-(7-methyl-2-naphthalen-1-yl-imidazo[1,2-a]pyridin-3-yl)-amin | 56 | 355,48 | 356,6 |
| 17 | [2-(2-Fluor-phenyl)-7-methylimidazo[1,2-a]pyridin-3-yl]-(1,1,3,3-tetramethyl-butyl)-amin | 51 | 353,48 | 354,3 |
| 18 | (2,7-Dimethyl-imidazo[1,2-a]pyridin-3-ylamino)-essigsäuremethylester | 50 | 233,27 | 234,3 |
| 19 | Methylidyne-[6-(7-methyl-2-pyridin-3-yl-imidazo[1,2-a]pyridin-3-ylamino)-hexyl]-ammonium | 53 | 334,44 | 334,4 |
| 21 | Cyclohexyl-[2-(2-fluor-phenyl)-7-methyl-imidazo[1,2-a]pyridin-3-yl]-amin | 53 | 323,41 | 324,5 |
| 23 | Cyclohexyl-(7-methyl-2-pyridin-3-yl-imidazo[1,2-a]pyridin-3-yl)-amin | 74 | 306,41 | 307,4 |
| 26 | Cyclohexyl-(2-furan-2-yl-5,7-dimethyl-imidazo[1,2-a]pyridin-3-yl)-amin | 62 | 309,41 | 310,4 |
| 27 | Cyclohexyl-(5,7-dimethyl-2-pyridin-3-yl-imidazo[1,2-a]pyridin-3-yl)-amin | 62 | 320,43 | 321,3 |
| 28 | (2-Furan-2-yl-7-methyl-imidazo[1,2-a]pyridin-3-yl)-(1,1,3,3-tetramethylbutyl)-amin | 50 | 325,45 | 326,3 |
| 29 | (7-Methyl-2-pyridin-4-yl-imidazo[1,2-a]pyridin-3-yl)-(1,1,3,3-tetramethylbutyl)-amin | 69 | 336,48 | 337,4 |
| 30 | Butyl-(2-cyclohexyl-7-methyl-imidazo[1,2-a]pyridin-3-yl)-amin | 56 | 285,43 | 286,4 |
| 31 | 3-[5,7-Dimethyl-3-(1,1,3,3-tetramethylbutylamino)-imidazo[1,2-a]pyridin-2-yl]-phenol | 60 | 365,52 | 366,3 |
| 32 | (2,6-Dimethyl-phenyl)-(5,7-dimethyl-2-o-tolyl-imidazo[1,2-a]pyridin-3-yl)-amin | 59 | 355,48 | 356,3 |
| 34 | (2,6-Dimethyl-phenyl)-[2-(2-fluorphenyl)-5,7-dimethyl-imidazo[1,2-a]pyridin-3-yl]-amin | 61 | 359,44 | 360,3 |
| 35 | Cyclohexyl-(2,5,7-trimethyl-imidazo[1,2-a]pyridin-3-yl)-amin | 55 | 257,38 | 258,4 |
| 36 | [5,7-Dimethyl-2-(1H-pyrrol-2-yl)-imidazo[1,2-a]pyridin-3-yl]-(1,1,3,3-tetramethyl-butyl)-amin | 69 | 338,49 | 339,5 |
| 37 | Butyl-(2-cyclohexyl-5,7-dimethyl-imidazo[1,2-a]pyridin-3-yl)-amin | 58 | 299,46 | 300,3 |
| 38 | (5,7-Dimethyl-2-o-tolyl-imidazo[1,2-a]pyridin-3-yl)-(1,1,3,3-tetramethylbutyl)-amin | 60 | 363,54 | 364,3 |
| 39 | [2-(2,3-Dimethoxy-phenyl)-7-methyl-imidazo[1,2-a]pyridin-3-yl]-(2,6-dimethyl-phenyl)-amin | 58 | 387,48 | 388,4 |
| 40 | (2,7-Dimethyl-imidazo[1,2-a]pyridin-3-yl)-(1,1,3,3-tetramethyl-butyl)-amin | 61 | 273,42 | 274,3 |
| 41 | [2-(5-[1,3]Dioxolan-2-yl-furan-2-yl)-7-methyl-imidazo[1,2-a]pyridin-3-yl]-(1,1,3,3-tetramethyl-butyl)-amin | 57 | 397,51 | 398,4 |
| 42 | [2-(3-Brom-thiophen-2-yl)-5,7-dimethyl-imidazo[1,2-a]pyridin-3-yl]-(2,6-dimethyl-phenyl)-amin | 55 | 426,38 | 426,3/428 ,2 |
| 43 | (2,6-Dimethyl-phenyl)-[2-(2-fluorphenyl)-7-methyl-imidazo[1,2-a]pyridin-3-yl]-amin | 71 | 345,42 | 346,3 |
| 44 | (2-Cyclohexyl-5,7-dimethyl-imidazo[1,2-a]pyridin-3-yl)-(1,1,3,3-tetramethyl-butyl)-amin | 71 | 355,56 | 356,3 |
| 45 | [6-(2-Furan-2-yl-5,7-dimethyl-imidazo[1,2-a]pyridin-3-ylamino)-hexyl]-methylidyne-ammonium | 54 | 337,44 | 337,4 |
| 46 | (7-Methyl-2-o-tolyl-imidazo[1,2-a]pyridin-3-yl)-(1,1,3,3-tetramethyl-butyl)-amin | 59 | 349,52 | 350,4 |
| 47 | [2-(2,3-Dichlor-phenyl)-7-methyl-imidazo[1,2-a]pyridin-3-yl]-(2,6-dimethyl-phenyl)-amin | 69 | 396,32 | 396,3/ 398,3 |
| 48 | [2-(2,3-Dimethoxy-phenyl)-5,7-dimethyl-imidazo[1,2-a]pyridin-3-yl]-(2,6-dimethyl-phenyl)-amin | 68 | 401,5 | 402,3 |
| 49 | Butyl-[2-(2,3-dimethoxy-phenyl)-5,7-dimethyl-imidazo[1,2-a]pyridin-3-yl]-amin | 67 | 353,46 | 354,3 |
| 50 | Methylidyne-[6-(7-methyl-2-p-tolyl-imidazo[1,2-a]pyridin-3-ylamino)-hexyl]-ammonium | 78 | 347,48 | 347,5 |
| 52 | Essigsäure 5-(3-cyclohexylamino-5,7-dimethyl-imidazo[1,2-a]pyridin-2-yl)-furan-2-ylmethylester | 64 | 381,47 | 382,4 |
| 53 | [2-(2-Methoxy-phenyl)-5,7-dimethyl-imidazo[1,2-a]pyridin-3-yl]-(1,1,3,3-tetramethyl-butyl)-amin | 61 | 379,54 | 380,3 |
| 54 | [2-(3,4-Dimethoxy-phenyl)-7-methyl-imidazo[1,2-a]pyridin-3-yl]-(2,6-dimethyl-phenyl)-amin | 64 | 387,48 | 388,3 |
| 55 | 3-(3-Butylamino-7-methyl-imidazo[1,2-a]pyridin-2-yl)-phenol | 63 | 295,38 | 296,2 |
| 56 | (2-Benzofuran-2-yl-7-methyl-imidazo[1,2-a]pyridin-3-yl)-(1,1,3,3-tetramethyl-butyl)-amin | 60 | 375,51 | 376,4 |
| 57 | (2-Benzofuran-2-yl-5,7-dimethyl-imidazo[1,2-a]pyridin-3-yl)-(2,6-dimethyl-phenyl)-amin | 57 | 381,47 | 382,4 |
| 58 | Essigsäure 5-(3-cyclohexylamino-7-methyl-imidazo[1,2-a]pyridin-2-yl)-furan-2-ylmethylester | 64 | 367,44 | 368,4 |
| 59 | [6-(5,7-Dimethyl-2-pyridin-3-yl-imidazo[1,2-a]pyridin-3-ylamino)-hexyl]-methylidyne-ammonium | 57 | 348,47 | 348,4 |
| 60 | Butyl-[2-(2-methoxy-phenyl)-7-methyl-imidazo[1,2-a]pyridin-3-yl]-amin | 52 | 309,41 | 310,3 |
| 61 | {6-[2-(2-Methoxy-phenyl)-5,7-dimethyl-imidazo[1,2-a]pyridin-3-ylamino]-hexyl}-methylidyne-ammonium | 73 | 377,51 | 377,3 |
| 62 | {5-[5,7-Dimethyl-3-(1,1,3,3-tetramethyl-butylamino)-imidazo[1,2-a]pyridin-2-yl]-furan-2-yl}-methanol | 55 | 369,5 | 370,4 |
| 63 | (7-Methyl-2-naphthalen-1-yl-imidazo[1,2-a]pyridin-3-yl)-(1,1,3,3-tetramethyl-butyl)-amin | 52 | 385,55 | 386,3 |
| 66 | (2-Benzofuran-2-yl-5,7-dimethyl-imidazo[1,2-a]pyridin-3-yl)-(1,1,3,3-tetramethyl-butyl)-amin | 53 | 389,54 | 390,4 |
| 69 | Cyclohexyl-(2,7-dimethyl-imidazo[1,2-a]pyridin-3-yl)-amin | 55 | 243,35 | 244,4 |
| 70 | [2-(2,3-Dichlorphenyl)-8-methyl-imidazo[1,2-a]pyridin-3-yl]-(1,1,3,3-tetramethyl-butyl)-amin | 52 | 404,38 | 404,3/ 406,2 |
| 71 | (7-Methyl-2-p-tolyl-imidazo[1,2-a]pyridin-3-yl)-(1,1,3,3-tetramethyl-butyl)-amin | 58 | 349,52 | 350,3 |
| 72 | (2,6-Dimethyl-phenyl)-[2-(2-methoxy-phenyl)-7-methyl-imidazo[1,2-a]pyridin-3-yl]-amin | 53 | 357,45 | 358,3 |
| 73 | 3-(3-Butylamino-5,7-dimethyl-imidazo[1,2-a]pyridin-2-yl)-phenol | 61 | 309,41 | 310,2 |
| 74 | Butyl-[2-(2,3-dimethoxy-phenyl)-7-methyl-imidazo[1,2-a]pyridin-3-yl]-amin | 62 | 339,43 | 340,4 |
| 75 | {6-[5,7-Dimethyl-2-(2-trifluormethyl-phenyl)-imidazo[1,2-a]pyridin-3-ylamino]-hexyl}-methylidyne-ammonium | 56 | 415,48 | 415,3 |
| 77 | Cyclohexyl-(2-cyclohexyl-5,7-dimethyl-imidazo[1,2-a]pyridin-3-yl)-amin | 63 | 325,49 | 326,4 |
| 78 | [2-(2,3-Dichlorphenyl)-5,7-dimethyl-imidazo[1,2-a]pyridin-3-yl]-(2,6-dimethyl-phenyl)-amin | 61 | 410,34 | 410,3/ 412,2 |
| 79 | (2,6-Dimethyl-phenyl)-[2-(2-methoxy-phenyl)-5,7-dimethyl-imidazo[1,2-a]pyridin-3-yl]-amin | 66 | 371,48 | 372,3 |
| 80 | {2-[5-(2-Chlorphenyl)-furan-2-yl)-7-methyl-imidazo[1,2-a]pyridin-3-yl}-(1,1,3,3-tetramethyl-butyl)-amin | 52 | 435,99 | 436,4/ 437,2/ 438,4 |
| 81 | 5-[7-Methyl-3-(1,1,3,3-tetramethyl-butylamino)-imidazo[1,2-a]pyridin-2-yl]-furan-2-carbonsäure | 60 | 369,46 | 370,4 |
| 82 | Cyclohexyl-[2-(2-methoxy-phenyl)-8-methyl-imidazo[1,2-a]pyridin-3-yl]-amin | 62 | 335,45 | 336,4 |
| 83 | 3-[7-Methyl-3-(1,1,3,3-tetramethyl-butylamino)-imidazo[1,2-a]pyridin-2-yl]-phenol | 60 | 351,49 | 352,4 |
| 84 | [2-(2,3-Dichlorphenyl)-5,7-dimethyl-imidazo[1,2-a]pyridin-3-yl]-(1,1,3,3-tetramethyl-butyl)-amin | 54 | 418,41 | 418,2/ 420,2 |
| 85 | [2-(2,4-Dichlorphenyl)-7-methyl-imidazo[1,2-a]pyridin-3-yl]-(1,1,3,3-tetramethyl-butyl)-amin | 61 | 404,38 | 404,4/ 406,2 |
| 86 | [2-(5-Bromfuran-2-yl)-7-methyl-imidazo[1,2-a]pyridin-3-yl]-(1,1,3,3-tetramethyl-butyl)-amin | 54 | 404,35 | 404,3/ 406,3 |
| 87 | 5-(3-Cyclohexylamino-5,7-dimethyl-imidazo[1,2-a]pyridin-2-yl)-furan-2-carbonsäure | 54 | 353,42 | 354,4 |
| 88 | [6-(2-Cyclohexyl-5,7-dimethyl-imidazo[1,2-a]pyridin-3-ylamino)-hexyl]-methylidyne-ammonium | 57 | 353,53 | 353,4 |
| 89 | [2-(2,4-Dichlorphenyl)-5,7-dimethyl-imidazo[1,2-a]pyridin-3-yl]-(1,1,3,3-tetramethyl-butyl)-amin | 68 | 418,41 | 418,3/ 420,2 |
| 90 | (2-Benzofuran-2-yl-7-methyl-imidazo[1,2-a]pyridin-3-yl)-(2,6-dimethyl-phenyl)-amin | 53 | 367,45 | 368,4 |
| 91 | 5-(3-Cyclohexylamino-7-methyl-imidazo[1,2-a]pyridin-2-yl)-furan-2-carbonsäure | 64 | 339,39 | 340,4 |
| 92 | {6-[2-(2-Bromphenyl)-5,7-dimethyl-imidazo[1,2-a]pyridin-3-ylamino]-hexyl}-methylidyne-ammonium | 60 | 426,38 | 425,3/ 427,2 |
| 95 | (5,7-Dimethyl-2-p-tolyl-imidazo[1,2-a]pyridin-3-yl)-(1,1,3,3-tetramethyl-butyl)-amin | 70 | 363,54 | 364,4 |
| 96 | [2-(2,3-Dichlorphenyl)-8-methyl-imidazo[1,2-a]pyridin-3-yl]-(2,6-dimethyl-phenyl)-amin | 55 | 396,32 | 396,3/ 398,3 |
| 97 | Methylidyne-[6-(7-methyl-2-o-tolyl-imidazo[1,2-a]pyridin-3-ylamino)-hexyl]-ammonium | 62 | 347,48 | 347,5 |
| 98 | {2-[5-(3-Chlorphenyl)-furan-2-yl]-7-methyl-imidazo[1,2-a]pyridin-3-yl}-(1,1,3,3-tetramethyl-butyl)-amin | 62 | 435,99 | 436,4/ 438,3 |
| 99 | Cyclohexyl-[7-methyl-2-(5-nitro-furan-2-yl)-imidazo[1,2-a]pyridin-3-yl]-amin | 66 | 340,38 | 341,4 |
| 100 | [2-(2-Bromphenyl)-7-methyl-imidazo[1,2-a]pyridin-3-yl]-cyclohexyl-amin | 50 | 384,32 | 384,4/ 386,4/ 387,3 |
| 101 | [2-(2-Methoxy-phenyl)-7-methyl-imidazo[1,2-a]pyridin-3-yl]-(1,1,3,3-tetramethyl-butyl)-amin | 58 | 365,52 | 366,4 |
| 102 | {5-[7-Methyl-3-(1,1,3,3-tetramethyl-butylamino)-imidazo[1,2-a]pyridin-2-yl]-furan-2-yl}-methanol | 59 | 355,48 | 356,5 |
| 103 | (6-{2-[5-(2-Chlorphenyl)-furan-2-yl]-5-methyl-imidazo[1,2-a]pyridin-3-ylamino}-hexyl)-methylidyne-ammonium | 50 | 433,96 | 433,4/ 435,4 |
| 104 | Cyclohexyl-[5,7-dimethyl-2-(5-nitro-furan-2-yl)-imidazo[1,2-a]pyridin-3-yl]-amin | 63 | 354,4 | 355,4 |
| 105 | Cyclohexyl-[2-(4,5-dimethyl-furan-2-yl)-7-methyl-imidazo[1,2-a]pyridin-3-yl]-amin | 65 | 323,43 | 324,4 |
| 106 | [6-(5,7-Dimethyl-2-o-tolyl-imidazo[1,2-a]pyridin-3-ylamino)-hexyl]-methylidyne-ammonium | 60 | 361,51 | 361,4 |
| 107 | Methylidyne-[6-(7-methyl-2-pyridin-2-yl-imidazo[1,2-a]pyridin-3-ylamino)-hexyl]-ammonium | 50 | 334,44 | 334,4 |
| 108 | [2-(2,3-Dimethoxy-phenyl)-5,7-dimethyl-imidazo[1,2-a]pyridin-3-yl]-(1,1,3,3-tetramethyl-butyl)-amin | 73 , | 409,57 | 410,4 |
| 109 | {6-[2-(3,4-Dimethoxy-phenyl)-7-methyl-imidazo[1,2-a]pyridin-3-ylamino]-hexyl}-methylidyne-ammonium | 51 | 393,51 | 393,4 |
| 111 | Cyclohexyl-(8-methyl-2-pyridin-4-yl-imidazo[1,2-a]pyridin-3-yl)-amin | 52 | 306,41 | 307,4 |
| 112 | [2-(2,3-Dichlor-phenyl)-6-methyl-imidazo[1,2-a]pyridin-3-yl]-(1,1,3,3-tetramethyl-butyl)-amin | 52 | 404,38 | 404.3/ 406.3 |
| 113 | 5-(3-Butylamino-imidazo[1,2-a]pyrazin-2-yl)-thiophen-2-carbonsäure | 54 | 316,38 | 317,3 |
| 114 | Cyclohexyl-(5,7-dimethyl-2-pyridin-2-yl-imidazo[1,2-a]pyridin-3-yl)-amin | 53 | 320,43 | 321,4 |
| 115 | (2-Benzofuran-2-yl-8-methyl-imidazo[1,2-a]pyridin-3-yl)-(1,1,3,3-tetramethyl-butyl)-amin | 59 | 375,51 | 376,4 |
| 116 | {6-[2-(2-Fluor-phenyl)-5,7-dimethyl-imidazo[1,2-a]pyridin-3-ylamino]-hexyl}-methylidyne-ammonium | 60 | 365,47 | 365,3 |
| 117 | [2-(2,3-Dimethoxy-phenyl)-8-methyl-imidazo[1,2-a]pyridin-3-yl]-(1,1,3,3-tetramethyl-butyl)-amin | 52 | 395,54 | 396,3 |
| 118 | Methylidyne-[6-(7-methyl-2-phenanthren-9-yl-imidazo[1,2-a]pyridin-3-ylamino)-hexyl]-ammonium | 56 | 433,57 | 433,5 |
| 121 | Cyclohexyl-(2-furan-2-yl-8-methyl-imidazo[1,2-a]pyridin-3-yl)-amin | 53 | 295,38 | 296,4 |
| 122 | Methylidyne-[6-(7-methyl-2-naphthalen-1-yl-imidazo[1,2-a]pyridin-3-ylamino)-hexyl]-ammonium | 53 | 383,51 | 383,4 |
| 124 | (6-Methyl-2-pyridin-4-yl-imidazo[1,2-a]pyridin-3-yl)-(1,1,3,3-tetramethyl-butyl)-amin | 68 | 336,48 | 337,4 |
| 126 | (7-Methyl-2-pyridin-3-yl-imidazo[1,2-a]pyrimidin-3-yl)-(1,1,3,3-tetramethyl-butyl)-amin | 50 | 337,47 | 338,4 |
| 128 | [6-(5,7-Dimethyl-2-naphthalen-1-yl-imidazo[1,2-a]pyridin-3-ylamino)-hexyl]-methylidyne-ammonium | 63 | 397,54 | 397,3 |
| 129 | 3-[3-(2,6-Dimethyl-phenylamino)-5,7-dimethyl-imidazo[1,2-a]pyridin-2-yl]-phenol | 61 | 357,45 | 358,3 |
| 130 | (2,6-Dimethyl-phenyl)-(8-methyl-2-o-tolyl-imidazo[1,2-a]pyridin-3-yl)-amin | 58 | 341,45 | 342,3 |
| 131 | {6-[2-(3-Hydroxy-phenyl)-8-methyl-imidazo[1,2-a]pyridin-3-ylamino]-hexyl}-methylidyne-ammonium | 55 | 349,45 | 349,4 |
| 132 | {5-[3-(2,6-Dimethyl-phenylamino)-7-methyl-imidazo[1,2-a]pyrimidin-2-yl]-furan-2-yl}-methanol | 52 | 348,4 | 349,4 |
| 133 | (8-Methyl-2-p-tolyl-imidazo[1,2-a]pyridin-3-yl)-(1,1,3,3-tetramethyl-butyl)-amin | 52 | 349,52 | 350,3 |
| 134 | [2-(2,4-Dichlorphenyl)-6-methyl-imidazo[1,2-a]pyridin-3-yl]-(2,6-dimethyl-phenyl)-amin | 56 | 396,32 | 396,2/ 398,2 |
| 135 | Butyl-[2-(2,4-dichlor-phenyl)-6-methyl-imidazo[1,2-a]pyridin-3-yl]-amin | 50 | 348,27 | 348,3/ 350,2 |
| 136 | Butyl-[2-(4-dimethylamino-naphthalen-1-yl)-imidazo[1,2-a]pyrazin-3-yl]-amin | 56 | 359,47 | 360,5 |
| 137 | {6-[2-(2-Brom-phenyl)-7-methyl-imidazo[1,2-a]pyridin-3-ylamino]-hexyl}-methylidyne-ammonium | 52 | 412,35 | 411,3/ 414,2 |
| 138 | Butyl-[2-(2-methoxy-phenyl)-6-methyl-imidazo[1,2-a]pyridin-3-yl]-amin | 52 | 309,41 | 310,3 |
| 139 | (2-Cyclohexyl-8-methyl-imidazo[1,2-a]pyridin-3-yl)-(1,1,3,3-tetramethyl-butyl)-amin | 51 | 341,54 | 342,5 |
| 140 | Cyclohexyl-(7-methyl-2-pyridin-2-yl-imidazo[1,2-a]pyridin-3-yl)-amin | 55 | 306,41 | 307,3 |
| 141 | Cyclohexyl-(2-furan-2-yl-7-methyl-imidazo[1,2-a]pyridin-3-yl)-amin | 64 | 295,38 | 296,4 |
| 142 | (2-Cyclohexyl-5,7-dimethyl-imidazo[1,2-a]pyridin-3-ylamino)-essigsäuremethylester | 59 | 315,41 | 316,4 |
| 313 | [7-Ethyl-2-(5-nitro-furan-2-yl)-imidazo[1,2-a]pyridin-3-yl]-(2-methoxy-benzyl)-amin | 32 | 392,42 | 393,9 |
| 314 | (2-Chlorbenzyl)-[7-ethyl-2-(5-nitro-furan-2-yl)-imidazo[1,2-a]pyridin-3-yl]-amin | 47 | 396,83 | 387,3 |
| 315 | [7-Ethyl-2-(5-methyl-furan-2-yl)-imidazo[1,2-a]pyridin-3-yl]-(2-methoxy-benzyl)-amin | 32 | 361,44 | 362,3 |
| 316 | (2-Chlor-benzyl)-(7-ethyl-2-furan-2-yl-imidazo[1,2-a]pyridin-3-yl)-amin | 52 | 351,84 | 352,4 |
| 317 | (3-Chlor-4-fluor-phenyl)-[7-ethyl-2-(5-methyl-furan-2-yl)-imidazo[1,2-a]pyridin-3-yl]-amin | 59 | 369,84 | 370,5 |
| 318 | (2-Benzofuran-2-yl-7-ethyl-imidazo[1,2-a]pyridin-3-yl)-(3-chloro-4-fluorophenyl)-amin | 52 | 405,86 | 406 |
| 319 | (2-Benzofuran-2-yl-7-ethyl-imidazo[1,2-a]pyridin-3-yl)-(3-chloro-phenyl)-amin | 53 | 387,87 | 388 |
| 320 | (3-Chlor-4-fluorphenyl)-{2-[5-(3-chlorphenyl)-furan-2-yl]-7-ethyl-imidazo[1,2-a]pyridin-3-yl}-amin | 52 | 466,34 | 466/468 |
| 321 | (3-Chlor-4-fluorphenyl)-{2-[5-(2-chlorphenyl)-furan-2-yl]-7-ethyl-imidazo[1,2-a]pyridin-3-yl}-amin | 50 | 466,34 | 466/468 |
| 322 | (3-Chlor-4-fluorphenyl)-[2-(4,5-dimethyl-furan-2-yl)-7-ethyl-imidazo[1,2-a]pyridin-3-yl]-amin | 49 | 383,85 | 384 |

Als Vergleichsbeispiel wurde 7-Nitroindazol in dem NOS-Assay getestet mit einer Hemmung (10 µM) von 50%.

### Allgemeine Arbeitsvorschrift 2 (AAV 2):

In einem mit einem Rührer versehenen Rundbodenröhrchen wurden ca. 0,05 mmol des jeweiligen nach AAV 1 erhaltenen Edukts der allgemeinen Struktur I in fester Form vorgelegt. Unter Rühren wurden bei 18 °C 2 ml Dichlormethan zugegeben. Die Lösung wurde mit 4 Äquivalenten Acetylchlorid (0,2 M Lösung in Dichlormethan) versetzt, und es wurde 4 h gerührt.

Anschließend wurde der Rührer entfernt, und die organischen Lösungen wurden in einer Vakuumzentrifuge bei 40-50 °C bis zur Trockene eingeengt. Zur Charakterisierung wurde ein ESI-MS aufgenommen.

Die gemäß AAV 2 hergestellten Beispiele 143-291 wurden im HTS-NOS-Assay (HTS) automatisiert getestet; die Ergebnisse sind in Tabelle 3 wiedergegeben.

**Tabelle 3**

| **Beispiel- Nr.** | **Verbindung** | **HTS-NOS- Assay: %- Hemmung (10 µM)** | **Masse** **berechnet** | **Masse** **gefunden** |
|---|---|---|---|---|
| 143 | N-(2-Furan-2-yl-5,7-dimethyl-imidazo[1,2-a]pyridin-3-yl)-N-(1,1,3,3-tetramethyl-butyl)-acetamid | 68 | 381,51 | (M-Acetyl) 340,5 |
| 144 | N-*tert*-Butyl-N-(7-methyl-2-pyridin-3-yl-imidazo[1,2-a]pyridin-3-yl)-acetamid | 59 | 322,41 | (M-Acetyl) 281,4 |
| 145 | N-*tert*-Butyl-N-(2-furan-2-yl-5,7-dimethyl-imidazo[1,2-a]pyridin-3-yl)-acetamid | 66 | 325,41 | (M-Acetyl) 284,3 |
| 146 | N-(5,7-Dimethyl-2-pyridin-4-yl-imidazo[1,2-a]pyridin-3-yl)-N-(1,1,3,3-tetramethyl-butyl)-acetamid | 59 | 392,54 | (M-Acetyl) 351,4 |
| 147 | N-(5,7-Dimethyl-2-pyridin-3-yl-imidazo[1,2-a]pyridin-3-yl)-N-(1,1,3,3-tetramethyl-butyl)-acetamid | 68 | 392,54 | (M-Acetyl) 351,5 |
| 148 | N-(2,6-Dimethyl-phenyl)-N-(5,7-dimethyl-2-o-tolyl-imidazo[1,2-a]pyridin-3-yl)-acetamid | 57 | 397,52 | (M-Acetyl) 356,4 |
| 149 | N-(2-Furan-2-yl-7-methyl-imidazo[1,2-a]pyridin-3-yl)-N-(1,1,3,3-tetramethyl-butyl)-acetamid | 55 | 367,49 | (M-Acetyl) 326,5 |
| 150 | N-(1,1,3,3-Tetramethyl-butyl)-N-(2,5,7-trimethyl-imidazo[1,2-a]pyridin-3-yl)-acetamid | 60 | 329,48 | (M-Acetyl) 288,5 |
| 151 | N-Cyclohexyl-N-(7-methyl-2-thiophen-2-yl-imidazo[1,2-a]pyridin-3-yl)-acetamid | 65 | 353,48 | (M-Acetyl) 312,5 |
| 152 | N-*tert*-Butyl-N-(2,5,7-trimethyl-imidazo[1,2-a]pyridin-3-yl)-acetamid | 66 | 273,37 | (M-Acetyl) 232,3 |
| 153 | 5-[3-(Acetyl-*tert*-butyl-amino)-imidazo[1,2-a]pyrazin-2-yl]-thiophen-2-carbonsäure | 78 | 358,41 | (M-Acetyl) 317,5 |
| 154 | 5-{3-[Acetyl-(1,1,3,3-tetramethyl-butyl)-amino]-5,7-dimethyl-imidazo[1,2-a]pyridin-2-yl}-furan-2-carbonsäure | 60 | 425,52 | (M-Acetyl) 384,6 |
| 155 | N-[2-(5-Hydroxymethyl-furan-2-yl)-5,7-dimethyl-imidazo[1,2-a]pyridin-3-yl]-N-(1,1,3,3-tetramethyl-butyl)-acetamid | 50 | 411,54 | 370,4; (M-Acetyl) 412,4 |
| 156 | N-[2-(3-Brom-thiophen-2-yl)-5,7-dimethyl-imidazo[1,2-a]pyridin-3-yl]-N-(2,6-dimethyl-phenyl)-acetamid | 74 | 468,42 | (M-Acetyl) 426,3/428,3 |
| 157 | N*-tert*-Butyl-N-(2-cyclohexyl-5,7-dimethyl-imidazo[1,2-a]pyridin-3-yl)-acetamid | 63 | 341,49 | (M-Acetyl) 300,4 |
| 158 | Essigsäure 5-[3-(acetyl-cyclohexyl-amino)-5,7-dimethyl-imidazo[1,2-a]pyridin-2-yl]-furan-2-ylmethyl ester | 54 | 423,51 | (M-Acetyl) 382,4 |
| 159 | {6-[Acetyl-(2-furan-2-yl-5,7-dimethyl-imidazo[1,2-a]pyridin-3-yl)-amino]-hexyl}-methylidyne-ammonium | 61 | 379,48 | (M-Acetyl) 337,4 |
| 160 | N-[2-(2,3-Dichlor-phenyl)-7-methyl-imidazo[1,2-a]pyridin-3-yl]-N-(2,6-dimethyl-phenyl)-acetamid | 55 | 438,35 | (M-Acetyl) 396,4/ 398,3 |
| 161 | N-[2-(3-Brom-thiophen-2-yl)-imidazo[1,2-a]pyridin-3-yl]-N-cyclohexyl-acetamid | 51 | 418,36 | (M-Acetyl) 376,5/ 378,4 |
| 162 | N-(5,7-Dimethyl-2-p-tolyl-imidazo[1,2-a]pyridin-3-yl)-N-(1,1,3,3-tetramethyl-butyl)-acetamid | 67 | 405,58 | (M-Acetyl) 364,4 |
| 163 | N-Cyclohexyl-N-(7-methyl-2-pyridin-4-yl-imidazo[1,2-a]pyridin-3-yl)-acetamid | 63 | 348,44 | (M-Acetyl) 307,4 |
| 164 | N-Cyclohexyl-N-[7-methyl-2-(2-trifluormethyl-phenyl)-imidazo[1,2-a]pyridin-3-yl]-acetamid | 61 | 415,45 | (M-Acetyl) 374,5 |
| 165 | N-(6,8-Dibrom-2-furan-2-yl-imidazo[1,2-a]pyridin-3-yl)-N-(1,1,3,3-tetramethyl-butyl)-acetamid | 67 | 511,26 | (M-Acetyl) 468,3/ 470,2/ 472,2 |
| 166 | N-(7-Methyl-2-pyridin-4-yl-imidazo[1,2-a]pyridin-3-yl)-N-(1,1,3,3-tetramethyl-butyl)-acetamid | 61 | 378,51 | (M-Acetyl) 337,4 |
| 167 | Acetic acid 5-[3-(acetyl-cyclohexyl-amino)-7-methyl-imidazo[1,2-a]pyridin-2-yl]-furan-2-ylmethyl ester | 68 | 409,48 | (M-Acetyl) 368,5 |
| 168 | N-(7-Methyl-2-pyridin-3-yl-imidazo[1,2-a]pyridin-3-yl)-N-(1,1,3,3-tetramethyl-butyl)-acetamid | 56 | 378,51 | (M-Acetyl) 337,4 |
| 169 | N-[2-(2,3-Dichlor-phenyl)-5,7-dimethyl-imidazo[1,2-a]pyridin-3-yl]-N-(2,6-dimethyl-phenyl)-acetamid | 57 | 452,38 | (M-Acetyl) 410,3/ 412,2/ 413,2 |
| 170 | N-Cyclohexyl-N-[5,7-dimethyl-2-(5-methyl-furan-2-yl)-imidazo[1,2-a]pyridin-3-yl]-acetamid | 65 | 365,47 | (M-Acetyl) 324,4 |
| 171 | N-Butyl-N-[2-(2,3-dimethoxy-phenyl)-5,7-dimethyl-imidazo[1,2-a]pyridin-3-yl]-acetamid | 63 | 395,5 | (M-Acetyl) 354,4 |
| 172 | N-[2-(2-Methoxy-phenyl)-5,7-dimethyl-imidazo[1,2-a]pyridin-3-yl]-N-(1,1,3,3-tetramethyl-butyl)-acetamid | 62 | 421,58 | (M-Acetyl) 380,4 |
| 173 | N-Cyclohexyl-N-[5,7-dimethyl-2-(5-nitro-furan-2-yl)-imidazo[1,2-a]pyridin-3-yl]-acetamid | 67 | 396,44 | (M-Acetyl) 355,4 |
| 174 | [Acetyl-(2,7-dimethyl-imidazo[1,2-a]pyridin-3-yl)-amino]-acetic acid methyl ester | 68 | 275,3 | (M-Acetyl) 234,4 |
| 175 | N-Cyclohexyl-N-(2,5,7-trimethyl-imidazo[1,2-a]pyridin-3-yl)-acetamid | 52 | 299,41 | (M-Acetyl) 258,4 |
| 176 | 5-{3-[Acetyl-(1,1,3,3-tetramethyl-butyl)-amino]-imidazo[1,2-a]pyridin-2-yl}-thiophen-2-carbonsäure | 50 | 413,53 | (M-Acetyl) 372,5 |
| 177 | N-[2-(2,4-Dichlor-phenyl)-5,7-dimethyl-imidazo[1,2-a]pyridin-3-yl]-N-(1,1,3,3-tetramethyl-butyl)-acetamid | 67 | 460,44 | (M-Acetyl) 418,3/ 420,3/ 421,2 |
| 178 | N-Cyclohexyl-N-[7-methyl-2-(5-nitro-furan-2-yl)-imidazo[1,2-a]pyridin-3-yl]-acetamid | 64 | 382,41 | (M-Acetyl) 341,5 |
| 179 | N-(2-*tert*-Butyl-5,7-dimethyl-imidazo[1,2-a]pyridin-3-yl)-N-(2,6-dimethyl-phenyl)-acetamid | 60 | 363,5 | (M-Acetyl) 322,4 |
| 180 | N-(2,6-Dimethyl-phenyl)-N-[2-(2-methoxy-phenyl)-5,7-dimethyl-imidazo[1,2-a]pyridin-3-yl]-acetamid | 54 | 413,52 | (M-Acetyl) 372,4 |
| 181 | N-[2-(3-Hydroxy-phenyl)-5,7-dimethyl-imidazo[1,2-a]pyridin-3-yl]-N-(1,1,3,3-tetramethyl-butyl)-acetamid | 60 | 407,55 | 408,3; (M-Acetyl) 366,4 |
| 182 | N-(2,6-Dimethyl-phenyl)-N-[2-(2-fluor-phenyl)-5,7-dimethyl-imidazo[1,2-a]pyridin-3-yl]-acetamid | 53 | 401,48 | (M-Acetyl) 360,4 |
| 183 | 5-[3-(Acetyl-*tert*-butyl-amino)-5-methyl-imidazo[1,2-a]pyridin-2-yl]-thiophen-2-carbonsäure | 50 | 371,45 | (M-Acetyl) 330,4 |
| 184 | N-(2,6-Dimethyl-phenyl)-N-[2-(2-methoxy-phenyl)-7-methyl-imidazo[1,2-a]pyridin-3-yl]-acetamid | 51 | 399,49 | (M-Acetyl) 358,5 |
| 185 | N-(7-Methyl-2-p-tolyl-imidazo[1,2-a]pyridin-3-yl)-N-(1,1,3,3-tetramethyl-butyl)-acetamid | 55 | 391,55 | (M-Acetyl) 350,4 |
| 186 | 5-{3-[Acetyl-(1,1,3,3-tetramethyl-butyl)-amino]-7-methyl-imidazo[1,2-a]pyridin-2-yl}-furan-2-carbonsäure | 65 | 411,5 | (M-Acetyl) 370,5 |
| 187 | N-Cyclohexyl-N-(7-methyl-2-pyridin-3-yl-imidazo[1,2-a]pyridin-3-yl)-acetamid | 67 | 348,44 | (M-Acetyl) 307,4 |
| 188 | N-[2-(5-[1,3]Dioxolan-2-yl-furan-2-yl)-5,7-dimethyl-imidazo[1,2-a]pyridin-3-yl]-N-(1,1,3,3-tetramethyl-butyl)-acetamid | 50 | 453,58 | (M-Acetyl) 412,3 |
| 189 | N-(2-Benzofuran-2-yl-5,7-dimethyl-imidazo[1,2-a]pyridin-3-yl)-N-(2,6-dimethyl-phenyl)-acetamid | 63 | 423,51 | (M-Acetyl) 382,5 |
| 190 | N-*tert*-Butyl-N-(2-furan-2-yl-7-methyl-imidazo[1,2-a]pyridin-3-yl)-acetamid | 69 | 311,38 | (M-Acetyl) 270,3 |
| 191 | N-*tert*-Butyl-N-(7-methyl-2-pyridin-2-yl-imidazo[1,2-a]pyridin-3-yl)-acetamid | 57 | 322,41 | (M-Acetyl) 281,3 |
| 192 | N-Cyclohexyl-N-(5,7-dimethyl-2-pyridin-3-yl-imidazo[1,2-a]pyridin-3-yl)-acetamid | 72 | 362,47 | (M-Acetyl) 321,4 |
| 193 | N-[2-(2,3-Dichlor-phenyl)-5,7-dimethyl-imidazo[1,2-a]pyridin-3-yl]-N-(1,1,3,3-tetramethyl-butyl)-acetamid | 55 | 460,44 | (M-Acetyl) 418,3/ 420,3/ 421,1 |
| 194 | N-[2-(2,3-Dimethoxy-phenyl)-5,7-dimethyl-imidazo[1,2-a]pyridin-3-yl]-N-(1,1,3,3-tetramethyl-butyl)-acetamid | 59 | 451,61 | (M-Acetyl) 410,4 |
| 195 | N-{2-[3-(4-chlor-phenoxy)-phenyl]-imidazo[1,2-a]pyridin-3-yl}-N-(2,6-dimethyl-phenyl)-acetamid | 54 | 481,98 | (M-Acetyl) 440,4/ 441,4/ 442,4 |
| 196 | N-[2-(5-[1,3]Dioxolan-2-yl-furan-2-yl)-7-methyl-imidazo[1,2-a]pyridin-3-yl]-N-(1,1,3,3-tetramethyl-butyl)-acetamid | 54 | 439,55 | (M-Acetyl) 398,4 |
| 197 | 5-[3-(Acetyl-cyclohexyl-amino)-5,7-dimethyl-imidazo[1,2-a]pyridin-2-yl]-furan-2-carbonsäure | 70 | 395,45 | (M-Acetyl) 354,4 |
| 198 | N-*tert*-Butyl-N-[7-methyl-2-(5-nitro-furan-2-yl)-imidazo[1,2-a]pyridin-3-yl]-acetamid | 54 | 356,38 | (M-Acetyl) 314,4 |
| 199 | N-[2-(2-Methoxy-phenyl)-7-methyl-imidazo[1,2-a]pyridin-3-yl]-N-(1,1,3,3-tetramethyl-butyl)-acetamid | 57 | 407,55 | (M-Acetyl) 366,4 |
| 200 | N-[2-(5-Methyl-furan-2-yl)-imidazo[1,2-a]pyridin-3-yl]-N-(1,1,3,3-tetramethyl-butyl)-acetamid | 58 | 367,49 | (M-Acetyl) 326,4/ 327,4 |
| 201 | 5-[3-(Acetyl-*tert*-butyl-amino)-7-methyl-imidazo[1,2-a]pyridin-2-yl]-furan-2-carbonsäure | 54 | 355,39 | (M-Acetyl) 314,4 |
| 202 | N-[2-(4,5-Dimethyl-furan-2-yl)-imidazo[1,2-a]pyridin-3-yl]-N-(1,1,3,3-tetramethyl-butyl)-acetamid | 57 | 381,51 | (M-Acetyl) 340,6 |
| 203 | N-Cyclohexyl-N-(2-furan-2-yl-5,7-dimethyl-imidazo[1,2-a]pyridin-3-yl)-acetamid | 60 | 351,44 | (M-Acetyl) 310,4 |
| 204 | N-*tert*-Butyl-N-(7-methyl-2-naphthalen-1-yl-imidazo[1,2-a]pyridin-3-yl)-acetamid | 54 | 371,48 | (M-Acetyl) 330,4 |
| 205 | 5-{3-[Acetyl-(1,1,3,3-tetramethyl-butyl)-amino]-imidazo[1,2-a]pyrazin-2-yl}-thiophen-2-carbonsäure | 52 | 414,52 | (M-Acetyl) 373,4 |
| 206 | N-Butyl-N-(2-o-tolyl-imidazo[1,2-a]pyrimidin-3-yl)-acetamid | 54 | 322,41 | 339,4/ 340,4 |
| 207 | N-[2-(3,4-Dimethoxy-phenyl)-7-methyl-imidazo[1,2-a]pyridin-3-yl]-N-(2,6-dimethyl-phenyl)-acetamid | 51 | 429,51 | (M-Acetyl) 388,4 |
| 208 | N-*tert*-Butyl-N-(7-methyl-2-phenanthren-9-yl-imidazo[1,2-a]pyridin-3-yl)-acetamid | 63 | 421,54 | (M-Acetyl) 380,5 |
| 209 | N-(2,6-Dimethyl-phenyl)-N-[2-(2-fluor-phenyl)-7-methyl-imidazo[1,2-a]pyridin-3-yl]-acetamid | 62 | 387,45 | (M-Acetyl) 346,4/ 347,3 |
| 210 | N-[2-(2-Methoxy-phenyl)-8-methyl-imidazo[1,2-a]pyridin-3-yl]-N-(1,1,3,3-tetramethyl-butyl)-acetamid | 53 | 407,55 | (M-Acetyl) 366,4 |
| 211 | N-(2,6-Dimethyl-phenyl)-N-[2-(3-hydroxy-phenyl)-5,7-dimethyl-imidazo[1,2-a]pyridin-3-yl)-acetamid | 73 | 399,49 | (M-Acetyl) 358,4 |
| 212 | N-(2-*tert*-Butyl-7-methyl-imidazo[1,2-a]pyridin-3-yl)-N-(2,6-dimethyl-phenyl)-acetamid | 68 | 349,47 | (M-Acetyl) 308,4 |
| 213 | Acetic acid 4-{3-[acetyl-(2,6-dimethyl-phenyl)-amino]-6-brom-8-methyl-imidazo[1,2-a]pyridin-2-yl}-2-methoxy-phenyl ester | 69 | 536,43 | (M-Acetyl) 494,3/ 497,3 |
| 214 | N-*tert*-Butyl-N-[2-(5-[1,3]dioxolan-2-yl-furan-2-yl)-7-methyl-imidazo[1,2-a]pyridin-3-yl]-acetamid | 56 | 383,44 | (M-Acetyl) 336,2 |
| 215 | [6-(Acetyl-{7-methyl-2-[5-(2-nitro-phenyl)-furan-2-yl]-imidazo[1,2-a]pyridin-3-yl}-amino)-hexyl]-methylidyne-ammonium | 50 | 486,55 | 487,5; (M-Acetyl) 444,5 |
| 216 | N-(2-Benzofuran-2-yl-7-methyl-imidazo[1,2-a]pyridin-3-yl)-N-(1,1,3,3-tetramethyl-butyl)-acetamid | 50 | 417,55 | (M-Acetyl) 376,4/ 377,3 |
| 217 | N-(2-Benzofuran-2-yl-5,7-dimethyl-imidazo[1,2-a]pyridin-3-yl)-N-(1,1,3,3-tetramethyl-butyl)-acetamid | 63 | 431,57 | (M-Acetyl) 390,4/ 391,4 |
| 218 | 5-[3-(Acetyl-*tert*-butyl-amino)-imidazo[1,2-a]pyridin-2-yl]-thiophen-2-carbonsäure | 60 | 357,43 | (M-Acetyl) 316,5 |
| 219 | N-(2-Cyclohexyl-5,7-dimethyl-imidazo[1,2-a]pyridin-3-yl)-N-(1,1,3,3-tetramethyl-butyl)-acetamid | 62 | 397,6 | (M-Acetyl) 356,5 |
| 220 | N-*tert*-Butyl-N-[2-(5-methyl-furan-2-yl)-imidazo[1,2-a]pyridin-3-yl]-acetamid | 61 | 311,38 | (M-Acetyl) 270,4 |
| 221 | N-*tert*-Butyl-N-[2-(5-methylsulfanyl-thiophen-2-yl)-imidazo[1,2-a]pyrimidin-3-yl]-acetamid | 54 | 360,5 | (M-Acetyl) 319,4 |
| 222 | N-[2-(4,5-Dimethyl-furan-2-yl)-5-methyl-imidazo[1,2-a]pyridin-3-yl]-N-(1,1,3,3-tetramethyl-butyl)-acetamid | 53 | 395,54 | (M-Acetyl) 354,5 |
| 223 | N-Bulyl-N-[2-(2,4-dichlor-phenyl)-imidazo[1,2-a]pyrimidin-3-yl]-acetamid | 62 | 377,27 | 377,4/ 379,4 |
| 224 | N-[2-(3-Brom-thiophen-2-yl)-6-methyl-imidazo[1,2-a]pyridin-3-yl]-N-cyclohexyl-acetamid | 53 | 432,38 | (M-Acetyl) 390,4/ 392, 4 |
| 225 | 5-{3-[Acetyl-(2,6-dimethyl-phenyl)-amino]-6-methyl-imidazo[1,2-a]pyridin-2-yl}-thiophen-2-carbonsäure | 55 | 419,5 | (M-Acetyl) 378,4 |
| 226 | N-Butyl-N-[2-(2,3-dimethoxy-phenyl)-7-methyl-imidazo[1,2-a]pyridin-3-yl]-acetamid | 62 | 381,47 | 382,5 |
| 227 | N-*tert*-Butyl-N-[2-(2,3-dichlor-phenyl)-imidazo[1,2-a]pyridin-3-yl]-acetamid | 54 | 376,28 | (M-Acetyl) 334,3/ 336,3 |
| 228 | N-(2-Furan-2-yl-5-propyl-imidazo[1,2-a]pyridin-3-yl)-N-(1,1,3,3-tetramethyl-butyl)-acetamid | 53 | 395,54 | (M-Acetyl) 354,4 |
| 229 | 5-[3-(Acetyl-cyclohexyl-amino)-imidazo[1,2-a]pyridin-2-yl]-thiophen-2-carbonsäure | 59 | 383,46 | (M-Acetyl) 342,5 |
| 230 | 5-{3-[Acetyl-(1,1,3,3-tetramethyl-butyl)-amino]-8-methyl-imidazo[1,2-a]pyridin-2-yl}-furan-2-carbonsäure | 52 | 411,5 | (M-Acetyl) 370,6 |
| 231 | 3-(Acetyl-butyl-amino)-2-pyridin-2-yl-imidazo[1,2-a]pyridin-8-carbonsäure | 52 | 352,39 | 353,5 |
| 232 | {6-[Acetyl-(5,7-dimethyl-2-pyridin-3-yl-imidazo[1,2-a]pyridin-3-yl)-amino]-hexyl}-methylidyne-ammoium | 59 | 390,51 | (M-Acetyl) 348,5 |
| 233 | N-*tert*-Butyl-N-[2-(5-methylsulfanyl-thiophen-2-yl)-imidazo[1,2-a]pyrazin-3-yl]-acetamid | 53 | 360,5 | (M-Acetyl) 319,2 |
| 234 | 5-[3-(Acetyl-cyctohexyl-amino)-5-methyl-imidazo[1,2-a]pyridin-2-yl]-thiophen-2-carbonsäure | 58 | 397,49 | (M-Acetyl) 356,4 |
| 235 | N-[2-(5-Methylsulfanyl-thiophen-2-yl)-imidazo[1,2-a]pyrazin-3-yl]-N-(1,1,3,3-tetramethyl-butyl)-acetamid | 58 | 416,6 | (M-Acetyl) 375,3 |
| 236 | N-[2-(2,3-Dichlor-phenyl)-5-methyl-imidazo[1,2-a]pyridin-3-yl]-N-(2,6-dimethyl-phenyl)-acetamid | 58 | 438,35 | (M-Acetyl) 396,3/ 398,3 |
| 237 | N-Butyl-N-[2-(2-methoxy-phenyl)-imidazo[1,2-a]pyridin-3-yl]-acetamid | 55 | 337,42 | 338,5 |
| 238 | (6-{Acetyl-[2-(2-methoxy-phenyl)-6-nitro-imidazo[1,2-a]pyridin-3-yl]-amino}-hexyl)-methylidyne-ammonium | 52 | 436,49 | 436,5 |
| 239 | N-(2-Benzofuran-2-yl-7-methyl-imidazo[1,2-a]pyridin-3-yl)-N-(2,6-dimethyl-phenyl)-acetamid | 52 | 409,48 | (M-Acetyl) 368,5 |
| 240 | (6-{Acetyl-[2-(2-methoxy-phenyl)-5,7-dimethyl-imidazo[1,2-a]pyridin-3-yl]-amino}-hexyl)-methylidyne-ammonium | 61 | 419,54 | (M-Acetyl) 377,5 |
| 241 | {6-[Acetyl-(7-methyl-2-pyridin-3-yl-imidazo[1,2-a]pyridin-3-yl)-amino]-hexyl}-methylidyne-ammonium | 62 | 376,48 | (M-Acetyl) 334,5 |
| 242 | N-(6-Methyl-2-p-tolyl-imidazo[1,2-a]pyridin-3-yl)-N-(1,1,3,3-tetramethyl-butyl)-acetamid | 58 | 391,55 | (M-Acetyl) 350,4 |
| 243 | Acetic acid 5-{3-[acetyl-(2,6-dimethyl-phenyl)-amino]-5,7-dimethyl-imidazo[1,2-a]pyridin-2-yl}-furan-2-ylmethyl ester | 62 | 445,51 | (M-Acetyl) 404,4 |
| 244 | {Acetyl-[2-(3-hydroxy-phenyl)-6-methyl-imidazo[1,2-a]pyridin-3-yl]-amino}-acetic acid methyl ester | 50 | 353,37 | 354,4; (M-Acetyl) 312,4 |
| 245 | N-*tert*-Butyl-N-[2-(2-trifluormethyl-phenyl)-imidazo[1,2-a]pyridin-3-yl]-acetamid | 56 | 375,39 | (M-Acetyl) 334,3 |
| 246 | N-Butyl-N-[2-(2-chlor-4-fluor-phenyl)-imidazo[1,2-a]pyridin-3-yl]-acetamid | 55 | 359,83 | 360,4 |
| 247 | N-[2-(2,4-Dichlor-phenyl)-imidazo[1,2-a]pyridin-3-yl]-N-(2,6-dimethyl-phenyl)-acetamid | 56 | 424,33 | (M-Acetyl) 382,4/ 384,3 |
| 248 | 5-{3-[Acetyl-(1,1,3,3-tetramethyl-butyl)-amino]-7-methyl-imidazo[1,2-a]pyrimidin-2-yl}-furan-2-carbonsäure | 52 | 412,48 | (M-Acetyl) 371,8 |
| 249 | Acetic acid 5-{3-[acetyl-(1,1,3,3-tetramethyl-butyl)-amino]-imidazo[1,2-a]pyrimidin-2-yl}-furan-2-ylmethyl ester | 54 | 426,51 | (M-Acetyl) 385,4 |
| 250 | N-(2,7-Dimethyl-imidazo[1,2-a]pyridin-3-yl)-N-(1,1,3,3-tetramethyl-butyl)-acetamid | 58 | 315,46 | (M-Acetyl) 274,5 |
| 251 | Acetic acid 4-[3-(acetyl-cyclohexyl-amino)-5-amino-7-chlor-imidazo[1,2-a]pyrimidin-2-yl]-2-methoxy-phenyl ester | 51 | 471,94 | 472,4; (M-Acetyl) 430,4/ 432,4 |
| 252 | Acetic acid 4-[3-(acetyl-cyclohexyl-amino)-5,7-dimethy)-imidazo[1,2-a]pyrimidin-2-yl]-2-methoxy-phenyl ester | 53 | 450,53 | (M-Acetyl) 409,5 |
| 253 | N-[6-Brom-2-(2-chlor-6-fluor-phenyl)-8-methyl-imidazo[1,2-a]pyridin-3-yl)-N-cyclohexyl-acetamid | 51 | 478,79 | (M-Acetyl) 436,4/ 438,3/ 440,3 |
| 254 | N-[2-(2-chlor-6-fluor-phenyl)-5-methyl-imidazo[1,2-a]pyridin-3-yl]-N-cyclohexyl-acetamid | 51 | 399,89 | (M-Acetyl) 358,3 |
| 255 | N-Butyl-N-[2-(2,3-dichlor-phenyl)-imidazo[1,2-a]pyrimidin-3-yl]-acetamid | 65 | 377,27 | 377,4/ 379,4 |
| 256 | N-[2-(5-chlor-thiophen-2-yl)-imidazo[1,2-a]pyrimidin-3-yl]-N-(1,1,3,3-tetramethyl-butyl)-acetamid | 55 | 404,96 | (M-Acetyl) 363,3/ 365,3/ 367,3 |
| 257 | [Acetyl-(2-cyclohexyl-7-methyl-imidazo[1,2-a]pyridin-3-yl)-amino]-acetic acid methyl ester | 56 | 343,42 | (M-Acetyl) 302,5 |
| 258 | N-*tert*-Butyl-N-[2-(2-chlor-6-fluor-phenyl)-imidazo[1,2-a]pyridin-3-yl]-acetamid | 60 | 359,83 | (M-Acetyl) 318,3/ 320,3 |
| 259 | N-Cyclohexyl-N-(5,7-dimethyl-2-pyridin-2-yl-imidazo[1,2-a]pyridin-3-yl)-acetamid | 73 | 362,47 | (M-Acetyl) 321,4 |
| 260 | Acetic acid 5-[3-(acetyl-cyclohexyl-amino)-5-methyl-imidazo[1,2-a]pyridin-2-yl]-furan-2-ylmethyl ester | 51 | 409,48 | (M-Acetyl) 368,6 |
| 261 | N-(2,6-Dimethyl-phenyl)-N-[6-methyl-2-(2-trifluormethyl-phenyl)-imidazo[1,2-a]pyridin-3-yl]-acetamid | 61 | 437,46 | (M-Acetyl) 396,4 |
| 262 | N-Cyclohexyl-N-(2-furan-2-yl-7-methyl-imidazo[1,2-a]pyridin-3-yl)-acetamid | 54 | 337,42 | (M-Acetyl) 296, 5 |
| 263 | N-Cyclohexyl-N-(7-methyl-2-pyridin-2-yl-imidazo[1,2-a]pyridin-3-yl)-acetamid | 64 | 348,44 | 349,4; (M-Acetyl) 307,4 |
| 264 | N-Cyclohexyl-N-[2-(5-[1,3]dioxolan-2-yl-furan-2-yl)-5-methyl-imidazo[1,2-a]pyridin-3-yl]-acetamid | 50 | 409,48 | (M-Acetyl) 368,4 |
| 265 | N-*tert*-Butyl-N-(5-propyl-2-pyridin-3-yl-imidazo[1,2-a]pyridin-3-yl)-acetamid | 54 | 350,46 | (M-Acetyl) 309,3 |
| 266 | N-*tert*-Butyl-N-[2-(5-methyl-thiophen-2-yl)-imidazo[1,2-a]pyrimidin-3-yl]-acetamid | 53 | 328,43 | (M-Acetyl) 287,3 |
| 267 | 3-[Acetyl-(1,1,3,3-tetramethyl-butyl)-amino]-2-furan-2-yl-imidazo[1,2-a]pyridin-8-carbonsäure | 62 | 397,47 | (M-Acetyl) 356,7 |
| 268 | N-*tert*-Butyl-N-[2-(4,5-dimethyl-furan-2-yl)-6-methyl-imidazo[1,2-a]pyridin-3-yl]-acetamid | 51 | 339,43 | (M-Acetyl) 298,4 |
| 269 | N-{2-[3-(4-chlor-phenoxy)-phenyl]-imidazo[1,2-a]pyridin-3-yl}-N-cyclohexyl-acetamid | 51 | 459,97 | 460,4/ 462,4; (M - Acetyl) 418,5/ 419,4 |
| 270 | Acetic acid 4-[3-(acetyl-cyclohexyl-amino)-imidazo[1,2-a]pyrimidin-2-yl]-2-methoxy-phenyl ester | 52 | 422,48 | 423,4; (M-Acetyl) 381,4 |
| 271 | N-[2-(5-Brom-furan-2-yl)-8-methyl-imidazo[1,2-a]pyridin-3-yl]-N-(1,1,3,3-tetramethyl-butyl)-acetamid | 51 | 446,39 | (M-Acetyl) 404,4/ 406,3 |
| 272 | N-(2,6-Dimethyl-phenyl)-N-[2-(3-hydroxy-phenyl)-5,7-dimethyl-imidazo[1,2-a]pyrimidin-3-yl]-acetamid | 54 | 400,48 | 359,5 (M-Acetyl) 401,4 |
| 273 | N-Cyclohexyl-N-[2-(2,3-dichlor-phenyl)-imidazo[1,2-a]pyridin-3-yl]-acetamid | 55 | 402,32 | (M-Acetyl) 360,4/ 362,4 |
| 274 | N-Cyclohexyl-N-[2-(2,4-dichlor-phenyl)-5-methyl-imidazo[1,2-a]pyridin-3-yl]-acetamid | 64 | 416,35 | (M-Acetyl) 374,4/ 376,3 |
| 275 | N-Cyclohexyl-N-[2-(2,4-dichlor-phenyl)-imidazo[1,2-a]pyrimidin-3-yl]-acetamid | 59 | 403,31 | (M-Acetyl) 361,4/ 363,3 |
| 276 | [Acetyl-(2-o-tolyl-imidazo[1,2-a]pyrazin-3-yl)-amino]-acetic acid methyl ester | 50 | 338,36 | (M-Acetyl) 297,4 |
| 277 | N-*tert*-Butyl-N-(6,8-dichlor-2-thiophen-2-yl-imidazo[1,2-a]pyridin-3-yl)-acetamid | 53 | 382,31 | (M-Acetyl) 340,3/ 342,2 |
| 278 | N-*tert*-Butyl-N-(5-propyl-2-thiophen-2-yl-imidazo[1,2-a]pyridin-3-yl)-acetamid | 55 | 355,5 | (M-Acetyl) 340,3/ 342,2 |
| 279 | {6-[Acetyl-(7-methyl-2-p-tolyl-imidazo[1,2-a]pyridin-3-yl)-amino]-hexyl}-methylidyne-ammonium | 71 | 389,52 | 389,6 (M-Acetyl) 347,6 |
| 280 | N-Butyl-N-(6-methyl-2-p-tolyl-imidazo[1,2-a]pyridin-3-yl)-acetamid | 53 | 335,45 | 336,5; (M-Acetyl) 294,5 |
| 281 | (6-{Acetyl-[2-(2-methoxy-phenyl)-7-methyl-imidazo[1,2-a]pyridin-3-yl]-amino}-hexyl)-methylidyne-ammonium | 62 | 405,52 | 405,5 |
| 282 | 5-{3-[Acetyl-(1,1,3,3-tetramethyl-butyl)-amino]-6-methyl-imidazo[1,2-a]pyridin-2-yl}-furan-2-carbonsäure | 51 | 411,5 | (M-Acetyl) 370,4 |
| 283 | N-Butyl-N-[2-(3,4,5-trimethoxy-phenyl)-imidazo[1,2-a]pyrazin-3-yl]-acetamid | 51 | 398,46 | 399,5, (M-Acetyl) 357,5 |
| 284 | N-Butyl-N-[2-(3-hydroxy-phenyl)-imidazo[1,2-a]pyrimidin-3-yl]-acetamid | 51 | 324,38 | 325,4; (M-Acetyl) 283,3 |
| 285 | [Acetyl-(2-o-tolyl-imidazo[1,2-a]pyrimidin-3-yl)-amino]-acetic acid methyl ester | 51 | 338,36 | 339,3; (M-Acetyl) 297,4 |
| 286 | N-(2-Benzofuran-2-yl-8-methyl-imidazo[1,2-a]pyridin-3-yl)-N-(1,1,3,3-tetramethyl-butyl)-acetamid | 59 | 417,55 | (M-Acetyl) 376,4/ 377,4 |
| 287 | N-Butyl-N-(7-methyl-2-p-tolyl-imidazo[1,2-a]pyrimidin-3-yl)-acetamid | 58 | 336,43 | 337,5 |
| 288 | N-*tert*-Butyl-N-(6,8-dibrom-2-methyl-imidazo[1,2-a]pyridin-3-yl)-acetamid | 62 | 403,12 | (M-Acetyl) 362,2 |
| 289 | {6-[Acetyl-(5,7-dimethyl-2-pyridin-2-yl-imidazo[1,2-a]pyridin-3-yl)-amino]-hexyl}-methylidyne-ammonium | 63 | 390,51 | (M-Acetyl) 348,5 |
| 290 | N-*tert*-Butyl-N-[2-(2-ethoxy-naphthalen-1-yl)-7-methyl-imidazo[1,2-a]pyridin-3-yl]-acetamid | 67 | 415,53 | (M-Acetyl) 374,4 |
| 291 | N-*tert*-Butyl-N-[2-(2-chlor-4-fluorphenyl)-imidazo[1,2-a]pyridin-3-yl]-acetamid | 53 i | 359,83 | (M-Acetyl) 318,3/ 319,2 |

### Allgemeine Arbeitsvorschrift 3 (AAV 3)

### (Äquivalente bedeuten Stoffmengenäquivalente zum eingesetzten Isonitril):

Im Reaktionsgefäß wurde zunächst 1,15 Äquivalente des heterocyclischen Amins der allgemeinen Struktur II in Dichlormethan (2 ml je mmol eingesetztem Isonitril IV) suspendiert bzw. gelöst. Hierzu wurden nacheinander 1,5 Äquivalente Aldehyd III, ein Äquivalent Isonitril IV und schließlich wäßrige Perchlorsäurelösung (20 m%; 0,098 ml je mmol eingesetztem Isonitril) zugegeben und der Ansatz für zwanzig Stunden bei Raumtemperatur gerührt.

Zur Aufarbeitung wurden gesättigte Natriumchloridlösung (ca. 5 ml je mmol eingesetztem Isonitril) und Dichlormethan (ca. 4 ml je mmol eingesetztem Isonitril) zugegeben, die Phasen getrennt und die organische Phase noch zweimal mit Dichlormethan (je ca. 2 ml je mmol eingesetztem isonitril) extrahiert. Die vereinigten organischen Phasen wurden nacheinander mit Pufferlösung (pH 10; Merck Art.-Nr. 1.09438.1000; ca. 2 ml je mmol eingesetztem Isonitril) und ges. Natriumchloridlösung (ca. 2 ml je mmol eingesetztem Isonitril) gewaschen, über Natriumsulfat getrocknet, filtriert, am Rotationsverdampfer im Vakuum eingeengt und im Ölpumpenvakuum von Lösungsmittelresten befreit.

Das erhaltene Rohprodukt wurde entweder direkt einer Hydrochloridfällung zugeführt (Lösen der Rohbase in ca. 10 ml 2-Butanon je Gramm Base; Zugabe eines halben Moläquivalents Wasser, gefolgt von 1,1 Moläquivalenten Chlortrimethylsilan und Rühren über Nacht.), oder mit Hexan (ca. 10 ml je mmol eingesetztem Isonitril) unter Rühren zum Rückfluß erhitzt. Löste sich das Rohprodukt nicht vollständig wurde heiß abdekantiert. Nach Erkalten der Hexanlösung wurde ein gegebenenfalls erhaltener Feststoff abfiltriert und im Ölpumpenvakuum getrocknet. Etwaige Nachfällungen wurden getrennt analog behandelt. Das erhaltene Filtrat wurde am Rotationsverdampfer eingeengt und der Rückstand wiederum im Ölpumpenvakuum getrocknet. Auf diesem Wege wurden bis zu vier Fraktionen erhalten:
- 0:: Keine Behandlung mit Hexan
- 1:: In Hexan unlöslicher Rückstand
- 2:: Aus Hexanlösung beim Erkalten ausgefallener Feststoff
- 3:: Nachfällung
- 4:: Rückstand aus zur Trockne eingeengter Hexanlösung

Aus den jeweils erhaltenen Fraktionen wurde durch dünnschichtchromatographische und/oder NMR-spektroskopische Untersuchungen die Produktfraktion(en) identifiziert (in der Regel der aus der Hexanlösung ausgefallene Feststoff).

Von einem Teil einer Produktfraktion wurde schließlich ein Hydrochlorid gefällt (s.o.).

Die gemäß AAV 3 hergestellten Beispiele 292-298 wurden im Citrullin-Assay getestet; die Ergebnisse sind in Tabelle 4 wiedergegeben. Ferner wurden beispielhaft gemäß AAV 3 hergestellt: Cyclohexyl-(7-methyl-2-phenyl-imidazo[1,2-a]pyridin-3-yl)-amin-Hydrochlorid, (2-Furan-2-yl-7-methyl-imidazo[1,2-a]pyrimidin-3-yl)-(1,1,3,3-tetramethyl-butyl)-amin-Hydrochlorid und *tert*-Butyl-[2-(4-nitro-phenyl)-imidazo[1,2-a]pyrazin-3-yl]-amin-Hydrochlorid.

**Tabelle 4**

| **Beispiel- Nr.** | **Name** | **Ansatz** | **Ausbeute** | **Produkt- fraktion** | **Citrullin- Assay** |
|---|---|---|---|---|---|
| | | mmol Isonitril | g Produkt-fraktion | | IC50 (µM) |
| 292 | Cyclohexyl-[7-methyl-2-(2-trifluoromethyl-phenyl)-imidazo[1,2-a]pyridin-3-yl]-amin; Hydrochlorid | 21,3 | 5,93 | 0 | 2,4 |
| 295 | Cyclohexyl-(5,7-dimethyl-2-pyridin-4-yl-imidazo[1,2-a]pyridin-3-yl)-amin; Hydrochlorid | 50,4 | 12,2 | 2 | 9,2 |
| 296 | (2-Furan-2-yl-5,7-dimethyl-imidazo[1,2-a]pyridin-3-yl)-(1,1,3,3-tetramethyl-butyl)-amin; Hydrochlorid | 47,9 | 13,9 | 4 | 2,5 |
| 298 | [2-(2-Fluorphenyl)-7-methyl-imidazo[1,2-a]pyridin-3-yl]-(1,1,3,3-tetramethyl-butyl)-amin; Hydrochlorid | 43,1 | 15,9 | 4 | 4,3 |
| 299 | Cyclohexyl-(7-methyl-2-phenyl-imidazo[1,2-a]pyridin-3-yl)-amin; Hydrochlorid | 5,00 | 1,64 | 2 | |
| 300 | (2-Furan-2-yl-7-methyl-imidazo[1,2-a]pyrimidin-3-yl)-(1,1,3,3-tetramethyl-butyl)-amin; Hydrochlorid | 43,1 | 10,2 | 2 + 3 | |

Als Vergleichsbeispiel wurde im Citrullin-Assay der aus dem Stand der Technik bekannte NOS-Inhibitor 7-Nitroindazol mit einem IC₅₀-Wert von 5,23 µM getestet.

Ferner wurden die Beispielsverbindungen 302 bis 312 gemäß AAV 3 hergestellt und im oben beschriebenen NOS-Assay auf %-Hemmung getestet. Die Ergebnisse sind in Tabelle 5 wiedergegeben.

**Tabelle 5**

| **Beispiel Nr.** | **Name** | **NOS- Assay** | **Ansatz** | **Ausbeute** | **Produkt- fraktion** |
|---|---|---|---|---|---|
| | | % Hemmung | mmol Isonitril | g Produkt-fraktion | |
| 302 | Cyclohexyl-(2-furan-3-yl-5,7-dimethyl-imidazo[1,2-a]pyridin-3-yl)-amin-Hydrochlorid | 54 | 9.2 | 1.9 | 2 |
| 303 | Cyclopentyl-(2-furan-3-yl-5,7-dimethyl-imidazo[1,2-a]pyridin-3-yl)-amin-Hydrochlorid | 57 | 10.5 | 2.1 | 2 |
| 304 | [2-(4-Brom-2-fluorphenyl)-5,7-dimethyl-imidazo[1,2-a]pyridin-3-yl]-cyclopentyl-amin-Hydrochlorid | 56 | 6.3 | 1.9 | 4 |
| 305 | Cyclopentyl-{5,7-dimethyl-2-[5-(2-nitro-phenyl)-furan-2-yl]-imidazo[1,2-a]pyridin-3-yl}-amin-Hydrochlorid | 55 | 6.3 | 3 | 2 |
| 306 | {2-[5-(4-Chlorphenyl)-furan-2-yl]-5,7-dimethyl-imidazo[1,2-a]pyridin-3-yl}-cyclopentyl-amin-Hydrochlorid | 73 | 6.7 | 1.7 | 2 |
| 307 | Cyclopentyl-(2-furan-3-yl-5,7-dimethyl-imidazo[1,2-a]pyridin-3-yl)-amin-Hydrochlorid | 57 | 10.5 | 2.6 | 4 |
| 308 | (2-Furan-3-yl-5,7-dimethyl-imidazo[1,2-a]pyridin-3-yl)-(1,1,3,3-tetramethyl-butyl)-amin-Hydrochlorid | 50 | 25.1 | 5.6 | 2 |
| 309 | Benzyl-(7-methyl-2-thiophen-3-yl-imidazo[1,2-a]pyridin-3-yl)-amin-Hydrochlorid | 45 | 21.3 | 4.1 | 2 |
| 310 | Cyclohexyl-(2-furan-3-yl-5,7-dimethyl-imidazo[1,2-a]pyridin-3-yl)-amin-Hydrochlorid | 52 | 22.9 | 5.2 | 2 |
| 311 | (2-Furan-3-yl-7-methyl-imidazo[1,2-a]pyridin-3-yl)-(1,1,3,3-tetramethyl-butyl)-amin-Hydrochlorid | 45 | 25.1 | 2.9 | 2 |
| 312 | (5,7-Dimethyl-2-thiophen-3-yl-imidazo[1,2-a]pyridin-3-yl)-(1,1,3,3-tetramethyl-butyl)-amin-Hydrochlorid | 74 | 18 | 4.6 | 4 |

### Allgemeine Arbeitsvorschrift 4 (AAV 4):

Das gemäß AAV 3 erhaltene Edukt (Produktfraktion) wurde im Reaktionsgefäß in Tetrahydrofuran (ca. 3 ml je mmol Edukt) vorgelegt, unter Rühren bei -15 bis -5 °C 1,10 Stoffmengenäquivalente n-Butyllithiumlösung in Hexan (1,6 mol/l) zugetropft und eine Stunde nachgerührt. Anschließend wurden 1,05 Stoffmengenäquivalente des Acetylchlorids zugetropft und über Nacht unter Erwärmung auf Raumtemperatur gerührt.

Zur Aufarbeitung wurde auf 0 bis 5 °C gekühlt und halbgesättigte Ammoniumchloridlösung (ca. 1,5 ml je mmol Edukt) zugegeben. Es wurde dreimal mit Ether (ca. 1,5 ml je mmol Edukt) extrahiert, die vereinigten Extrakte über Natriumsulfat getrocknet, filtiert und eingeengt.

Ein Teil des so erhaltenen Produkts wurde nach dünnschichtchromatographischer und/oder NMR-spektroskopischer Untersuchungen einer Hydrochloridfällung gem. AAV 3 zugeführt.

Bei den gemäß AAV 4 beispielhaft hergestellten Verbindungen handelt es sich um N-{2-[3-(4-Chlorphenoxy)-phenyl]-imidazo[1,2-a]pyridin-3-yl}-N-cyclohexyl-acetamid-Hydrochlorid, N-Cyclohexyl-N-(7-methyl-2-o-tolyl-imidazo[1,2-a]pyridin-3-yl)-acetamid-Hydrochlorid und N-(2,6-Dimethyl-phenyl)-N-[2-(2,4-dimethyl-phenyl)-5-methyl-imidazo[1,2-a]pyridin-3-yl]-acetamid-Hydrochlorid.

### Allgemeine Arbeitsvorschrift 5 (AAV 5):

Das gemäß AAV 4 erhaltene Edukt wurde im Reaktionsgefäß vorgelegt, es wurden unter Rühren zehn Stoffmengenäquivalente des jeweiligen Säurehalogenids zugegeben und eine Stunde bei 40 °C gerührt.

Das Reaktionsgemisch wurde in wenig Dichlormethan aufgenommen, das Produkt durch Zugabe von Ether und ggf. Hexan ausgefällt und anschließend umkristallisiert.

Mit dieser Vorgenhensweise wurde aufgrund des Wassergehalts der verwendeten Lösungsmittel in der Regel das gewünschte Produkt als Hydrohalogenid erhalten oder alternativ einer Hydrochloridfällung gem. AAV 3 zugeführt.

Beispielhaft wurde gemäß AAV 5 1-Acetyl-3-(acetyl-cyclohexyl-amino)-7-methyl-2-o-tolyl-imidazo[1,2-a]pyridin-1-ium Chlorid-Hydrochlorid hergestellt.

### Pharmazeutische Formulierung für die erfindungsgemäße Verwendung

1 g des Hydrochlorids von (5,7-Dimethyl-2-p-tolyl-imidazo[1,2-a]pyridin-3-yl)-(1,1,3,3-tetramethyl-butyl)-amin wurde in 1 I Wasser für Injektionszwecke bei Raumtemperatur gelöst und anschließend durch Zugabe von Natriumchlorid auf isotone Bedingungen eingestellt.

In gleicher Weise wurde eine isotonische Lösung von 1 g (5,7-Dimethyl-2-thiophen-3-yl-imidazo[1,2-a]pyridin-3-yl)-(1,1,3,3-tetramethyl-butyl)-amin-Hydrochlorid in 1 I Wasser hergestellt.

## Patentansprüche

1. Verwendung einer Verbindung der allgemeinen Struktur I oder eines ihrer pharmazeutisch annehmbaren Salze 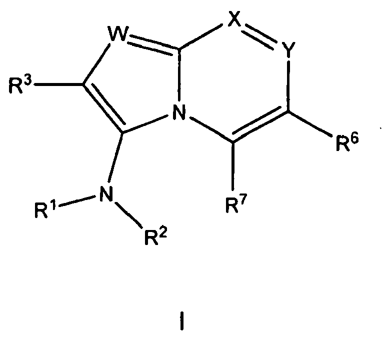 worin
X CR⁴ oder N bedeutet,
Y CR⁵ oder N bedeutet und
X und Y nicht zugleich N bedeuten,
W N oder NR⁸ bedeutet,
R¹ C₁₋₁₂-Alkyl, wobei Alkyl geradkettig oder verzweigt ist und gesättigt oder ungesättigt ist und unsubstituiert oder ein- oder mehrfach substituiert ist, C₃₋₈-Cycloalkyl oder CH₂-C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,
R² Wasserstoff oder C(=O)R⁹ bedeutet,
R³ C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert ist, Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,
R⁴, R⁵, R⁶ und R⁷ unabhängig voneinander Wasserstoff oder C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl oder CH₂-C₃₋₈-Cycloalkyl, wobei Cycloalkyl gesättigt oder ungesättigt ist und unsubstituiert oder ein- oder mehrfach substituiert ist, F, Cl, Br, I, CN, NO₂, NH₂, C(=O)R⁹, CO₂H, CO₂R¹⁰, OH oder OR¹¹ bedeuten,
oder
R⁴ und R⁵ oder R⁵ und R⁶ oder R⁶ und R⁷ für eine viergliedrige gesättigte oder ungesättigte Kohlenwasserstoffbrücke mit keinem, 1, 2 oder 3 Heteroatomen, die aus der Gruppe, die N, O und S enthält, ausgewählt sind, stehen und die anderen Reste von R⁴, R⁵, R⁶ und R⁷ Wasserstoff bedeuten,
R⁸ C(=O)R⁹ bedeutet,
R⁹ C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl oder CH₂-C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-Aryl oder C₁₋₈-Alkyl-Heteroaryl bedeutet, wobei Alkyl geradkettig oder verzweigt ist und gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet, und
R¹⁰ und R¹¹ unabhängig voneinander C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl oder CH₂-C₃₋₈-Cycloalkyl, wobei Cycloalkyl gesättigt oder ungesättigt ist und unsubstituiert oder ein- oder mehrfach substituiert ist, Aryl, wobei Aryl unsubstituiert oder ein- oder mehrfach substituiert ist, C₁₋₈-Alkyl-Aryl, wobei Alkyl geradkettig oder verzweigt ist und gesättigt oder ungesättigt ist und unsubstituiert oder ein- oder mehrfach substituiert ist und Aryl unsubstituiert oder ein- oder mehrfach substituiert ist, bedeuten,
mit Ausnahme von Verbindungen der allgemeinen Struktur I, in denen zugleich
R¹ = tert.-Butyl,
R² = H,
R³ = C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert ist, Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,
X=CR⁴ mit R⁴ = H,
Y = CR⁵ mit R⁵ = CH₃,
R⁶ = H und
R⁷ = H oder C₁₋₄-Alkanyl, wobei Alkanyl geradkettig oder verzweigt und unsubstituiert oder ein- oder mehrfach substituiert ist, bedeuten,
wobei man im Zusammenhang mit "Alkyl" und "Alkanyl" der ausgenommenen Verbindungen unter dem Begriff "substituiert" die Substitution eines Wasserstoffrestes durch F, Cl, Br, I, -CN, NH₂, NH-Alkyl, NH-Aryl, NH-Heteroaryl, NH-Alkyl-Aryl, NH-Alkyl-Heteroaryl, NH-Heterocyclyl, NH-Alkyl-OH, N(Alkyl)₂, N(Alkyl-Aryl)₂, N(Alkyl-Heteroaryl)₂, N(Heterocyclyl)₂, N(Alkyl-OH)₂, NO, NO₂, SH, S-Alkyl, S-Aryl, S-Heteroaryl, S-Alkyl-Aryl, S-Alkyl-Heteroaryl, S-Heterocyclyl, S-Alkyl-OH, S-Alkyl-SH, OH, O-Alkyl, O-Aryl, O-Heteroaryl, O-Alkyl-Aryl, O-Alkyl-Heteroaryl, O-Heterocyclyl, O-Alkyl-OH, CHO, C(=O)C₁₋₆-Alkyl, C(=S)C₁₋₆-Alkyl, C(=O)Aryl, C(=S)Aryl, C(=O)C₁₋₆-Alkyl-Aryl, 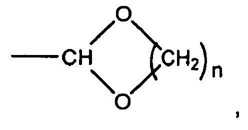 mit n = 1, 2 oder 3, C(=S)C₁₋₆-Alkyl-Aryl, C(=O)-Heteroaryl, C(=S)-Heteroaryl, C(=O)-Heterocyclyl, C(=S)-Heterocyclyl, CO₂H, CO₂-Alkyl, CO₂-Alkyl-Aryl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Alkyl-Heteroaryl)₂, C(=O)N(Heterocyclyl)₂, SO-Alkyl, SO₂-Alkyl, SO₂NH₂, SO₃H, Cycloalkyl, Aryl, Heteroaryl oder Heterocyclyl versteht, wobei unter mehrfach substituierten Resten solche Reste zu verstehen sind, die entweder an verschiedenen oder an gleichen Atomen mehrfach substituiert sind,
zur Herstellung eines Medikaments zur Behandlung von Entzündungsschmerz.

2. Verwendung einer Verbindung der allgemeinen Struktur I oder eines ihrer pharmazeutisch annehmbaren Salze 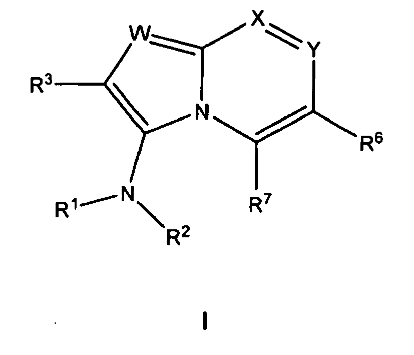 worin
X CR⁴ oder N bedeutet,
Y CR⁵ oder N bedeutet und
X und Y nicht zugleich N bedeuten,
W N oder NR⁸ bedeutet,
R¹ C₁₋₁₂-Alkyl, wobei Alkyl geradkettig oder verzweigt ist und gesättigt oder ungesättigt ist und unsubstituiert oder ein- oder mehrfach substituiert ist, C₃₋₈-Cycloalkyl oder CH₂-C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,
R² Wasserstoff oder C(=O)R⁹ bedeutet,
R³ C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl; wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert ist, Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,
R⁴, R⁵, R⁶ und R⁷ unabhängig voneinander Wasserstoff oder C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl oder CH₂-C₃₋₈-Cycloalkyl, wobei Cycloalkyl gesättigt oder ungesättigt ist und unsubstituiert oder ein- oder mehrfach substituiert ist, F, Cl, Br, l, CN, NO₂, NH₂, C(=O)R⁹, CO₂H, CO₂R¹⁰, OH oder OR¹¹ bedeuten,
oder
R⁴ und R⁵ oder R⁵ und R⁶ oder R⁶ und R⁷ für eine viergliedrige gesättigte oder ungesättigte Kohlenwasserstoffbrücke mit keinem, 1, 2 oder 3 Heteroatomen, die aus der Gruppe, die N, O und S enthält, ausgewählt sind, stehen und die anderen Reste von R⁴, R⁵, R⁶ und R⁷ Wasserstoff bedeuten,
R⁸ C(=O)R⁹ bedeutet,
R⁹ C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl oder CH₂-C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-Aryl oder C₁₋₈-Alkyl-Heteroaryl bedeutet, wobei Alkyl geradkettig oder verzweigt ist und gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet, und
R¹⁰ und R¹¹ unabhängig voneinander C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl oder CH₂-C₃₋₈-Cycloalkyl, wobei Cycloalkyl gesättigt oder ungesättigt ist und unsubstituiert oder ein- oder mehrfach substituiert ist, Aryl, wobei Aryl unsubstituiert oder ein- oder mehrfach substituiert ist, C₁₋₈-Alkyl-Aryl, wobei Alkyl geradkettig oder verzweigt ist und gesättigt oder ungesättigt ist und unsubstituiert oder ein- oder mehrfach substituiert ist und Aryl unsubstituiert oder ein- oder mehrfach substituiert ist, bedeuten,
mit Ausnahme von Verbindungen der allgemeinen Struktur I, in denen zugleich
R¹ = tert.-Butyl,
R² = H,
R³ = C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert ist, Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,
X = CR⁴ mit R⁴ = H,
Y = CR⁵ mit R⁵ = CH₃,
R⁶ = H und
R⁷ = H oder C₁₋₄-Alkanyl, wobei Alkanyl geradkettig oder verzweigt und unsubstituiert oder ein- oder mehrfach substituiert ist, bedeuten,
wobei man im Zusammenhang mit "Alkyl" und "Alkanyl" der ausgenommenen Verbindungen unter dem Begriff "substituiert" die Substitution eines Wasserstoffrestes durch F, Cl, Br, I, -CN, NH₂, NH-Alkyl, NH-Aryl, NH-Heteroaryl, NH-Alkyl-Aryl, NH-Alkyl-Heteroaryl, NH-Heterocyclyl, NH-Alkyl-OH, N(Alkyl)₂, N(Alkyl-Aryl)₂, N(Alkyl-Heteroaryl)₂, N(Heterocyclyl)₂, N(Alkyl-OH)₂, NO, NO₂, SH, S-Alkyl, S-Aryl, S-Heteroaryl, S-Alkyl-Aryl, S-Alkyl-Heteroaryl, S-Heterocyclyl, S-Alkyl-OH, S-Alkyl-SH, OH, O-Alkyl, O-Aryl, O-Heteroaryl, O-Alkyl-Aryl, O-Alkyl-Heteroaryl, O-Heterocyclyl, O-Alkyl-OH, CHO, C(=O)C₁₋₆-Alkyl, C(=S)C₁₋₆-Alkyl, C(=O)Aryl, C(=S)Aryl, C(=O)C₁₋₆-Alkyl-Aryl, 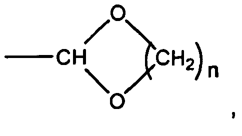 mit n = 1, 2 oder 3, C(=S)C₁₋₆-Alkyl-Aryl, C(=O)-Heteroaryl, C(=S)-Heteroaryl, C(=O)-Heterocyclyl, C(=S)-Heterocyclyl, CO₂H, CO₂-Alkyl, CO₂-Alkyl-Aryl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Alkyl-Heteroaryl)₂, C(=O)N(Heterocyclyl)₂, SO-Alkyl, SO₂-Alkyl, SO₂NH₂, SO₃H, Cycloalkyl, Aryl, Heteroaryl oder Heterocyclyl versteht, wobei unter mehrfach substituierten Resten solche Reste zu verstehen sind, die entweder an verschiedenen oder an gleichen Atomen mehrfach substituiert sind,
zur Herstellung eines Medikaments zur Behandlung von Migräne.

3. Verwendung einer Verbindung der allgemeinen Struktur I oder eines ihrer pharmazeutisch annehmbaren Salze 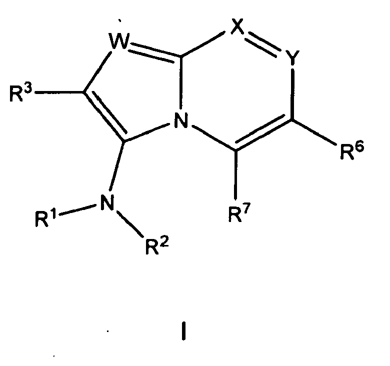 worin
X CR⁴ oder N bedeutet,
Y CR⁵ oder N bedeutet und
X und Y nicht zugleich N bedeuten,
W N oder NR⁸ bedeutet,
R¹ C₁₋₁₂-Alkyl, wobei Alkyl geradkettig oder verzweigt ist und gesättigt oder ungesättigt ist und unsubstituiert oder ein- oder mehrfach substituiert ist, C₃₋₈-Cycloalkyl oder CH₂-C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,
R² Wasserstoff oder C(=O)R⁹ bedeutet,
R³ C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert ist, Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,
R⁴, R⁵, R⁶ und R⁷ unabhängig voneinander Wasserstoff oder C₁₋₈-Alkyl, wobei Alkyl geradkettig oder verzweigt ist und gesättigt ist und unsubstituiert oder ein- oder mehrfach substituiert ist, C₃₋₈-Cycloalkyl oder CH₂-C₃₋₈-Cycloalkyl, wobei Cycloalkyl gesättigt oder ungesättigt ist und unsubstituiert oder ein- oder mehrfach substituiert ist, F, Cl, Br, I, CN, NO₂, NH₂, C(=O)R⁹, CO₂H, CO₂R¹⁰, OH bedeuten, oder
R⁴ und R⁵ oder R⁵ und R⁶ oder R⁶ und R⁷ für eine viergliedrige gesättigte oder ungesättigte Kohlenwasserstoffbrücke mit keinem, 1, 2 oder 3 Heteroatomen, die aus der Gruppe, die N, O und S enthält, ausgewählt sind, stehen und die anderen Reste von R⁴, R⁵, R⁶ und R⁷ Wasserstoff bedeuten,
R⁸ C(=O)R⁹ bedeutet,
R⁹ C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl oder CH₂-C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-Aryl oder C₁₋₈-Alkyl-Heteroaryl bedeutet, wobei Alkyl geradkettig oder verzweigt ist und gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet, und
R¹⁰ C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl oder CH₂-C₃₋₈-Cycloalkyl, wobei Cycloalkyl gesättigt oder ungesättigt ist und unsubstituiert oder ein- oder mehrfach substituiert ist, Aryl, wobei Aryl unsubstituiert oder ein- oder mehrfach substituiert ist, C₁₋₈-Alkyl-Aryl, wobei Alkyl geradkettig oder verzweigt ist und gesättigt oder ungesättigt ist und unsubstituiert oder ein- oder mehrfach substituiert ist und Aryl unsubstituiert oder ein- oder mehrfach substituiert ist, bedeutet,
mit Ausnahme von Verbindungen der allgemeinen Struktur I, in denen zugleich
R¹ = tert.-Butyl,
R² = H,
R³ = C₁₋₈-Alkyl, wobei Alkyl geradkettig oder verzweigt ist und gesättigt oder ungesättigt ist und unsubstituiert oder ein- oder mehrfach substituiert ist, C₃₋₈-Cycloalkyl, wobei Cycloalkyl gesättigt oder ungesättigt ist und unsubstituiert oder ein- oder mehrfach substituiert ist, Heterocyclyl, wobei Heterocyclyl gesättigt oder ungesättigt ist und unsubstituiert oder ein- oder mehrfach substituiert ist, Aryl, wobei Aryl unsubstituiert oder ein- oder mehrfach substituiert ist, Heteroaryl, wobei Heteroaryl unsubstituiert oder ein- oder mehrfach substituiert ist, C₁₋₈-Alkyl-C₃₋₈-Cycloalkyl, C₁₋₈-Alkyl-Heterocyclyl, C₁₋₈-Alkyl-Aryl oder C₁₋₈-Alkyl-Heteroaryl, wobei Alkyl geradkettig oder verzweigt ist und gesättigt oder ungesättigt ist und unsubstituiert oder ein- oder mehrfach substituiert ist, Cycloalkyl gesättigt oder ungesättigt ist und unsubstituiert oder ein- oder mehrfach substituiert ist, Heterocyclyl gesättigt oder ungesättigt ist und unsubstituiert oder ein- oder mehrfach substituiert ist, Aryl unsubstituiert oder ein- oder mehrfach substituiert ist und Heteroaryl unsubstituiert oder ein- oder mehrfach substituiert ist, bedeutet,
X = CR⁴ mit R⁴ = H,
Y = CR⁵ mit R⁵ = CH₃,
R⁶ = H und
R⁷ = H oder C₁₋₄-Alkanyl, wobei Alkanyl geradkettig oder verzweigt und unsubstituiert oder ein- oder mehrfach substituiert ist, bedeuten,
wobei man im Zusammenhang mit "Alkyl" und "Alkanyl" der ausgenommenen Verbindungen unter dem Begriff "substituiert" die Substitution eines Wasserstoffrestes durch F, Cl, Br, I, -CN, NH₂, NH-Alkyl, NH-Aryl, NH-Heteroaryl, NH-Alkyl-Aryl, NH-Alkyl-Heteroaryl, NH-Heterocyclyl, NH-Alkyl-OH, N(Alkyl)₂, N(Alkyl-Aryl)₂, N(Alkyl-Heteroaryl)₂, N(Heterocyclyl)₂, N(Alkyl-OH)₂, NO, NO₂, SH, S-Alkyl, S-Aryl, S-Heteroaryl, S-Alkyl-Aryl, S-Alkyl-Heteroaryl, S-Heterocyclyl, S-Alkyl-OH, S-Alkyl-SH, OH, O-Alkyl, O-Aryl, O-Heteroaryl, O-Alkyl-Aryl, O-Alkyl-Heteroaryl, O-Heterocyclyl, O-Alkyl-OH, CHO, C(=O)C₁₋₆-Alkyl, C(=S)C₁₋₆-Alkyl, C(=O)Aryl, C(=S)Aryl, C(=O)C₁₋₆-Alkyl-Aryl, 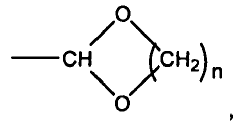 mit n = 1, 2 oder 3, C(=S)C₁₋₆-Alkyl-Aryl, C(=O)-Heteroaryl, C(=S)-Heteroaryl, C(=O)-Heterocyclyl, C(=S)-Heterocyclyl, CO₂H, CO₂-Alkyl, CO₂-Alkyl-Aryl, C(=O)NH₂, C(=O)NH-Alkyl, C(=O)NHAryl, C(=O)NH-Heterocyclyl, C(=O)N(Alkyl)₂, C(=O)N(Alkyl-Aryl)₂, C(=O)N(Alkyl-Heteroaryl)₂, C(=O)N(Heterocyclyl)₂, SO-Alkyl, SO₂-Alkyl, SO₂NH₂, SO₃H, Cycloalkyl, Aryl, Heteroaryl oder Heterocyclyl versteht, wobei unter mehrfach substituierten Resten solche Reste zu verstehen sind, die entweder an verschiedenen oder an gleichen Atomen mehrfach substituiert sind,
zur Herstellung eines Medikaments zur Behandlung von Multipler Sklerose, Morbus Parkinson, Morbus Alzheimer, Morbus Huntington, cerebraler Ischämie, Diabetes, Meningitis, Arteriosklerose und/oder für die Wundheilung.

## Claims

1. Use of a compound having the general structure I or of a pharmaceutically acceptable salt thereof 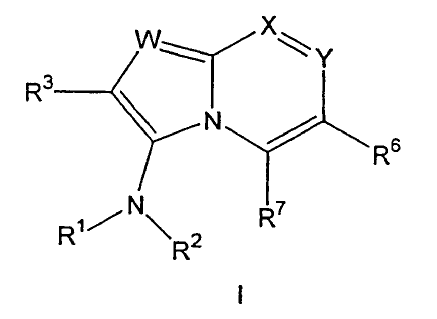 wherein
X represents CR⁴ or N,
Y represents CR⁵ or N and
X and Y do not simultaneously represent N,
W represents N or NR⁸,
R¹ represents C₁₋₁₂-alkyl, wherein alkyl is straight-chain or branched and is saturated or unsaturated and is unsubstituted or mono- or poly-substituted, C₃₋₈-cycloalkyl or CH₂-C₃₋₈-cycloalkyl, wherein cycloalkyl is saturated or unsaturated and is unsubstituted or mono- or poly-substituted, heterocyclyl, wherein heterocyclyl is saturated or unsaturated and is unsubstituted or mono- or poly-substituted, aryl, wherein aryl is unsubstituted or mono- or poly-substituted, heteroaryl, wherein heteroaryl is unsubstituted or mono- or poly-substituted, C₁₋₈alkyl-aryl or C₁₋₈-alkyl-heteroaryl, wherein alkyl is straight-chain or branched and is saturated or unsaturated and is unsubstituted or mono- or poly-substituted, aryl is unsubstituted or mono- or poly-substituted and heteroaryl is unsubstituted or mono- or poly-substituted,
R² represents hydrogen or C(=O)R⁹,
R³ represents C₁₋₈-alkyl, wherein alkyl is straight-chain or branched and is saturated or unsaturated and is unsubstituted or mono- or poly-substituted, C₃₋₈-cycloalkyl, wherein cycloalkyl is saturated or unsaturated and is unsubstituted or mono- or poly-substituted, heterocyclyl, wherein heterocyclyl is saturated or unsaturated and is unsubstituted or mono- or poly-substituted, aryl, wherein aryl is unsubstituted or mono- or poly-substituted, heteroaryl, wherein heteroaryl is unsubstituted or mono- or poly-substituted, C₁₋₈-alkyl-C₃₋₈-cycloalkyl, C₁₋₈-alkyl-heterocyclyl, C₁₋₈-alkyl-aryl or C₁₋₈-alkyl-heteroaryl, wherein alkyl is straight-chain or branched and is saturated or unsaturated and is unsubstituted or mono- or poly-substituted, cycloalkyl is saturated or unsaturated and is unsubstituted or mono- or poly-substituted, heterocyclyl is saturated or unsaturated and is unsubstituted or mono- or poly-substituted, aryl is unsubstituted or mono- or poly-substituted and heteroaryl is unsubstituted or mono- or poly-substituted,
R⁴, R⁵, R⁶ and R⁷ each independently of the others represents hydrogen or C₁₋₈-alkyl, wherein alkyl is straight-chain or branched and is saturated or unsaturated and is unsubstituted or mono- or poly-substituted, C₃₋₈-cycloalkyl or CH₂-C₃₋₈-cycloalkyl, wherein cycloalkyl is saturated or unsaturated and is unsubstituted or mono- or poly-substituted, F, Cl, Br, I, CN, NO₂, NH₂, C (=O) R⁹, CO₂H, CO₂R¹⁰, OH or OR¹¹, or
R⁴ and R⁵ or R⁵ and R⁶ or R⁶ and R⁷ represent a four-membered saturated or unsaturated hydrocarbon bridge having zero, 1, 2 or 3 hetero atoms selected from the group containing N, O and S, and the other radicals of R⁴, R⁵, R⁶ and R⁷ represent hydrogen,
R⁸ represents C (=O) R⁹,
R⁹ represents C₁₋₈-alkyl, wherein alkyl is straight-chain or branched and is saturated or unsaturated and is unsubstituted or mono- or poly-substituted, C₃₋₈-cycloalkyl or CH₂-C₃₋₈-cycloalkyl, wherein cycloalkyl is saturated or unsaturated and is unsubstituted or mono- or poly-substituted, heterocyclyl, wherein heterocyclyl is saturated or unsaturated and is unsubstituted or mono- or poly-substituted, aryl, wherein aryl is unsubstituted or mono- or poly-substituted, heteroaryl, wherein heteroaryl is unsubstituted or mono- or poly-substituted, C₁₋₈-alkyl-aryl or C₁₋₈-alkyl-heteroaryl, wherein alkyl is straight-chain or branched and is saturated or unsaturated and is unsubstituted or mono- or poly-substituted, aryl is unsubstituted or mono- or poly-substituted and heteroaryl is unsubstituted or mono- or poly-substituted, and
R¹⁰ and R¹¹ each independently of the other represents C₁₋₈-alkyl, wherein alkyl is straight-chain or branched and is saturated or unsaturated and is unsubstituted or mono- or poly-substituted, C₃₋₈-cycloalkyl or CH₂-C₃₋₈-cycloalkyl, wherein cycloalkyl is saturated or unsaturated and is unsubstituted or mono- or poly-substituted, aryl, wherein aryl is unsubstituted or mono- or poly-substituted, C₁₋₈-alkyl-aryl, wherein alkyl is straight-chain or branched and is saturated or unsaturated and is unsubstituted or mono- or poly-substituted and aryl is unsubstituted or mono- or poly-substituted,
with the exception of compounds having the general structure I in which simultaneously
R¹ = *tert*-butyl
R² = H
R³ = C₁₋₈-alkyl, wherein alkyl is straight-chain or branched and is saturated or unsaturated and is unsubstituted or mono- or poly-substituted, C₃₋₈-cycloalkyl, wherein cycloalkyl is saturated or unsaturated and is unsubstituted or mono- or poly-substituted, heterocyclyl, wherein heterocyclyl is saturated or unsaturated and is unsubstituted or mono- or poly-substituted, aryl, wherein aryl is unsubstituted or mono- or poly-substituted, heteroaryl, wherein heteroaryl is unsubstituted or mono- or poly-substituted, C₁₋₈-alkyl-C₃₋₈-cycloalkyl, C₁₋₈-alkyl-heterocyclyl, C₁₋₈-alkyl-aryl or C₁₋₈-alkyl-heteroaryl, wherein alkyl is straight-chain or branched and is saturated or unsaturated and is unsubstituted or mono- or poly-substituted, cycloalkyl is saturated or unsaturated and is unsubstituted or mono- or poly-substituted, heterocyclyl is saturated or unsaturated and is unsubstituted or mono- or poly-substituted, aryl is unsubstituted or mono- or poly-substituted and heteroaryl is unsubstituted or mono- or poly-substituted,
X = CR⁴ wherein R⁴ = H,
Y = CR⁵ wherein R⁵ = CH₃,
R⁶ = H and
R⁷ = H or C₁₋₄-alkanyl, wherein alkanyl is straight-chain or branched and is unsubstituted or mono- or poly-substituted,
wherein in connection with "alkyl" and "alkanyl" in the excluded compounds the term "substituted" is understood to mean the substitution of a hydrogen radical by F, Cl, Br, I, -CN, NH₂, NH-alkyl, NH-aryl, NH-heteroaryl, NH-alkyl-aryl, NH-alkyl-heteroaryl, NH-heterocyclyl, NH-alkyl-OH, N(alkyl)₂, N(alkyl-aryl)₂, N(alkyl-heteroaryl)₂, N(heterocyclyl)₂, N(alkyl-OH)₂, NO, NO₂, SH, S-alkyl, S-aryl, S-heteroaryl, S-alkyl-aryl, S-alkyl-heteroaryl, S-heterocyclyl, S-alkyl-OH, S-alkyl-SH, OH, O-alkyl, O-aryl, O-heteroaryl, O-alkyl-aryl, O-alkyl-heteroaryl, O-heterocyclyl, O-alkyl-OH, CHO, C (=O) C₁₋₆-alkyl, C (=S) C₁₋₆-alkyl, C (=O) aryl, C(=S)aryl, C (=O) C₁₋₆-alkyl-aryl, 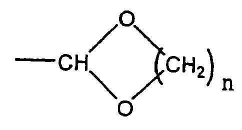 wherein n = 1, 2 or 3, C (=S) C₁₋₆-alkyl-aryl, C (=O) -heteroaryl, C(=S)-heteroaryl, C(=O)-heterocyclyl, C(=S)-heterocyclyl, CO₂H, CO₂-alkyl, CO₂-alkyl-aryl, C(=O)NH₂, C(=O)NH-alkyl, C(=O)NH-aryl, C(=O)NH-heterocyclyl, C(=O)N(alkyl)₂, C(=O)N(alkyl-aryl)₂, C(=O)N(alkyl-heteroaryl)₂, C(=O)N(heterocyclyl)₂, SO-alkyl, SO₂-alkyl, SO₂NH₂, SO₃H, cycloalkyl, aryl, heteroaryl or by heterocyclyl, where polysubstituted radicals are to be understood to be radicals that are polysubstituted either on different atoms or on the same atom,
in the preparation of a medicament for the treatment of inflammatory pain.

2. Use of a compound having the general structure I or of a pharmaceutically acceptable salt thereof 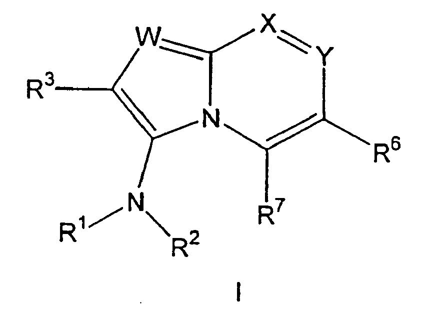 wherein
X represents CR⁴ or N,
Y represents CR⁵ or N and
X and Y do not simultaneously represent N,
W represents N or NR⁸,
R¹ represents C₁₋₁₂-alkyl, wherein alkyl is straight-chain or branched and is saturated or unsaturated and is unsubstituted or mono- or poly-substituted, C₃₋₈-cycloalkyl or CH₂-C₃₋₈-cycloalkyl, wherein cycloalkyl is saturated or unsaturated and is unsubstituted or mono- or poly-substituted, heterocyclyl, wherein heterocyclyl is saturated or unsaturated and is unsubstituted or mono- or poly-substituted, aryl, wherein aryl is unsubstituted or mono- or poly-substituted, heteroaryl, wherein heteroaryl is unsubstituted or mono- or poly-substituted, C₁₋₈-alkyl-aryl or C₁₋₈-alkyl-heteroaryl, wherein alkyl is straight-chain or branched and is saturated or unsaturated and is unsubstituted or mono- or poly-substituted, aryl is unsubstituted or mono- or poly-substituted and heteroaryl is unsubstituted or mono- or poly-substituted,
R² represents hydrogen or C(=O)R⁹,
R³ represents C₁₋₈-alkyl, wherein alkyl is straight-chain or branched and is saturated or unsaturated and is unsubstituted or mono- or poly-substituted, C₃₋₈-cycloalkyl, wherein cycloalkyl is saturated or unsaturated and is unsubstituted or mono- or poly-substituted, heterocyclyl, wherein heterocyclyl is saturated or unsaturated and is unsubstituted or mono- or poly-substituted, aryl, wherein aryl is unsubstituted or mono- or poly-substituted, heteroaryl, wherein heteroaryl is unsubstituted or mono- or poly-substituted, C₁₋₈-alkyl-C₃₋₈-cycloalkyl, C₁-₈-alkyl-heterocyclyl, C₁₋₈-alkyl-aryl or C₁₋₈-alkyl-heteroaryl, wherein alkyl is straight-chain or branched and is saturated or unsaturated and is unsubstituted or mono- or poly-substituted, cycloalkyl is saturated or unsaturated and is unsubstituted or mono- or poly-substituted, heterocyclyl is saturated or unsaturated and is unsubstituted or mono- or poly-substituted, aryl is unsubstituted or mono- or poly-substituted and heteroaryl is unsubstituted or mono- or poly-substituted,
R⁴ R⁵, R⁶ and R⁷ each independently of the others represents hydrogen or C₁₋₈-alkyl, wherein alkyl is straight-chain or branched and is saturated or unsaturated and is unsubstituted or mono- or poly-substituted, C₃₋₈-cycloalkyl or CH₂-C₃₋₈-cycloalkyl, wherein cycloalkyl is saturated or unsaturated and is unsubstituted or mono- or poly-substituted, F, Cl, Br, I, CN, NO₂, NH₂, C(=O)R⁹, CO₂H, CO₂R¹⁰, OH or OR¹¹, or
R⁴ and R⁵ or R⁵ and R⁶ or R⁶ and R⁷ represent a four-membered saturated or unsaturated hydrocarbon bridge having zero, 1, 2 or 3 hetero atoms selected from the group containing N, O and S, and the other radicals of R⁴, R⁵, R⁶ and R⁷ represent hydrogen,
R⁸ represents C(=O)R⁹,
R⁹ represents C₁₋₈-alkyl, wherein alkyl is straight-chain or branched and is saturated or unsaturated and is unsubstituted or mono- or poly-substituted, C₃₋₈-cycloalkyl or CH₂-C₃₋₈-cycloalkyl, wherein cycloalkyl is saturated or unsaturated and is unsubstituted or mono- or poly-substituted, heterocyclyl, wherein heterocyclyl is saturated or unsaturated and is unsubstituted or mono- or poly-substituted, aryl, wherein aryl is unsubstituted or mono- or poly-substituted, heteroaryl, wherein heteroaryl is unsubstituted or mono- or poly-substituted, C₁₋₈-alkyl-aryl or C₁₋₈-alkyl-heteroaryl, wherein alkyl is straight-chain or branched and is saturated or unsaturated and is unsubstituted or mono- or poly-substituted, aryl is unsubstituted or mono- or poly-substituted and heteroaryl is unsubstituted or mono- or poly-substituted, and
R¹⁰ and R¹¹ each independently of the other represents C₁₋₈-alkyl, wherein alkyl is straight-chain or branched and is saturated or unsaturated and is unsubstituted or mono- or poly-substituted, C₃-₈-cycloalkyl or CH₂-C₃₋₈-cycloalkyl, wherein cycloalkyl is saturated or unsaturated and is unsubstituted or mono- or poly-substituted, aryl, wherein aryl is unsubstituted or mono- or poly-substituted, C₁₋₈-alkyl-aryl, wherein alkyl is straight-chain or branched and is saturated or unsaturated and is unsubstituted or mono- or poly-substituted and aryl is unsubstituted or mono- or poly-substituted,
with the exception of compounds having the general structure I in which simultaneously
R¹ = *tert*-butyl
R² = H
R³ = C₁₋₈-alkyl, wherein alkyl is straight-chain or branched and is saturated or unsaturated and is unsubstituted or mono- or poly-substituted, C₃₋₈-cycloalkyl, wherein cycloalkyl is saturated or unsaturated and is unsubstituted or mono- or poly-substituted, heterocyclyl, wherein heterocyclyl is saturated or unsaturated and is unsubstituted or mono- or poly-substituted, aryl, wherein aryl is unsubstituted or mono- or poly-substituted, heteroaryl, wherein heteroaryl is unsubstituted or mono- or poly-substituted, C₁₋₈-alkyl-C₃₋₈-cycloalkyl, C₁₋₈-alkyl-heterocyclyl, C₁₋₈-alkyl-aryl or C₁₋₈-alkyl-heteroaryl, wherein alkyl is straight-chain or branched and is saturated or unsaturated and is unsubstituted or mono- or poly-substituted, cycloalkyl is saturated or unsaturated and is unsubstituted or mono- or poly-substituted, heterocyclyl is saturated or unsaturated and is unsubstituted or mono- or poly-substituted, aryl is unsubstituted or mono- or poly-substituted and heteroaryl is unsubstituted or mono- or poly-substituted,
X = CR⁴ wherein R⁴ = H,
Y = CR⁵ wherein R⁵ = CH₃,
R⁶ = H and
R⁷ = H or C₁₋₄-alkanyl, wherein alkanyl is straight-chain or branched and is unsubstituted or mono- or poly-substituted,
wherein in connection with "alkyl" and "alkanyl" in the excluded compounds the term "substituted" is understood to mean the substitution of a hydrogen radical by F, Cl, Br, I, -CN, NH₂, NH-alkyl, NH-aryl, NH-heteroaryl, NH-alkyl-aryl, NH-alkyl-heteroaryl, NH-heterocyclyl, NH-alkyl-OH, N(alkyl)₂, N(alkyl-aryl)₂, N(alkyl-heteroaryl)₂, N(heterocyclyl)₂, N(alkyl-OH)₂, NO, NO₂, SH, S-alkyl, S-aryl, S-heteroaryl, S-alkyl-aryl, S-alkyl-heteroaryl, S-heterocyclyl, S-alkyl-OH, S-alkyl-SH, OH, O-alkyl, O-aryl, O-heteroaryl, O-alkyl-aryl, 0-alkyl-heteroaryl, O-heterocyclyl, O-alkyl-OH, CHO, C(=O)C₁₋₆-alkyl, C(=S)C₁₋₆-alkyl, C(=O)aryl, C(=S)aryl, C(=O)C₁₋₆-alkyl-aryl, 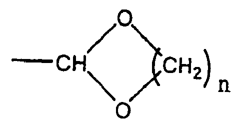 wherein n = 1, 2 or 3, C (=S) C₁₋₆-alkyl-aryl, C (=O) -heteroaryl, C(=S)-heteroaryl, C(=O)-heterocyclyl, C(=S)-heterocyclyl, CO₂H, CO₂-alkyl, CO₂-alkyl-aryl, C(=O)N_{H2}, C (=O) NH-alkyl, C(=O)NH-aryl, C(=O)NH-heterocyclyl, C(=O)N(alkyl)₂, C(=O)N(alkyl-aryl)₂, C(=O)N(alkyl-heteroaryl)₂, C(=O)N(heterocyclyl)₂, SO-alkyl, SO₂-alkyl, SO₂NH₂, SO₃H, cycloalkyl, aryl, heteroaryl or by heterocyclyl, where polysubstituted radicals are to be understood to be radicals that are polysubstituted either on different atoms or on the same atom,
in the preparation of a medicament for the treatment of migraine.

3. Use of a compound having the general structure I or of a pharmaceutically acceptable salt thereof 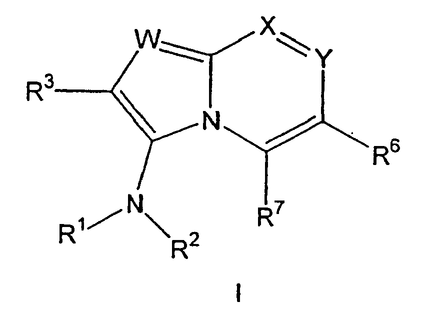 wherein
X represents CR⁴ or N,
Y represents CR⁵ or N and
X and Y do not simultaneously represent N,
W represents N or NR⁸,
R¹ represents C₁₋₁₂-alkyl, wherein alkyl is straight-chain or branched and is saturated or unsaturated and is unsubstituted or mono- or poly-substituted, C₃₋₈-cycloalkyl or CH₂-C₃₋₈-cycloalkyl, wherein cycloalkyl is saturated or unsaturated and is unsubstituted or mono- or poly-substituted, heterocyclyl, wherein heterocyclyl is saturated or unsaturated and is unsubstituted or mono- or poly-substituted, aryl, wherein aryl is unsubstituted or mono- or poly-substituted, heteroaryl, wherein heteroaryl is unsubstituted or mono- or poly-substituted, C₁₋₈-alkyl-aryl or C₁₋₈-alkyl-heteroaryl, wherein alkyl is straight-chain or branched and is saturated or unsaturated and is unsubstituted or mono- or poly-substituted, aryl is unsubstituted or mono- or poly-substituted and heteroaryl is unsubstituted or mono- or poly-substituted,
R² represents hydrogen or C(=O)R⁹,
R³ represents C₁₋₈-alkyl, wherein alkyl is straight-chain or branched and is saturated or unsaturated and is unsubstituted or mono- or poly-substituted, C₃₋₈-cycloalkyl, wherein cycloalkyl is saturated or unsaturated and is unsubstituted or mono- or poly-substituted, heterocyclyl, wherein heterocyclyl is saturated or unsaturated and is unsubstituted or mono- or poly-substituted, aryl, wherein aryl is unsubstituted or mono- or poly-substituted, heteroaryl, wherein heteroaryl is unsubstituted or mono- or poly-substituted, C₁₋₈-alkyl-C₃₋₈-cycloalkyl, C₁₋₈-alkyl-heterocyclyl, C₁₋₈-alkyl-aryl or C₁₋₈-alkyl-heteroaryl, wherein alkyl is straight-chain or branched and is saturated or unsaturated and is unsubstituted or mono- or poly-substituted, cycloalkyl is saturated or unsaturated and is unsubstituted or mono- or poly-substituted, heterocyclyl is saturated or unsaturated and is unsubstituted or mono- or poly-substituted, aryl is unsubstituted or mono- or poly-substituted and heteroaryl is unsubstituted or mono- or poly-substituted,
R⁴ R⁵, R⁶ and R⁷ each independently of the others represents hydrogen or C₁₋₈-alkyl, wherein alkyl is straight-chain or branched and is saturated and is unsubstituted or mono- or poly-substituted, C₃₋₈-cycloalkyl or CH₂-C₃₋₈-cycloalkyl, wherein cycloalkyl is saturated or unsaturated and is unsubstituted or mono- or poly-substituted, F, Cl, Br, I, CN, NO₂, NH₂, C(=O)R⁹, CO₂H, CO₂R¹⁰ or OH, or
R⁴ and R⁵ or R⁵ and R⁶ or R⁶ and R⁷ represent a four-membered saturated or unsaturated hydrocarbon bridge having zero, 1, 2 or 3 hetero atoms selected from the group containing N, O and S, and the other radicals of R⁴, R⁵, R⁶ and R⁷ represent hydrogen,
R⁸ represents C(=O)R⁹,
R⁹ represents C₁₋₈-alkyl, wherein alkyl is straight-chain or branched and is saturated or unsaturated and is unsubstituted or mono- or poly-substituted, C₃₋₈-cycloalkyl or CH₂-C₃₋₈-cycloalkyl, wherein cycloalkyl is saturated or unsaturated and is unsubstituted or mono- or poly-substituted, heterocyclyl, wherein heterocyclyl is saturated or unsaturated and is unsubstituted or mono- or poly-substituted, aryl, wherein aryl is unsubstituted or mono- or poly-substituted, heteroaryl, wherein heteroaryl is unsubstituted or mono- or poly-substituted, C₁₋₈-alkyl-aryl or C₁₋₈-alkyl-heteroaryl, wherein alkyl is straight-chain or branched and is saturated or unsaturated and is unsubstituted or mono- or poly-substituted, aryl is unsubstituted or mono- or poly-substituted and heteroaryl is unsubstituted or mono- or poly-substituted, and
R¹⁰ represents C₁₋₈-alkyl, wherein alkyl is straight-chain or branched and is saturated or unsaturated and is unsubstituted or mono- or poly-substituted, C₃₋₈-cycloalkyl or CH₂-C₃₋₈-cycloalkyl, wherein cycloalkyl is saturated or unsaturated and is unsubstituted or mono- or poly-substituted, aryl, wherein aryl is unsubstituted or mono- or poly-substituted, C₁-₈-alkyl-aryl, wherein alkyl is straight-chain or branched and is saturated or unsaturated and is unsubstituted or mono- or poly-substituted and aryl is unsubstituted or mono- or poly-substituted,
with the exception of compounds having the general structure I in which simultaneously
R¹ = *tert*-butyl
R² = H
R³ = C₁₋₈-alkyl, wherein alkyl is straight-chain or branched and is saturated or unsaturated and is unsubstituted or mono- or poly-substituted, C₃₋₈-cycloalkyl, wherein cycloalkyl is saturated or unsaturated and is unsubstituted or mono- or poly-substituted, heterocyclyl, wherein heterocyclyl is saturated or unsaturated and is unsubstituted or mono- or poly-substituted, aryl, wherein aryl is unsubstituted or mono- or poly-substituted, heteroaryl, wherein heteroaryl is unsubstituted or mono- or poly-substituted, C₁₋₈-alkyl-C₃₋₈-cycloalkyl, C₁₋₈-alkyl-heterocyclyl, C₁₋₈-alkyl-aryl or C₁₋₈-alkyl-heteroaryl, wherein alkyl is straight-chain or branched and is saturated or unsaturated and is unsubstituted or mono- or poly-substituted, cycloalkyl is saturated or unsaturated and is unsubstituted or mono- or poly-substituted, heterocyclyl is saturated or unsaturated and is unsubstituted or mono- or poly-substituted, aryl is unsubstituted or mono- or poly-substituted and heteroaryl is unsubstituted or mono- or poly-substituted,
X = CR⁴ wherein R⁴ = H,
Y =CR⁵ wherein R⁵ = CH₃,
R⁶ = H and
R⁷= H or C₁₋₄-alkanyl, wherein alkanyl is straight-chain or branched and is unsubstituted or mono- or poly-substituted,
wherein in connection with "alkyl" and "alkanyl" in the excluded compounds the term "substituted" is understood to mean the substitution of a hydrogen radical by F, Cl, Br, I, -CN, NH₂, NH-alkyl, NH-aryl, NH-heteroaryl, NH-alkyl-aryl, NH-alkyl-heteroaryl, NH-heterocyclyl, NH-alkyl-OH, N(alkyl)₂, N(alkyl-aryl)₂, N(alkyl-heteroaryl)₂, N(heterocyclyl)₂, N(alkyl-OH)₂, NO, NO₂, SH, S-alkyl, S-aryl, S-heteroaryl, S-alkyl-aryl, S-alkyl-heteroaryl, S-heterocyclyl, S-alkyl-OH, S-alkyl-SH, OH, O-alkyl, O-aryl, O-heteroaryl, O-alkyl-aryl, O-alkyl-heteroaryl, O-heterocyclyl, O-alkyl-OH, CHO, C(=O)C₁₋₆-alkyl, C(=S)C₁₋₆-alkyl, C(=O)aryl, C(=S)aryl, C(=O)C₁₋₆-alkyl-aryl, 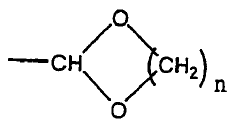 wherein n = 1, 2 or 3, C(=S)C₁₋₆-alkyl-aryl, C(=O)-heteroaryl, C(=S)-heteroaryl, C(=O)-heterocyclyl, C(=S)-heterocyclyl, CO₂H, CO₂-alkyl, CO₂-alkyl-aryl, C(=O)NH₂, C(=O)NH-alkyl, C(=O)NH-aryl, C(=O)NH-heterocyclyl, C(=O)N(alkyl)₂, C(=O)N(alkyl-aryl)₂, C(=O)N(alkyl-heteroaryl)₂, C(=O)N(heterocyclyl)₂, SO-alkyl, SO₂-alkyl, SO₂NH₂, SO₃H, cycloalkyl, aryl, heteroaryl or by heterocyclyl, where polysubstituted radicals are to be understood to be radicals that are polysubstituted either on different atoms or on the same atom,
in the preparation of a medicament for the treatment of multiple sclerosis, Parkinson's disease, Alzheimer's disease, Huntington's disease, cerebral ischaemia, diabetes, meningitis, arteriosclerosis and/or for healing wounds.

## Revendications

1. Utilisation d'un composé de formule générale I ou de l'un de ses sels acceptables du point de vue pharmaceutique 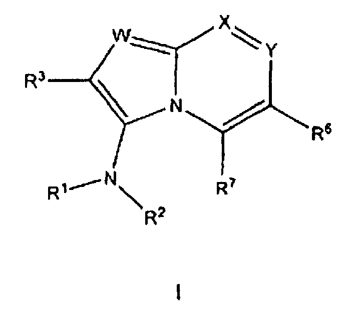 dans laquelle
X représente CR⁴ ou N,
Y représente CR⁵ ou N et
X et Y ne représentent pas N simultanément,
W représente N ou NR⁸,
R¹ est un reste alkyle en C₁ à C₁₂, ce reste alkyle étant linéaire ou ramifié, saturé ou non saturé et non substitué ou portant un ou plusieurs substituants, un reste cycloalkyle en C₃ à C₈ ou CH₂-(cycloalkyle en C₃ à C₈), le reste cycloalkyle étant saturé ou non saturé et non substitué ou portant un ou plusieurs substituants, un reste hétérocyclyle, ce reste hétérocyclyle étant saturé ou non saturé et non substitué ou portant un ou plusieurs substituants, un reste aryle, ce reste aryle étant non substitué ou portant un ou plusieurs substituants, un reste hétéroaryle, ce reste hétéroaryle étant non substitué ou portant un ou plusieurs substituants, un reste (alkyle en C₁ à C₈) -aryle ou (alkyle en C₁ à C₈)-hétéroaryle, la partie alkyle étant linéaire ou ramifiée, saturée ou non saturée et non substituée ou portant un ou plusieurs substituants, la partie aryle étant non substituée ou portant un ou plusieurs substituants et la partie hétéroaryle étant non substituée ou portant un ou plusieurs substituants,
R² désigne l'hydrogène ou un groupe C(=O)R⁹,
R³ est un reste alkyle en C₁ à C₈, ce reste alkyle étant linéaire ou ramifié, saturé ou non saturé et non substitué ou portant un ou plusieurs substituants, un reste cycloalkyle en C₃ à C₈, ce reste cycloalkyle étant saturé ou non saturé et non substitué ou portant un ou plusieurs substituants, un reste hétérocyclyle, ce reste hétérocyclyle étant saturé ou non saturé et non substitué ou portant un ou plusieurs substituants, un reste aryle, ce reste aryle étant non substitué ou portant un ou plusieurs substituants, un reste hétéroaryle, ce reste hétéroaryle étant non substitué ou portant un ou plusieurs substituants, un reste (alkyle en C₁ à C₈)-(cycloalkyle en C₃ à C₈), (alkyle en C₁ à C₈) -hétérocyclyle, (alkyle en C₁ à C₈) -aryle ou (alkyle en C₁ à C₈) -hétéroaryle, la partie alkyle étant linéaire ou ramifiée, saturée ou non saturée et non substituée ou portant un ou plusieurs substituants, la partie cycloalkyle étant saturée ou non saturée et non substituée ou portant un ou plusieurs substituants, la partie hétérocyclyle étant saturée ou non saturée et non substituée ou portant un ou plusieurs substituants, la partie aryle étant non substituée ou portant un ou plusieurs substituants et la partie hétéroaryle étant non substituée ou portant un ou plusieurs substituants,
R⁴, R⁵, R⁶ et R⁷ représentent indépendamment les uns des autres l'hydrogène ou un reste alkyle en C₁ à C₈, ce reste alkyle étant linéaire ou ramifié, saturé ou non saturé et non substitué ou portant un ou plusieurs substituants, un reste cycloalkyle en C₃ à C₈ ou CH₂- (cycloalkyle en C₃ à C₈), ce reste cycloalkyle étant saturé ou non saturé et non substitué ou substitué une ou plusieurs fois, F, Cl, Br, I, CN, NO₂, NH₂, C(=O)R⁹, CO₂H, CO₂R¹⁰, OH ou OR¹¹,
ou bien
R⁴ et R⁵ ou R⁵ et R⁶ ou R⁶ et R⁷ représentent un pont hydrocarboné saturé ou non saturé à quatre chaînons avec 0, 1, 2 ou 3 hétéroatomes, qui sont choisis dans le groupe comprenant N, O et S, et les autres groupes R⁴, R⁵, R⁶ et R⁷ restants représentent l'hydrogène,
R⁸ est un groupe C (=O) R⁹,
R⁹ est un reste alkyle en C₁ à C₈, ce reste alkyle étant linéaire ou ramifié, saturé ou non saturé et non substitué ou portant un ou plusieurs substituants, un reste cycloalkyle en C₃ à C₈ ou CH₂-(cycloalkyle en C₃ à C₈), ce reste cycloalkyle étant saturé ou non saturé, non substitué ou portant un ou plusieurs substituants, un reste hétérocyclyle, ce reste hétérocyclyle étant saturé ou non saturé et non substitué ou portant un ou plusieurs substituants, un reste aryle, ce reste aryle étant non substitué ou portant un ou plusieurs substituants, un reste hétéroaryle, ce reste hétéroaryle étant non substitué ou portant un plusieurs substituants, un reste (alkyle en C₁ à C₈)-aryle ou (alkyle en C₁ à C₈) -hétéroaryle, la partie alkyle étant linéaire ou ramifiée, saturée ou non saturée et non substituée ou portant un ou plusieurs substituants, la partie aryle étant non substituée ou portant un ou plusieurs substituants et la partie hétéroaryle étant non substituée ou portant un ou plusieurs substituants, et
R¹⁰ et R¹¹ représentent indépendamment l'un de l'autre un reste alkyle en C₁ à C₈, ce reste alkyle étant linéaire ou ramifié, saturé ou non saturé et non substitué ou portant un ou plusieurs substituants, un reste cycloalkyle en C₃ à C₈ ou CH₂-(cycloalkyle en C₃ à C₈), ce reste cycloalkyle étant saturé ou non saturé et non substitué ou portant un ou plusieurs substituants, un reste aryle, ce reste aryle étant non substitué ou portant un ou plusieurs substituants, un reste (alkyle en C₁ à C₈)-aryle, la partie alkyle étant linéaire ou ramifiée, saturée ou non saturée et non substituée ou portant un ou plusieurs substituants et la partie aryle étant non substituée ou portant un ou plusieurs substituants,
à l'exception de composés de formule générale I, dans lesquels, simultanément
R¹ est un reste tertiobutyle
R² représente H,
R³ est un reste alkyle en C₁ à C₈, ce reste alkyle étant linéaire ou ramifié et saturé ou non saturé et non substitué ou substitué une ou plusieurs fois, un reste cycloalkyle en C₃ à C₈, ce reste cycloalkyle étant saturé ou non saturé et non substitué ou substitué une ou plusieurs fois, un reste hétérocyclyle, ce reste étant saturé ou non saturé et non substitué ou substitué une ou plusieurs fois, un reste aryle, ce reste aryle étant non substitué ou substitué une ou plusieurs fois, un reste hétéroaryle, ce reste hétéroaryle étant non substitué ou substitué une ou plusieurs fois, un reste (alkyle en C₁ à C₈)-(cycloalkyle en C₃ à C₈), (alkyle en C₁ à C₈)-hétérocyclyle, (alkyle en C₁ à C₈)-aryle ou (alkyle en C₁ à C₈)-hétéroaryle, la partie alkyle étant linéaire ou ramifiée et saturée ou non saturée et non substituée ou substituée une ou plusieurs fois, la partie cycloalkyle étant saturée ou non saturée et non substituée ou substituée une ou plusieurs fois, la partie hétérocyclyle étant saturée ou non saturée et non substituée ou substituée une ou plusieurs fois, la partie aryle étant non substituée ou substituée une ou plusieurs fois et la partie hétéroaryle étant non substituée ou substituée une ou plusieurs fois,
X représente un groupe CR⁴, dans lequel R⁴ représente l'hydrogène,
Y représente un groupe CR⁵, dans lequel R⁵ est un reste CH₃,
R⁶ représente H et
R⁷ représente H ou un reste alcanyle en C₁ à C₄, ce reste alcanyle étant linéaire ou ramifié et non substitué ou substitué une ou plusieurs fois,
le terme "substitué" utilisé à propos des restes "alkyle" et "alcanyle" des composés exceptés signifiant le remplacement d'un reste hydrogène par F, Cl, Br, I, -CN, NH₂, NH-alkyle, NH-aryle, NH-hétéroaryle, NH-alkyl-aryle, NH-alkyl-hétéroaryle, NH-hétérocyclyle, NH-alkyl-OH, N(alkyle)₂, N(alkyl-aryle)₂, N(alkyl-hétéroaryle)₂, N(hétérocyclyle)₂, N(alkyl-OH)₂, NO, NO₂, SH, S-alkyle, S-aryle, S-hétéroaryle, S-alkyl-aryle, S-alkyl-hétéroaryle, S-hétérocyclyle, S-alkyl-OH, S-alkyl-SH, OH, O-alkyle, O-aryle, O-hétéroaryle, O-alkyl-aryle, O-alkyl-hétéroaryle, O-hétérocyclyle, O-alkyl-OH, CHO, C(=O)(alkyle en C₁ à C₆), C(=S) (alkyle en C₁ à C₆), C(=O)aryle, C(=S)aryle, C(=O) (alkyle en C₁ à C₆) aryle, 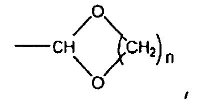 avec n = 1, 2 ou 3, C (=S) (alkyle en C₁ à C₆) aryle, C (=O) -hétéroaryle, C(=S)-hétéroaryle, C(=O)-hétérocyclyle, C(=S)-hétérocyclyle, CO₂H, CO₂-alkyle, CO₂-alkyl-aryle, C (=O) NH₂, C(=O)NH-alkyle, C(=O)NH-aryle, C(=O)NH-hétérocyclyle, C(=O)N(alkyle)₂, C(=O)N(alkyl-aryle)₂, C(=O)N(alkyl-hétéroaryle)₂, C(=O)N-(hétérocyclyle)₂, SO-alkyle, SO₂-alkyle, SO₂NH₂, SO₃H, cycloalkyle, aryle, hétéroaryle ou hétérocyclyle, des restes substitués plusieurs fois ayant pour définition des restes qui sont substitués plusieurs fois sur des atomes différents ou sur de mêmes atomes ,
pour la préparation d'un médicament destiné au traitement de douleurs inflammatoires.

2. Utilisation d'un composé de formule générale I ou de l'un de ses sels acceptables du point de vue pharmaceutique 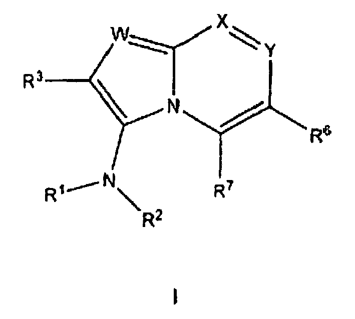 formule dans laquelle
X représente CR⁴ ou N,
Y représente CR⁵ ou N et
X et Y ne représentent pas N simultanément,
W représente N ou NR⁸,
R¹ est un reste alkyle en C₁ à C₁₂, ce reste alkyle étant linéaire ou ramifié, saturé ou non saturé et non substitué ou portant un ou plusieurs substituants, un reste cycloalkyle en C₃ à C₈ ou CH₂-(cycloalkyle en C₃ à C₈), le reste cycloalkyle étant saturé ou non saturé et non substitué ou portant un ou plusieurs substituants, un reste hétérocyclyle, ce reste hétérocyclyle étant saturé ou non saturé et non substitué ou portant un ou plusieurs substituants, un reste aryle, ce reste aryle étant non substitué ou portant un ou plusieurs substituants, un reste hétéroaryle, ce reste hétéroaryle étant non substitué ou portant un ou plusieurs substituants, un reste (alkyle en C₁ à C₈) -aryle ou (alkyle en C₁ à C₈) - hétéroaryle, la partie alkyle étant linéaire ou ramifiée, saturée ou non saturée et non substituée ou portant un ou plusieurs substituants, la partie aryle étant non substituée ou portant un ou plusieurs substituants et la partie hétéroaryle étant non substituée ou portant un ou plusieurs substituants,
R² désigne l'hydrogène ou un groupe C(=O)R⁹,
R³ est un reste alkyle en C₁ à C₈, ce reste alkyle étant linéaire ou ramifié, saturé ou non saturé et non substitué ou portant un ou plusieurs substituants, un reste cycloalkyle en C₃ à C₈, ce reste cycloalkyle étant saturé ou non saturé et non substitué ou portant un ou plusieurs substituants, un reste hétérocyclyle, ce reste hétérocyclyle étant saturé ou non saturé et non substitué ou portant un ou plusieurs substituants, un reste aryle, ce reste aryle étant non substitué ou portant un ou plusieurs substituants, un reste hétéroaryle, ce reste hétéroaryle étant non substitué ou portant un ou plusieurs substituants, un reste (alkyle en C₁ à C₈)-(cycloalkyle en C₃ à C₈), (alkyle en C₁ à C₈)-hétérocyclyle, (alkyle en C₁ à C₈) -aryle ou (alkyle en C₁ à C₈)-hétéroaryle, la partie alkyle étant linéaire ou ramifiée, saturée ou non saturée et non substituée ou portant un ou plusieurs substituants, la partie cycloalkyle étant saturée ou non saturée et non substituée ou portant un ou plusieurs substituants, la partie hétérocyclyle étant saturée ou non saturée et non substituée ou portant un ou plusieurs substituants, la partie aryle étant non substituée ou portant un ou plusieurs substituants et la partie hétéroaryle étant non substituée ou portant un ou plusieurs substituants,
R⁴, R⁵, R⁶ et R⁷ représentent indépendamment les uns des autres l'hydrogène ou un reste alkyle en C₁ à C₈, ce reste alkyle étant linéaire ou ramifié, saturé ou non saturé et non substitué ou portant un ou plusieurs substituants, un reste cycloalkyle en C₃ à C₈ ou CH₂-(cycloalkyle en C₃ à C₈), ce reste cycloalkyle étant saturé ou non saturé et non substitué ou substitué une ou plusieurs fois, F, Cl, Br, I, CN, NO₂, NH₂, C(=O)R⁹, CO₂H, CO₂R¹⁰, OH ou OR¹¹,
ou bien
R⁴ et R⁵ ou R⁵ et R⁶ ou R⁶ et R⁷ représentent un pont hydro-carboné saturé ou non saturé à quatre chaînons avec 0, 1, 2 ou 3 hétéroatomes, qui sont choisis dans le groupe comprenant N, O et S, et les autres groupes R⁴, R⁵, R⁶ et R⁷ restants représentent l'hydrogène,
R⁸ est un groupe C(=O)R⁹,
R⁹ est un reste alkyle en C₁ à C₈, ce reste alkyle étant linéaire ou ramifié, saturé ou non saturé et non substitué ou portant un ou plusieurs substituants, un reste cycloalkyle en C₃ à C₈ ou CH₂- (cycloalkyle en C₃ à C₈), ce reste cycloalkyle étant saturé ou non saturé, non substitué ou portant un ou plusieurs substituants, un reste hétérocyclyle, ce reste hétérocyclyle étant saturé ou non saturé et non substitué ou portant un ou plusieurs substituants, un reste aryle, ce reste aryle étant non substitué ou portant un ou plusieurs substituants, un reste hétéroaryle, ce reste hétéroaryle étant non substitué ou portant un plusieurs substituants, un reste (alkyle en C₁ à C₈) -aryle ou (alkyle en C₁ à C₈)-hétéroaryle, la partie alkyle étant linéaire ou ramifiée, saturée ou non saturée et non substituée ou portant un ou plusieurs substituants, la partie aryle étant non substituée ou portant un ou plusieurs substituants et la partie hétéroaryle étant non substituée ou portant un ou plusieurs substituants, et
R¹⁰ et R¹¹ représentent indépendamment l'un de l'autre un reste alkyle en C₁ à C₈, ce reste alkyle étant linéaire ou ramifié, saturé ou non saturé et non substitué ou portant un ou plusieurs substituants, un reste cycloalkyle en C₃ à C₈ ou CH₂-(cycloalkyle en C₃ à C₈), ce reste cycloalkyle étant saturé ou non saturé et non substitué ou portant un ou plusieurs substituants, un reste aryle, ce reste aryle étant non substitué ou portant un ou plusieurs substituants, un reste (alkyle en C₁ à C₈)-aryle, la partie alkyle étant linéaire ou ramifiée, saturée ou non saturée et non substituée ou portant un ou plusieurs substituants et la partie aryle étant non substituée ou portant un ou plusieurs substituants, à l'exception de composés de formule générale I, dans lesquels, simultanément
R¹ est un reste tertiobutyle,
R² représente H,
R³ est un reste alkyle en C₁ à C₈, ce reste alkyle étant linéaire ou ramifié et saturé ou non saturé et non substitué ou substitué une ou plusieurs fois, un reste cycloalkyle en C₃ à C₈, ce reste cycloalkyle étant saturé ou non saturé et non substitué ou substitué une ou plusieurs fois, un reste hétérocyclyle, ce reste étant saturé ou non saturé et non substitué ou substitué une ou plusieurs fois, un reste aryle, ce reste aryle étant non substitué ou substitué une ou plusieurs fois, un reste hétéroaryle, ce reste hétéroaryle étant non substitué ou substitué une ou plusieurs fois, un reste (alkyle en C₁ à C₈)-(cycloalkyle en C₃ à C₈), (alkyle en C₁ à C₈)-hétérocyclyle, (alkyle en C₁ à C₈)-aryle ou (alkyle en C₁ à C₈) -hétéroaryle, la partie alkyle étant linéaire ou ramifiée et saturée ou non saturée et non substituée ou substituée ou plusieurs fois, la partie cycloalkyle étant saturée ou non saturée et non substituée ou substituée une ou plusieurs fois, la partie hétérocyclyle étant saturée ou non saturée et non substituée ou substituée une ou plusieurs fois, la partie aryle étant non substituée ou substituée une ou plusieurs fois et la partie hétéroaryle étant non substituée ou substituée une ou plusieurs fois,
X représente un groupe CR⁴, dans lequel R⁴ représente l'hydrogène
Y représente un groupe CR⁵, dans lequel R⁵ est un reste CH₃,
R⁶ représente H et
R⁷ représente H ou un reste alcanyle en C₁ à C₄, ce reste alcanyle étant linéaire ou ramifié et non substitué ou substitué une ou plusieurs fois,
le terme "substitué" utilisé à propos des restes "alkyle" et "alcanyle" des composés exceptés signifiant le remplacement d'un reste hydrogène par F, Cl, Br, I, -CN, NH₂, NH-alkyle, NH-aryle, NH-hétéroaryle, NH-alkyl-aryle, NH-alkyl-hétéroaryle, NH-hétérocyclyle, NH-alkyl-OH, N(alkyle)₂, N(alkyl-aryle)₂, N(alkyl-hétéroaryle)₂, N(hétérocyclyle)₂, N(alkyl-OH)₂, NO, NO₂, SH, S-alkyle, S-aryle, S-hétéroaryle, S-alkyl-aryle, S-alkyl-hétéroaryle, S-hétérocyclyle, S-alkyl-OH, S-alkyl-SH, OH, O-alkyle, O-aryle, O-hétéroaryle, O-alkyl-aryle, O-alkyl-hétéroaryle, O-hétérocyclyle, O-alkyl-OH, CHO, C(=O)(alkyle en C₁ à C₆), C(=S) (alkyle en C₁ à C₆), C(=O)aryle, C (=S) aryle, C (=O) (alkyle en C₁ à C₆) aryle, 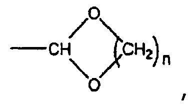 avec n = 1, 2 ou 3, C(=S)(alkyle en C₁ à C₆)aryle, C(=O)-hétéroaryle, C(=S)-hétéroaryle, C(=O)-hétérocyclyle, C(=S)-hétérocyclyle, CO₂H, CO₂-alkyle, CO₂-alkyl-aryle, C(=O)NH₂, C(=O)NH-alkyle, C(=O)NH-aryle, C(=O)NH-hétérocyclyle, C(=O)N(alkyle)₂, C(=O)N(alkyl-aryle)₂, C(=O)N(alkyl-hétéroaryle)₂, C(=O)N-(hétérocyclyle)₂, SO-alkyle, SO₂-alkyle, SO₂NH₂, SO₃H, cycloalkyle, aryle, hétéroaryle ou hétérocyclyle, des restes substitués plusieurs fois ayant pour définition des restes qui sont substitués plusieurs fois sur des atomes différents ou sur de mêmes atomes,
pour la préparation d'un médicament destiné au traitement de la migraine.

3. Utilisation d'un composé de formule générale I ou de l'un de ses sels acceptables du point de vue pharmaceutique 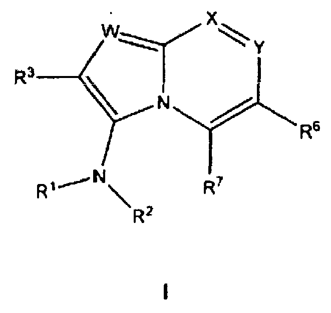 formule dans laquelle
X représente CR⁴ ou N,
Y représente CR⁵ ou N et
X et Y ne représentent pas N simultanément,
W représente N ou NR⁸,
R¹ est un reste alkyle en C₁ à C₁₂, ce reste alkyle étant linéaire ou ramifié, saturé ou non saturé et non substitué ou portant un ou plusieurs substituants, un reste cycloalkyle en C₃ à C₈ ou CH₂- (cycloalkyle en C₃ à C₈), le reste cycloalkyle étant saturé ou non saturé et non substitué ou portant un ou plusieurs substituants, un reste hétérocyclyle, ce reste hétérocyclyle étant saturé ou non saturé et non substitué ou portant un ou plusieurs substituants, un reste aryle, ce reste aryle étant non substitué ou portant un ou plusieurs substituants, un reste hétéroaryle, ce reste hétéroaryle étant non substitué ou portant un ou plusieurs substituants, un reste (alkyle en C₁ à C₈) -aryle ou (alkyle en C₁ à C₈)-hétéroaryle, la partie alkyle étant linéaire ou ramifiée, saturée ou non saturée et non substituée ou portant un ou plusieurs substituants, la partie aryle étant non substituée ou portant un ou plusieurs substituants et la partie hétéroaryle étant non substituée ou portant un ou plusieurs substituants,
R² désigne l'hydrogène ou un groupe C(=O)R⁹,
R³ est un reste alkyle en C₁ à C₈, ce reste alkyle étant linéaire ou ramifié, saturé ou non saturé et non substitué ou portant un ou plusieurs substituants, un reste cycloalkyle en C₃ à C₈, ce reste cycloalkyle étant saturé ou non saturé et non substitué ou portant un ou plusieurs substituants, un reste hétérocyclyle, ce reste hétérocyclyle étant saturé ou non saturé et non substitué ou portant un ou plusieurs substituants, un reste aryle, ce reste aryle étant non substitué ou portant un ou plusieurs substituants, un reste hétéroaryle, ce reste hétéroaryle étant non substitué ou portant un ou plusieurs substituants, un reste (alkyle en C₁ à C₈)-(cycloalkyle en C₃ à C₈), (alkyle en C₁ à C₈)-hétérocyclyle, (alkyle en C₁ à C₈) -aryle ou (alkyle en C₁ à C₈) -hétéroaryle, la partie alkyle étant linéaire ou ramifiée, saturée ou non saturée et non substituée ou portant un ou plusieurs substituants, la partie cycloalkyle étant saturée ou non saturée et non substituée ou portant un ou plusieurs substituants, la partie hétérocyclyle étant saturée ou non saturée et non substituée ou portant un ou plusieurs substituants, la partie aryle étant non substituée ou portant un ou plusieurs substituants et la partie hétéroaryle étant non substituée ou portant un ou plusieurs substituants,
R⁴, R⁵, R⁶ et R⁷ représentent indépendamment les uns des autres l'hydrogène ou un reste alkyle en C₁ à C₈, ce reste alkyle étant linéaire ou ramifié, saturé et non substitué ou portant un ou plusieurs substituants, un reste cycloalkyle en C₃ à C₈ ou CH₂- (cycloalkyle en C₃ à C₈), ce reste cycloalkyle étant saturé ou non saturé et non substitué ou substitué une ou plusieurs fois, F, Cl, Br, I, CN, NO₂, NH₂, C(=O)R⁹, CO₂H, CO₂R¹⁰ ou OH,
ou bien
R⁴ et R⁵ ou R⁵ et R⁶ ou R⁶ et R⁷ représentent un pont hydro-carboné saturé ou non saturé à quatre chaînons avec 0, 1, 2 ou 3 hétéroatomes, qui sont choisis dans le groupe comprenant N, O et S, et les autres groupes R⁴, R⁵, R⁶ et R⁷ restants représentent l'hydrogène,
R⁸ est un groupe C(=O)R⁹,
R⁹ est un reste alkyle en C₁ à C₈, ce reste alkyle étant linéaire ou ramifié, saturé ou non saturé et non substitué ou portant un ou plusieurs substituants, un reste cycloalkyle en C₃ à C₈ ou CH₂-(cycloalkyle en C₃ à C₈), ce reste cycloalkyle étant saturé ou non saturé, non substitué ou portant un ou plusieurs substituants, un reste hétérocyclyle, ce reste hétérocyclyle étant saturé ou non saturé et non substitué ou portant un ou plusieurs substituants, un reste aryle, ce reste aryle étant non substitué ou portant un ou plusieurs substituants, un reste hétéroaryle, ce reste hétéroaryle étant non substitué ou portant un plusieurs substituants, un reste (alkyle en C₁ à C₈)-aryle ou (alkyle en C₁ à C₈)-hétéroaryle, la partie alkyle étant linéaire ou ramifiée, saturée ou non saturée et non substituée ou portant un ou plusieurs substituants, la partie aryle étant non substituée ou portant un ou plusieurs substituants et la partie hétéroaryle étant non substituée ou portant un ou plusieurs substituants, et
R¹⁰ représente un reste alkyle en C₁ à C₈, ce reste alkyle étant linéaire ou ramifié, saturé ou non saturé et non substitué ou portant un ou plusieurs substituants, un reste cycloalkyle en C₃ à C₈ ou CH₂-(cycloalkyle en C₃ à C₈), ce reste cycloalkyle étant saturé ou non saturé et non substitué ou portant un ou plusieurs substituants, un reste aryle, ce reste aryle étant non substitué ou portant un ou plusieurs substituants, un reste (alkyle en C₁ à C₈)-aryle, la partie alkyle étant linéaire ou ramifiée, saturée ou non saturée et non substituée ou portant un ou plusieurs substituants et la partie aryle étant non substituée ou portant un ou plusieurs substituants,
à l'exception de composés de formule générale I, dans lesquels, simultanément
R¹ est un reste tertiobutyle,
R² représente H,
R³ est un reste alkyle en C₁ à C₈, ce reste alkyle étant linéaire ou ramifié et saturé ou non saturé et non substitué ou substitué une ou plusieurs fois, un reste cycloalkyle en C₃ à C₈, ce reste cycloalkyle étant saturé ou non saturé et non substitué ou substitué une ou plusieurs fois, un reste hétérocyclyle, ce reste étant saturé ou non saturé et non substitué ou substitué une ou plusieurs fois, un reste aryle, ce reste aryle étant non substitué ou substitué une ou plusieurs fois, un reste hétéroaryle, ce reste hétéroaryle étant non substitué ou substitué une ou plusieurs fois, un reste (alkyle en C₁ à C₈)-(cycloalkyle en C₃ à C₈), (alkyle en C₁ à C₈)-hétérocyclyle, (alkyle en C₁ à C₈)-aryle ou (alkyle en C₁ à C₈) -hétéroaryle, la partie alkyle étant linéaire ou ramifiée et saturée ou non saturée et non substituée ou substituée ou plusieurs fois, la partie cycloalkyle étant saturée ou non saturée et non substituée ou substituée une ou plusieurs fois, la partie hétérocyclyle étant saturée ou non saturée et non substituée ou substituée une ou plusieurs fois, la partie aryle étant non substituée ou substituée une ou plusieurs fois et la partie hétéroaryle étant non substituée ou substituée une ou plusieurs fois,
X représente un groupe CR⁴, dans lequel R⁴ représente l'hydrogène,
Y représente un groupe CR⁵, dans lequel R⁵ est un reste CH₃,
R⁶ représente H et
R⁷ représente H ou un reste alcanyle en C₁ à C₄, ce reste alcanyle étant linéaire ou ramifié et non substitué ou substitué une ou plusieurs fois,
le terme "substitué" utilisé à propos des restes "alkyle" et "alcanyle" des composés exceptés signifiant le remplacement d'un reste hydrogène par F, Cl, Br, I, -CN, NH₂, NH-alkyle, NH-aryle, NH-hétéroaryle, NH-alkyl-aryle, NH-alkyl-hétéroaryle, NH-hétérocyclyle, NH-alkyl-OH, N(alkyle)₂, N(alkyl-aryle)₂, N(alkyl-hétéroaryle)₂, N(hétérocyclyle)₂, N(alkyl-OH)₂, NO, NO₂, SH, S-alkyle, S-aryle, S-hétéroaryle, S-alkyl-aryle, S-alkyl-hétéroaryle, S-hétérocyclyle, S-alkyl-OH, S-alkyl-SH, OH, O-alkyle, O-aryle, O-hétéroaryle, O-alkyl-aryle, O-alkyl-hétéroaryle, O-hétérocyclyle, O-alkyl-OH, CHO, C(=O)(alkyle en C₁ à C₆), C(=S) (alkyle en C₁ à C₆), C(=O)aryle, C (=S) aryle, C (=O) (alkyle en C₁ à C₆) aryle, 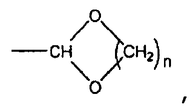 avec n = 1, 2 ou 3, C(=S) (alkyle en C₁ à C₆)aryle, C(=O)-hétéroaryle, C(=S)-hétéroaryle, C(=O)-hétérocyclyle, C(=S)-hétérocyclyle, CO₂H, CO₂-alkyle, CO₂-alkyl-aryle, C(=O)NH₂, C(=O)NH-alkyle, C(=O)NH-aryle, C(=O)NH-hétérocyclyle, C(=O)N(alkyle)₂, C(=O)N(alkyl-aryle)₂, C(=O)N(alkyl-hétéroaryle)₂, C(=O)N-(hétérocyclyle)₂, SO-alkyle, SO₂-alkyle, SO₂NH₂, SO₃H, cycloalkyle, aryle, hétéroaryle ou hétérocyclyle, des restes substitués plusieurs fois ayant pour définition des restes qui sont substitués plusieurs fois sur des atomes différents ou sur de mêmes atomes ,
pour la préparation d'un médicament destiné au traitement de la sclérose en plaques, de la maladie de Parkinson, de la maladie d'Alzheimer, de la maladie d'Huntington, de l'ischémie cérébrale, du diabète, de la méningite, de l'artériosclérose et/ou pour la cicatrisation.
